# EUROPEAN PATENT SPECIFICATION

(11) **EP 3 525 880 B1**
(45) Date of publication and mention of the grant of the patent: **17.11.2021**
(21) Application number: 17859554.2
(22) Date of filing: 12.10.2017
(51) Int. Cl.: A61N 2/00, A61N 2/02, A61N 1/36, A61N 7/00, A61H 23/00, A61B 5/053, H04R 25/00, A61N 1/04, A61N 1/08

(54) **MULTI-FACTOR CONTROL OF EAR STIMULATION**
MULTIFAKTORIELLE STEUERUNG EINER OHRSTIMULATION
COMMANDE MULTIFACTORIELLE DE STIMULATION DE L'OREILLE

(30) Priority: 12.10.2016 US 201615291358; 01.11.2016 US 201615340058; 01.11.2016 US 201615340145; 01.11.2016 US 201615340217
(43) Date of publication of application: 21.08.2019
(73) Proprietor: The Invention Science Fund II, LLC, Bellevue, WA 98005-4046 (US)
(72) Inventor: GOODALL, Eleanor V., Seattle, Washington 98144 (US); HYDE, Roderick A., Redmond, Washington 98052 (US); ISHIKAWA, Muriel Y., Livermore, California 94550-4921 (US); KITZAN, Melanie K., Bellevue, Washington 98005 (US); LEUTHARDT, Eric C., St. Louis, Missouri 63130 (US); MALAMUD, Mark A., Seattle, Washington 98119 (US); MALASKA, Stephen L., Snoqualmie, Washington 98065 (US); MYHRVOLD, Nathan P., Bellevue, Washington 98005-4046 (US); SCHEID, Brittany, St. Louis, Missouri 63130 (US); SHARADIN, Katherine E., Redmond, Washington 98052 (US); SWEENEY, Elizabeth A., Seattle, Washington 98133 (US); TEGREENE, Clarence T., Mercer Island, Washington 98040 (US); WHITMER, Charles, North Bend, Washington 98045 (US); WOOD, Lowell L., Jr., Bellevue, Washington 98004 (US); WOOD, Victoria Y. H., Livermore, California 94550-4921 (US); KARE, Jordin T., deceased (US)
(74) Representative: D Young & Co LLP
(86) International application number: PCT/US2017/056279
(87) International publication number: WO 2018/071630

(56) References cited:
- DE-B3-102013 011 541
- KR-A- 20140 123 044
- US-A- 4 966 164
- US-A- 4 966 164
- US-A1- 2006 020 161
- US-A1- 2008 249 594
- US-A1- 2008 288 016
- US-A1- 2013 030 321
- US-A1- 2016 279 435
- US-A1- 2017 027 812

## Description

### SUMMARY

Document US-A-4 966 164 discloses the most relevant prior art. The invention is defined in claim 1. Any embodiment which is in contradiction to the subject-matter of claim 1 is not part of the invention.

In an aspect, a neural stimulation system includes, but is not limited to, a neural signal sensor adapted to sense a neural signal from a subject, the neural signal indicative of a physiological status of the subject, a neural stimulator adapted to produce a stimulus responsive to the sensed neural signal, the stimulus configured to activate at least one sensory nerve fiber innervating at least a portion of a pinna of the subject, and a securing member configured to secure the neural stimulator to the pinna. In addition to the foregoing, other system aspects are described in the claims, drawings, and text forming a part of the disclosure set forth herein.

In an aspect, a method includes, but is not limited to, sensing with a neural signal sensor a neural signal indicative of a physiological status of a subject, the neural signal sensor located in or on a portion of a body of the subject, determining with signal analysis circuitry at least one parameter of the sensed neural signal, and delivering a neural stimulus with a neural stimulation device worn on a pinna of the subject responsive to the sensed neural signal, wherein the neural stimulus is configured to modulate the activity of at least one sensory nerve fiber innervating at least a portion of the pinna of the subject. In addition to the foregoing, other method aspects are described in the claims, drawings, and text forming a part of the disclosure set forth herein.

A wearable neural stimulation device includes, but is not limited to, a vibratory mechanical stimulator adapted to produce a vibratory stimulus of sufficient frequency and amplitude to modulate the activity of at least one mechanoreceptor with a receptive field on at least a portion of a pinna of a subject, and a securing member configured to secure the vibratory mechanical stimulator to the pinna. In addition to the foregoing, other device aspects are described in the claims, drawings, and text forming a part of the disclosure set forth herein.

In an aspect, a method includes, but is not limited to, delivering a vibratory mechanical stimulus to at least a portion of a pinna of a subject with a neural stimulation device worn on the pinna of the subject, wherein the vibratory mechanical stimulus is of sufficient frequency and amplitude to modulate the activity of at least one mechanoreceptor with a receptive field on the at least a portion of the pinna. In addition to the foregoing, other method aspects are described in the claims, drawings, and text forming a part of the disclosure set forth herein.

In an aspect, a neural stimulation system includes, but is not limited to, a wearable neural stimulation device and a personal computing device, the wearable neural stimulation device including a neural stimulator adapted to produce a stimulus for activating at least one sensory nerve fiber innervating at least a portion of a pinna of a subject, a securing member configured to secure the neural stimulator to the pinna, control circuitry incorporated into the wearable neural stimulation device for controlling operation of the neural stimulator, and first communication circuitry incorporated into the wearable neural stimulation device and operatively connected to the control circuitry, the first communication circuitry configured for at least one of sending a signal to and receiving a signal from a personal computing device; and the personal computing device including a user interface for at least one of presenting information to and receiving information from a user, control circuitry operatively connected to the user interface, second communication circuitry configured for at least one of sending a signal to and receiving a signal from the first communication circuitry, and instructions that when executed on the personal computing device cause the personal computing device to perform at least one of sending a signal to and receiving a signal from the wearable neural stimulation device via the second communication circuitry. In addition to the foregoing, other system aspects are described in the claims, drawings, and text forming a part of the disclosure set forth herein.

In an aspect, a system includes, but is not limited to, a personal computing device comprising circuitry for receiving a neural activity signal, the neural activity signal indicative of a physiological status of a subject, circuitry for determining a neural stimulus control signal based at least in part on the neural activity signal, and circuitry for outputting the neural stimulus control signal to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna. In addition to the foregoing, other system aspects are described in the claims, drawings, and text forming a part of the disclosure set forth herein.

In an aspect, a method includes, but is not limited to, receiving a neural activity signal at a personal computing device, the neural activity signal indicative of a physiological status of a subject, determining a neural stimulus control signal based at least in part on the neural activity signal, and outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna. In addition to the foregoing, other method aspects are described in the claims, drawings, and text forming a part of the disclosure set forth herein.

In an aspect, a computer program product includes, but is not limited to, a non-transitory signal-bearing medium bearing one or more instructions for receiving a neural activity signal, the neural activity signal indicative of a physiological status of a subject, one or more instructions for determining a neural stimulus control signal based at least in part on the neural activity signal, and one or more instructions for outputting the neural stimulus control signal to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna. In addition to the foregoing, other aspects of a computer program product are described in the claims, drawings, and text forming a part of the disclosure set forth herein.

In an aspect, a method includes, but is not limited to receiving a physiological activity signal at a personal computing device, the physiological activity signal indicative of a physiological status of a subject, determining a neural stimulus control signal based at least in part on the physiological activity signal, outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, and presenting information to the subject via a user interface. In addition to the foregoing, other method aspects are described in the claims, drawings, and text forming a part of the disclosure set forth herein.

In an aspect, a system includes, but is not limited to a personal computing device including circuitry for receiving a physiological activity signal at a personal computing device, the physiological activity signal indicative of a physiological status of a subject, circuitry for determining a neural stimulus control signal based at least in part on the physiological activity signal, the neural stimulus control signal is configured to control delivery of a neural stimulus by the external neural stimulator, the neural stimulus configured to activate at least one sensory nerve fiber innervating at least a portion of the pinna, circuitry for outputting the neural stimulus control signal from the personal computing device to a neural stimulation device including an external neural stimulator configured to be carried on a pinna of the subject, and circuitry for presenting information to the subject via a user interface. In addition to the foregoing, other personal computing device aspects are described in the claims, drawings, and text forming a part of the disclosure set forth herein.

In an aspect, a computer program product includes, but is not limited to, a non-transitory signal-bearing medium bearing one or more instructions for receiving a physiological activity signal, the physiological activity signal indicative of a physiological status of a subject, one or more instructions for determining a neural stimulus control signal based at least in part on the physiological activity signal, one or more instructions for outputting the neural stimulus control signal to a neural stimulation device including an external neural stimulator configured to be carried on an ear of a subject, wherein the neural stimulus control signal is configured to control delivery of a neural stimulus by the external neural stimulator, the neural stimulus configured to activate at least one sensory nerve fiber innervating at least a portion of the pinna, and one or more instructions for presenting information to the subject via a user interface. In addition to the foregoing, other computer program product aspects are described in the claims, drawings, and text forming a part of the disclosure set forth herein.

In an aspect, a system includes, but is not limited to a personal computing device including circuitry for receiving a physiological activity signal at a personal computing device, the physiological activity signal indicative of a physiological status of a subject, circuitry for determining a neural stimulus control signal based at least in part on the physiological activity signal, circuitry for outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, and circuitry for outputting an audio output signal via an audio output of the personal computing device. In addition to the foregoing, other system aspects are described in the claims, drawings, and text forming a part of the disclosure set forth herein.

In an aspect, a method includes, but is not limited to, receiving a physiological activity signal at a personal computing device, the physiological activity signal indicative of a physiological status of a subject, determining a neural stimulus control signal based at least in part on the physiological activity signal, outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, and outputting an audio output signal via an audio output of the personal computing device. In addition to the foregoing, other method aspects are described in the claims, drawings, and text forming a part of the disclosure set forth herein.

In an aspect, a computer program product includes, but is not limited to, a non-transitory signal-bearing medium bearing one or more instructions for receiving a physiological activity signal at a personal computing device, the physiological activity signal indicative of a physiological status of a subject, one or more instructions for determining a neural stimulus control signal based at least in part on the physiological activity signal, one or more instructions for outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, and one or more instructions for outputting an audio output signal via an audio output of the personal computing device. In addition to the foregoing, other computer program product aspects are described in the claims, drawings, and text forming a part of the disclosure set forth herein.

In an aspect, a method includes, but is not limited to, determining a vibratory stimulus control signal with stimulation control circuitry in a personal computing device, and outputting the vibratory stimulus control signal from the personal computing device to a wearable mechanical stimulation device including a vibratory mechanical stimulator configured to be carried on a pinna of a subject, wherein the vibratory stimulus control signal is configured to control delivery of a vibratory stimulus by the vibratory mechanical stimulator, the vibratory stimulus configured to activate at least one mechanoreceptor with a receptive field on at least a portion of the pinna. In addition to the foregoing, other method aspects are described in the claims, drawings, and text forming a part of the disclosure set forth herein.

In an aspect, a system includes, but is not limited to, a personal computing device including circuitry for determining a vibratory stimulus control signal, and circuitry for outputting the vibratory stimulus control signal to a wearable mechanical stimulation device including a vibratory mechanical stimulator configured to be carried on a pinna of a subject, wherein the vibratory stimulus control signal is configured to control delivery of a vibratory stimulus by the vibratory mechanical stimulator, the vibratory stimulus configured to activate at least one mechanoreceptor with a receptive field on at least a portion of the pinna. In addition to the foregoing, other system aspects are described in the claims, drawings, and text forming a part of the disclosure set forth herein.

In an aspect, a computer program product includes, but is not limited to, a non-transitory signal-bearing medium bearing one or more instructions for determining a vibratory stimulus control signal configured to control delivery of a vibratory stimulus by a vibratory mechanical stimulator, the vibratory stimulus configured to activate at least one mechanoreceptor with a receptive field on at least a portion of a pinna of a subject, and one or more instructions for outputting the vibratory stimulus control signal to a wearable mechanical stimulation device including the least one vibratory mechanical stimulator. In addition to the foregoing, other computer program product aspects are described in the claims, drawings, and text forming a part of the disclosure set forth herein.

In an aspect, a method includes, but is not limited to, receiving identifying information at a computing system, the identifying information identifying at least one of a subject and a neural stimulation device associated with the subject, the neural stimulation device configured to be carried on an ear of a subject and including an external neural stimulator, and transmitting a recommendation relating to a treatment regimen from the computing system to a personal computing device used by the subject, the treatment regimen including delivery of a neural stimulus to the subject with the external neural stimulator, the neural stimulus configured to activate at least one sensory nerve fiber innervating skin on or in the vicinity of the ear of the subject. In addition to the foregoing, other method aspects are described in the claims, drawings, and text forming a part of the disclosure set forth herein.

In an aspect, a system includes, but is not limited to, circuitry for receiving identifying information identifying at least one of a subject and a neural stimulation device associated with the subject, the neural stimulation device configured to be carried on an ear of a subject and including an external neural stimulator, and circuitry for providing a recommendation relating to a treatment regimen to the subject, the treatment regimen including delivery of a neural stimulus to the subject with the external neural stimulator, the neural stimulus configured to activate at least one sensory nerve fiber innervating skin on or in the vicinity of the ear of the subject. In addition to the foregoing, other system aspects are described in the claims, drawings, and text forming a part of the disclosure set forth herein.

In an aspect, a computer program product includes, but is not limited to, a non-transitory signal-bearing medium bearing one or more instructions for receiving identifying information identifying at least one of a subject and a neural stimulation device associated with the subject, the neural stimulation device configured to be carried on an ear of a subject and including an external neural stimulator, and one or more instructions for providing a recommendation relating to a treatment regimen to the subject, the treatment regimen including delivery of a neural stimulus to the subject with the external neural stimulator, the neural stimulus configured to activate at least one sensory nerve fiber innervating skin on or in the vicinity of the ear of the subject. In addition to the foregoing, other computer program product aspects are described in the claims, drawings, and text forming a part of the disclosure set forth herein.

In an aspect, a nerve stimulation earpiece includes an ear canal insert, and a concha insert. The ear canal insert is adapted to fit into an ear canal of a human subject. The ear canal insert includes at least one first electrode configured to electrically contact skin within the ear canal of the subject. The concha insert is adapted to fit within a concha of the subject. The concha insert includes a base portion configured to fit within a cavum of the concha of the subject, a wing portion configured to fit within a cymba of the concha of the subject, and at least one second electrode configured to electrically contact at least a portion of the concha of the subject. The nerve stimulation earpiece further includes at least one first electrical connector for connecting the at least one first electrode on the ear canal insert to a first electrical current source, and at least one second electrical connector for connecting the at least one second electrode on the concha insert to a second electrical current source.

In an aspect, an ear stimulation device controller is disclosed. The ear stimulation device controller includes a first analog output connector adapted to connect a first current signal to a first electrode of an ear canal insert of an ear stimulation device, and a second analog output connector adapted to connect a second current signal to a second electrode of a concha insert of the ear stimulation device. The ear stimulation device controller further includes a wireless microcontroller configured to control wireless communication between the ear stimulation device controller and a personal computing device to receive one or more stimulation parameters from the personal computing device; a digital stimulus signal generator configured to generate a digital stimulus signal based at least in part on the one or more stimulation parameters received from the personal computing device; a digital-to-analog converter for converting the digital stimulus signal to an analog voltage waveform; a current driver operably connected to the digital-to-analog converter and adapted generate a controlled current stimulus waveform responsive to the analog voltage waveform; and a power source operably connected to at least one of the wireless microcontroller, the digital stimulus signal generator, the digital-to-analog converter, and the current driver. The controlled current stimulus waveform is provided to the ear stimulation device via at least the first analog output connector and the second analog output connector.

In an aspect, a nerve stimulation system includes a nerve stimulation earpiece and an ear stimulation device controller operably coupled to the nerve stimulation earpiece. a nerve stimulation earpiece includes an ear canal insert, and a concha insert. The ear canal insert is adapted to fit into an ear canal of a human subject. The ear canal insert includes at least one first electrode configured to electrically contact skin within the ear canal of the subject. The concha insert is adapted to fit within a concha of the subject. The concha insert includes a base portion configured to fit within a cavum of the concha of the subject, a wing portion configured to fit within a cymba of the concha of the subject, and at least one second electrode configured to electrically contact at least a portion of the concha of the subject. The nerve stimulation earpiece further includes at least one first electrical connector for connecting the at least one first electrode on the ear canal insert to a first electrical current source, and at least one second electrical connector for connecting the at least one second electrode on the concha insert to a second electrical current source. The ear stimulation device controller includes a first analog output connector adapted to connect a first current signal to a first electrode of an ear canal insert of an ear stimulation device, and a second analog output connector adapted to connect a second current signal to a second electrode of a concha insert of the ear stimulation device. The ear stimulation device controller further includes a wireless microcontroller configured to control wireless communication between the ear stimulation device controller and a personal computing device to receive one or more stimulation parameters from the personal computing device; a digital stimulus signal generator configured to generate a digital stimulus signal based at least in part on the one or more stimulation parameters received from the personal computing device; a digital-to-analog converter for converting the digital stimulus signal to an analog voltage waveform; a current driver operably connected to the digital-to-analog converter and adapted generate a controlled current stimulus waveform responsive to the analog voltage waveform; and a power source operably connected to at least one of the wireless microcontroller, the digital stimulus signal generator, the digital-to-analog converter, and the current driver. The controlled current stimulus waveform is provided to the ear stimulation device via at least the first analog output connector and the second analog output connector.

In an aspect, a method of controlling an ear stimulation device with a personal computing device includes, but is not limited to, capturing, with image capture circuitry on the personal computing device, via a user-facing camera associated with the personal computing device, an image of a user of the personal computing device; processing the image, using image processing circuitry on the personal computing device, to determine at least one parameter; and controlling, with neural stimulus control signal determination circuitry on the personal computing device, based at least in part on the at least one parameter, delivery of a stimulus to at least one nerve innervating an ear of the user with the ear stimulation device. In a further aspect, the method includes processing the image, using the image processing circuitry, to determine the presence of at least one earpiece of the ear stimulation device located at an ear of the user; the ear of the user at which the at least one earpiece is located, the ear selected from a right ear of the user and a left ear of the user; and at least one attribute of the at least one earpiece indicative of usability of the at least one earpiece with one of the left or the right ear of the user; determining, using application software on the personal computing device, the ear at which the earpiece is usable, based on the at least one attribute of the at least one earpiece; determining, using application software on the personal computing device, whether the ear at which the at least one earpiece is located is the ear at which the earpiece is usable; and if the ear at which the at least one earpiece is located is not the ear at which the earpiece is usable, sending a control signal from the personal computing device to the ear stimulation device, under control of the neural stimulus control signal determination circuitry, to prevent delivery of a stimulus to the ear at which the earpiece is located via the earpiece. In addition to the foregoing, other method aspects are described in the claims, drawings, and text forming a part of the disclosure set forth herein.

In an aspect, an ear stimulation device control system includes, but is not limited to, a personal computing device; a user-facing camera associated with the personal computing device; image capture circuitry adapted to capture an image of a user of the personal computing device from the user-facing camera; image processing circuitry configured to process the image to determine at least one parameter; and neural stimulus control signal determination circuitry configured to control delivery of a stimulus to at least one nerve innervating an ear of the user with an ear stimulation device, based at least in part on the at least one parameter. In a further aspect, image processing circuitry includes an earpiece location module configured to process the image to determine the presence of at least one earpiece of the ear stimulation device located at an ear of the user; the ear of the user at which the at least one earpiece is located, the ear selected from a right ear of the user and a left ear of the user; and at least one attribute of the at least one earpiece indicative of usability of the at least one earpiece with one of the left or the right ear of the user; and the neural stimulus control signal determination circuitry is configured to determine the ear at which the earpiece is usable, based on the at least one attribute of the at least one earpiece; determine whether the ear at which the at least one earpiece is located is the ear at which the earpiece is usable; and if the ear at which the at least one earpiece is located is not the ear at which the earpiece is usable, send a control signal from the personal computing device to the ear stimulation device to prevent delivery of the stimulus to the a least one nerve innervating the ear of the user. In addition to the foregoing, other system aspects are described in the claims, drawings, and text forming a part of the disclosure set forth herein.

In an aspect, a method of controlling an ear stimulation device with a personal computing device includes, but is not limited, to detecting at electrical signal input circuitry, via at least one first electrode of an earpiece of an ear stimulation device, an electrical signal indicative of electrical contact of the at least one first electrode with the ear of a user of a personal computing device, wherein the at least one earpiece is operably connected to the personal computing device, and wherein the ear stimulation device is adapted to stimulate at least one nerve innervating the ear of the user of the personal computing device; determining, using contact determination circuitry on the personal computing device, whether the at least one first electrode is in good electrical contact with the ear of the user; if the at least one first electrode is not in good electrical contact with the ear of the user, sending a control signal from the personal computing device to the ear stimulation device, under control of neural stimulus control signal determination circuitry on the personal computing device, to prevent delivery via the earpiece of a stimulus to the ear at which the earpiece is located; and delivering, under control of notification circuitry on the personal computing device, a notification to the user relating to the status of the at least one first electrode. In addition to the foregoing, other method aspects are described in the claims, drawings, and text forming a part of the disclosure set forth herein.

In an aspect, an ear stimulation device control system includes, but is not limited to, a personal computing device configured to control delivery via an ear stimulation device of a stimulus to at least one nerve innervating an ear of a user of the personal computing device, the ear stimulation device including at least one first electrode, and the personal computing device including electrical signal circuitry adapted to receive an electrical signal indicative of electrical contact of the at least one first electrode with the ear of a user of the personal computing device; contact determination circuitry configured to determine whether the at least one first electrode is in good electrical contact with the ear of the user; neural stimulus control signal determination circuitry configured to send a control signal from the personal computing device to the ear stimulation device to prevent delivery of the stimulus if the at least one first electrode is not in good electrical contact with the ear of the user; and notification circuitry configured to deliver a notification to the user relating to the status of the at least one first electrode. In addition to the foregoing, other system aspects are described in the claims, drawings, and text forming a part of the disclosure set forth herein.

In an aspect, a personal computing device application for monitoring use of a nerve stimulation system by a user includes, but is not limited to, an audio delivery module adapted to control delivery of an audio signal from an audio signal source to an audio earpiece via an audio output of the personal computing device, the audio earpiece having associated therewith an ear stimulation device configured to stimulate a nerve innervating the ear of the user; a mood assessment module adapted to receive mood-related input from the user via a first input structure associated with the personal computing device; and assess a mood of the user based at least in part upon the mood-related input; a secondary factor input module adapted to receive at least one input relating to at least one secondary factor relating to the user via a second input structure associated with the personal computing device; user control module adapted to receive at least one user control input via a third input structure of the personal computing device, the user control input for controlling user-controllable stimulation parameters of the ear stimulation device; a stimulator control module adapted to determine at least one stimulus control parameter based on at least one of the mood of the user, the at least one secondary factor, and the at least one user control input; and a controller interface module for communicating the at least one stimulus control parameter to a stimulator controller adapted to control the ear stimulation device responsive to the at least one stimulus control parameter. In addition to the foregoing, other system aspects are described in the claims, drawings, and text forming a part of the disclosure set forth herein.

In an aspect, a method of controlling an ear stimulation device with a personal computing device includes, but is not limited to, receiving an audio signal at the personal computing device from an audio signal source; delivering the audio signal to an audio earpiece worn by a user via an audio output of the personal computing device, the audio earpiece having associated therewith an ear stimulation device configured to stimulate a nerve innervating the ear of the user; receiving with a mood assessment module, via a first input structure associated with the personal computing device, a mood-related input from the user; assessing, with the mood assessment module, a mood of the user based at least in part upon the mood-related input; receiving with a secondary factor input module, via a second input structure associated with the personal computing device, at least one input relating to at least one secondary factor relating to the user; receiving with a user control module, via a third input structure associated with the personal computing device, at least one user control input for controlling at least one user-controllable stimulation parameter of the ear stimulation device; determining, with a stimulator control module, at least one stimulus control parameter based on at least one of the mood of the user, the at least one secondary factor, and the at least one user control input; and communicating, with a controller interface module, at least one stimulus control parameter to a stimulator controller, the stimulator controller adapted to control the ear stimulation device responsive to the at least one stimulus control parameter. In addition to the foregoing, other method aspects are described in the claims, drawings, and text forming a part of the disclosure set forth herein.

Features from any of the disclosed embodiments can be used in combination with one another, without limitation. In addition, other features and advantages of the present disclosure will become apparent to those of ordinary skill in the art through consideration of the following detailed description and the accompanying drawings.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE FIGURES

**FIG. 1** is an illustration of the external anatomy of the ear of a human.
**FIG. 2A** is an illustration of a system including a neural stimulation device worn on the ear of a subject.
**FIG. 2B** is a block diagram of the system of **FIG. 2A****.**
**FIG. 3** depicts a stimulation device including a securing member configured to fit in the concha, and a clip securing member.
**FIG. 4A** depicts a stimulation device including a hanger-style securing member.
**FIG. 4B** depicts the stimulation device of **FIG. 4A** positioned on an ear.
**FIG. 5** depicts an embodiment of a stimulation device.
**FIG. 6** depicts an embodiment of a stimulation device.
**FIG. 7** is a block diagram of a neural stimulation system.
**FIG. 8** is a block diagram of a computing system.
**FIG. 9** is a flow diagram of a method.
**FIG. 10** is a block diagram of a neural stimulation device.
**FIG. 11** is a flow diagram of a method.
**FIG. 12** is a block diagram of a neural stimulation system.
**FIG. 13** is a block diagram of a system including a personal computing device.
**FIG. 14** is a flow diagram of a method.
**FIG. 15** is a block diagram of a computer program product relating to the method of **FIG. 14****.**
**FIG. 16** is a block diagram of a system including a personal computing device.
**FIG. 17** is a flow diagram of a method.
**FIG. 18** is a block diagram of a computer program product relating to the method of **FIG. 17****.**
**FIG. 19** is a block diagram of a system including a personal computing device.
**FIG. 20** is a flow diagram of a method.
**FIG. 21** is a block diagram of a computer program product relating to the method of **FIG. 20****.**
**FIG. 22** is a block diagram of a system including a personal computing device.
**FIG. 23** is a flow diagram of a method.
**FIG. 24** is a block diagram of a computer program product relating to the method of **FIG. 23****.**
**FIG. 25** is a block diagram of a system relating to operation of a neural stimulation device.
**FIG. 26** depicts data aspects relating to **FIG. 25****.**
**FIG. 27** is a flow diagram of a method.
**FIG. 28** is block diagram of a computer program product relating to the method of **FIG. 27****.**
**FIG. 29** is an illustration of an embodiment of a system for delivering neural stimulation in combination with a secondary stimulus.
**FIG. 30A** depicts a nerve stimulation earpiece.
**FIG. 30B** depicts an exploded view of the nerve stimulation earpiece shown in **FIG. 30A****.**
**FIG. 31** depicts a block diagram of the nerve stimulation earpiece shown in **FIGS. 30A-B.**
**FIG. 32A** depicts a nerve stimulation earpiece including an example mounting structure and an audio headphone.
**FIG. 32B** depicts the nerve stimulation earpiece shown in **FIG. 32A****.**
**FIG. 33A** depicts a nerve stimulation earpiece including an example mounting structure and an audio headphone.
**FIG. 33B** depicts the example nerve stimulation earpiece shown in **FIG. 33A****.**
**FIG. 34** depicts a nerve stimulation earpiece including an example mounting structure and an audio headphone.
**FIG. 35A** depicts a nerve stimulation earpiece and an audio headphone.
**FIG. 35B** depicts exploded view of the nerve stimulation earpiece and audio headphone shown in **FIG. 35A****.**
**FIG. 36** depicts side and top plan views of the concha insert shown in **FIGS. 35A-B****.**
**FIG. 37** depicts side and end views of the ear canal insert shown in **FIGS. 35A-B**.
**FIG. 38** depicts a nerve stimulation earpiece.
**FIG. 39A** depicts an external side view of the nerve stimulation earpiece shown in **FIG. 38** in an ear of a subject.
**FIG. 39B** depicts a sectional view along the plane defined by line A-A of FIG. 39A.
**FIG. 40** depicts a block diagram of an ear stimulation device controller.
**FIG. 41** depicts a block diagram of a printed circuit board of the ear stimulation device controller shown in **FIG. 40****.**
**FIG. 42** depicts a block diagram an example nerve stimulation system.
**FIG. 43** is a flow diagram of a method.
**FIG. 44** is a flow diagram of a method.
**FIG. 45** is a flow diagram of a method.
**FIG. 46** is a flow diagram of a method.
**FIG. 47** is a block diagram of a neural stimulation system.
**FIG. 48A** depicts a user interface for a neural stimulation system.
**FIG. 48B** depicts a user interface for a neural stimulation system.
**FIG. 49** is a block diagram of an embodiment of a neural stimulation system.
**FIG. 50** is a flow diagram of a method.
**FIG. 51** is a flow diagram of a method.
**FIG. 52** is a flow diagram of a method.
**FIG. 53** is a block diagram of a system including a personal computing device.
**FIG. 54** is a flow diagram of a method.
**FIG. 55** is a flow diagram of a method.
**FIG. 56** is a flow diagram of a method.

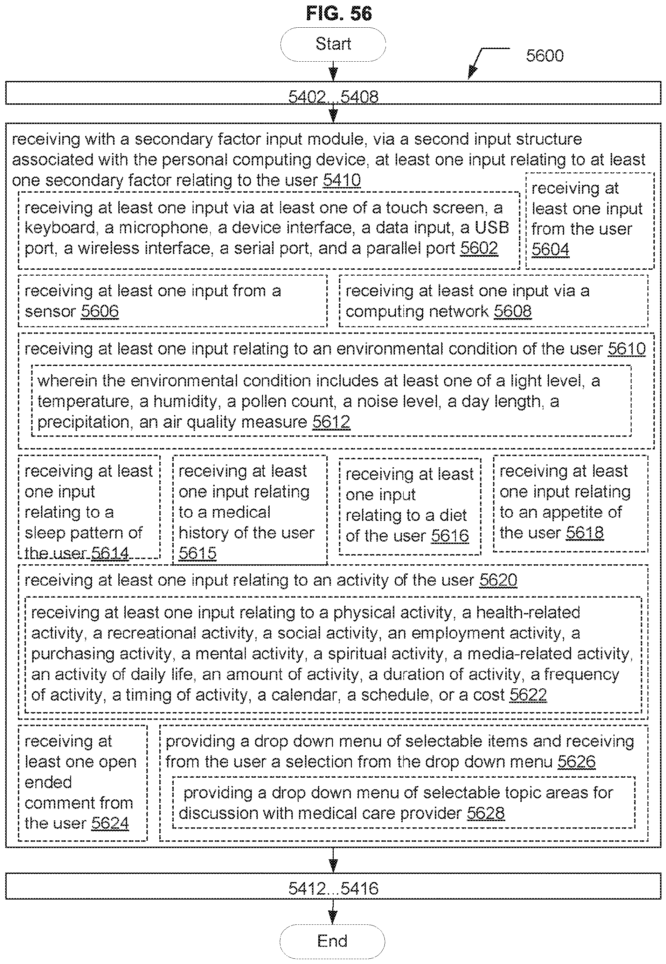

**FIG. 56** is a flow diagram of a method.

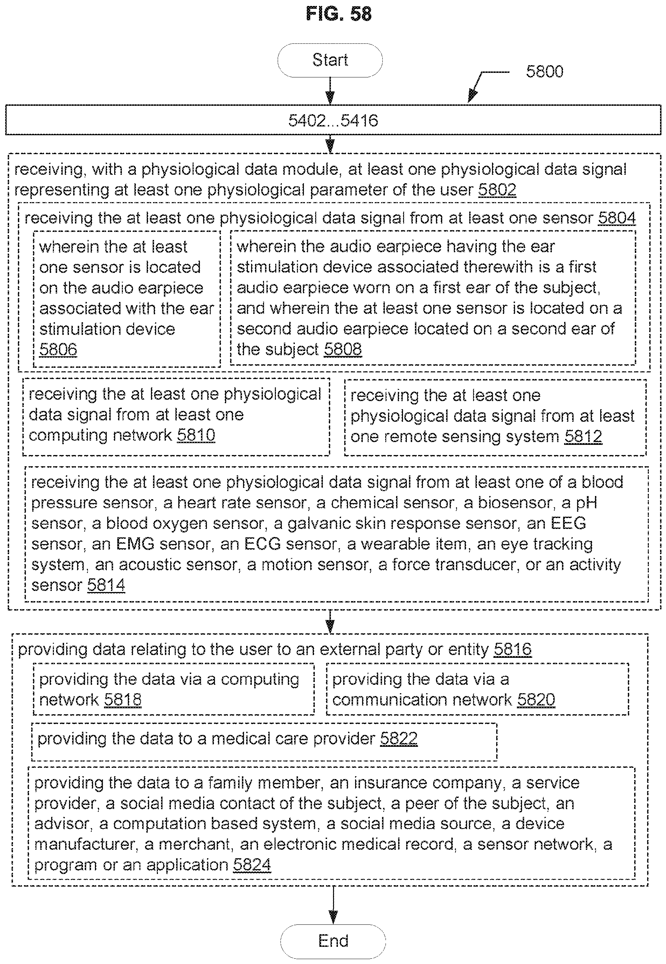

**FIG. 58** is a flow diagram of a method.

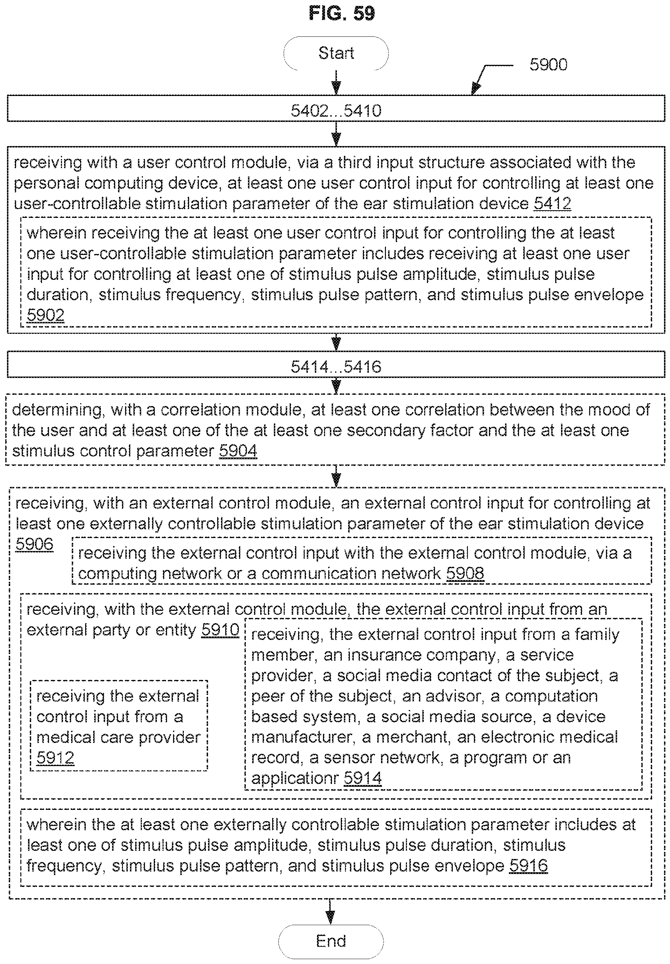

**FIG. 59** is a flow diagram of a method.

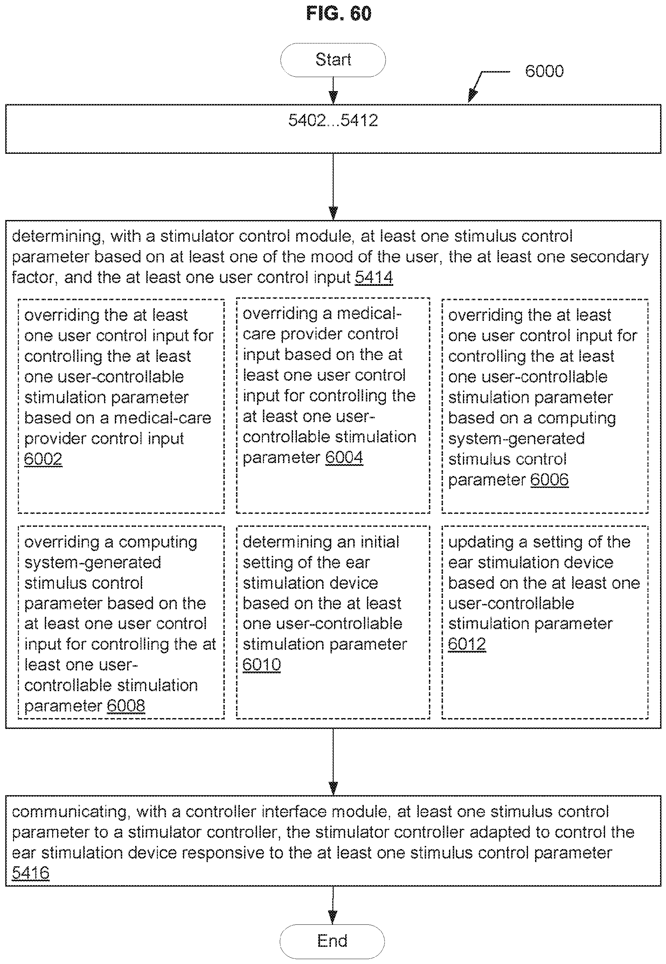

**FIG. 60** is a flow diagram of a method.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented here.

Various studies indicate that stimulation of the ear can have beneficial effects on the health of a subject. For example, Rong et al., "Transcutaneous vagus nerve stimulation for the treatment of depression: a study protocol for a double blinded randomized clinical trial," BMC Complementary and Alternative Medicine 2012, 12:255 describes the possibility of using transcutaneous stimulation of the vagus nerve via portions of the ear to treat major depressive disorder (MDD) and other disorders, including epilepsy, bipolar disorder, and morbid obesity. Ellrich, "Transcutaneous Vagus Nerve Stimulations," European Neurological Review, 2011; 6(4): 254-256 describes transcutaneous vagus nerve stimulation via the ear for treating epilepsy and depression.

Nerves innervating the skin on or in the vicinity of the ear of the subject include, e.g., the facial nerve (cranial nerve VII), the glossopharyngeal nerve (cranial nerve IX), the auricular branch of the vagus nerve (cranial nerve X), the auriculotemporal branch of trigeminal nerve (cranial nerve V), the lesser occipital nerve (spinal nerve C3), and the greater auricular nerve (spinal nerves C2, C3). These nerves contain various nerve fibers including sensory nerve fibers, including, for example, nerve fibers from skin mechanoreceptors. Various types of skin mechanoreceptors are well characterized and are innervated by fibers having diameters in the range of approximately 5 to 12 µm (also known as Aβ fibers). Skin mechanoreceptors include, for example, slowly adapting mechanoreceptors, which are more sensitive to continuous stimulation, and rapidly adapting mechanoreceptors, which are more sensitive to transient stimuli. Rapidly adapting mechanoreceptors include Pacinian corpuscles and Meissner's corpuscles, for example.

Mechanoreceptors are activated well by cyclical or vibratory (e.g., sinusoidal) mechanical stimuli having frequencies in the range of 1 Hz to 1000 Hz. In some aspects, such mechanical stimuli may include indentation of the skin by a few micrometers to a few millimeters. Pacinian corpuscles are thought to be most responsive to vibratory mechanical stimuli with frequencies in the range of 200 Hz - 300 Hz, while Meissner's Corpuscles are thought to be most responsive to vibratory mechanical stimuli with frequencies in the range of 30 - 40 Hz.

Electrical stimuli having sinusoidal or other waveforms are also effective for activating sensory fibers. Stimuli may be applied cyclically, for example. See e.g., Ellrich, "Transcutaneous Vagus Nerve Stimulations," European Neurological Review, 2011; 6(4):254-256.

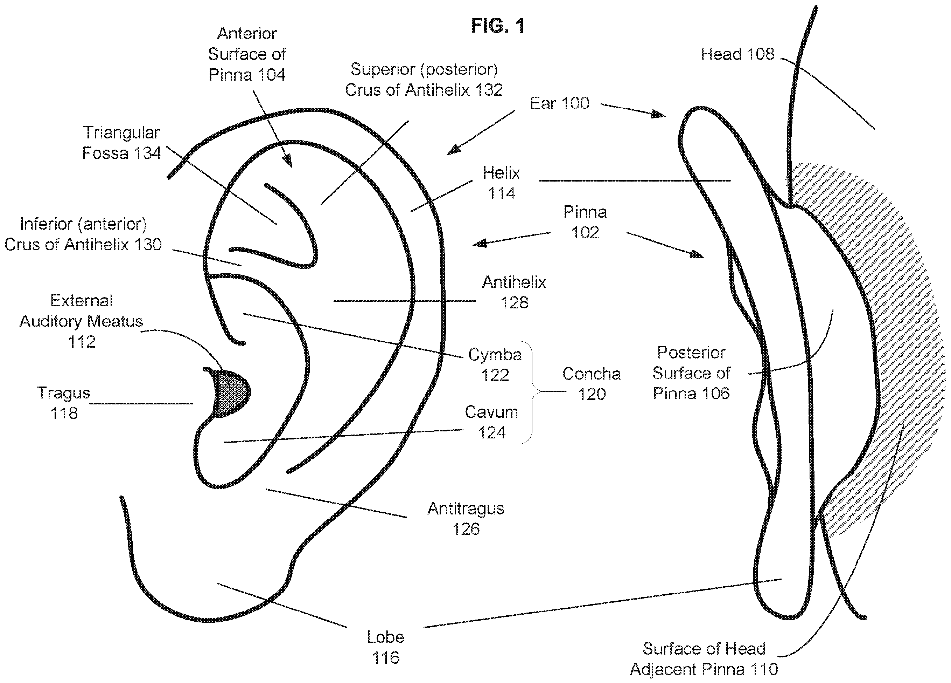

For reference, FIG. 1 depicts an ear 100 of a human subject, showing anatomical structures which may be referred to herein. The external portion of ear 100 is referred to as the pinna 102. FIG. 1 depicts a front/side view of ear 100, showing anterior surface of pinna 104, and a back view of ear 100, showing posterior surface of pinna 106 as well as head 108 of the subject. The surface of the head 108 adjacent the pinna 102 is indicated by shading and reference number 110. Anatomical features of the ear include external auditory meatus 112 (the external ear canal), helix 114, lobe 116, and tragus 118. Concha 120, the indented region in the vicinity of external auditory meatus 112, is comprised of cymba 122 and cavum 124, and bounded by antitragus 126 and antihelix 128. Antihelix 128 includes inferior (anterior) crus of antihelix 130 and superior (posterior) crus of antihelix 132, which bound triangular fossa 134.

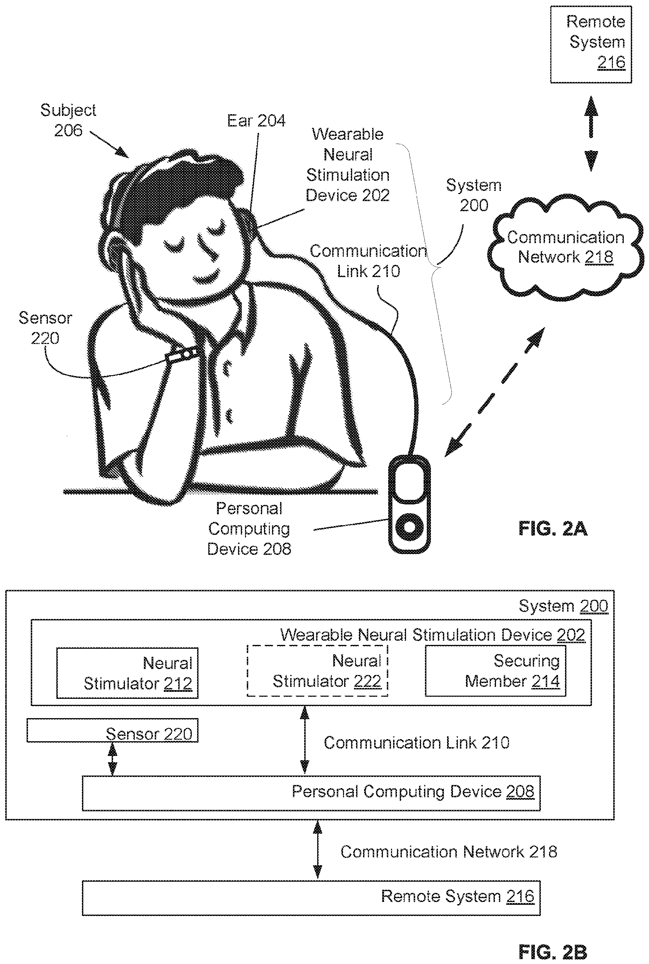

FIGS. 2A and 2B depict a generalized system 200 including a wearable neural stimulation device 202 for delivering a stimulus to an ear 204 of a subject 206. System 200 includes a personal computing device 208 in communication with wearable neural stimulation device 202 via communication link 210. Personal computing device 208 can be an audio player, a mobile phone, a computer, or any of various other devices having computing capability (e.g., microprocessor based devices) and including application software and/or suitable hardware for controlling operation of wearable neural stimulation device 202. In an aspect, personal computing device 208 is a wearable computing device. In an aspect, wearable neural stimulation device 202 is used to deliver a stimulus sufficient to activate one or more nerves or nerve branches innervating the skin on or in the vicinity of ear 204 of subject 206. In an aspect, personal computing device 208 is used to control delivery of the stimulus to ear 204 of subject 206. As illustrated in the block diagram of FIG. 2B, and described in greater detail herein below, wearable neural stimulation device 202 includes neural stimulator 212 and securing member 214 for securing neural stimulator 212 to ear 204. In an aspect, personal computing device 208 is configured to send, or receive, information relating to operation of the wearable neural stimulation device 202 to, or from, one or more remote system 216 via a communications network 218. Control of stimulation may be based on data from one or more sensor 220, including, but not limited to, physiological sensors, neural activity sensors, motion sensors, location sensors, or environmental sensors, for example. In some aspects, sensor 220 is worn by the subject at a location distinct from wearable neural stimulation system 202 (e.g., on an armband as depicted in FIG. 2A). In other aspects, one or more sensors are located on a wearable neural stimulation device that can be implanted in the subject, located on the personal computing device, or located elsewhere in the environment of the subject, as depicted and described in the following text and accompanying figures.

In the embodiment of FIGS. 2A and 2B, and in other embodiments described herein, neural stimulator 212 can be any of various types of neural stimulators, including but not limited to mechanical, electrical, magnetic, ultrasonic, optical, or chemical stimulators, as will be discussed in greater detail herein below. In an aspect, neural stimulation devices as described herein can include multiple (two or more) neural stimulators (see e.g., optional additional neural stimulator 222 in FIG. 2B). If multiple neural stimulators are used, they may all be of the same type, or may be of several different types.

In an aspect, neural stimulator 212 is a mechanical stimulator. In an aspect, a mechanical stimulator includes, for example, a vibratory mechanical stimulator that delivers a cyclical or vibrating mechanical stimulus to the skin of the ear of the subject. Vibratory mechanical stimulators can include, for example, various types of vibrating mechanical devices, e.g., electromechanical, piezoelectric, movable coil, electrostatic, magnetostrictive, isodynamic, and/or MEMS devices, for example as used for manufacturing small-scale speakers and microphones.

In an aspect, neural stimulator 212 includes a transcutaneous electrical stimulator for delivering a transcutaneous electrical stimulus. For example, neural stimulator 212 may include an electrode or electrical contact designed for contacting the skin surface, for example as described in Rong et al.,"Transcutaneous vagus nerve stimulation for the treatment of depression: a study protocol for a double blinded randomized clinical trial," BMC Complementary and Alternative Medicine 2012, 12:255. In an aspect, neural stimulator 212 includes a magnetic stimulator for delivering a transcutaneous magnetic stimulus. For example, such a magnetic stimulator may include one or more coil through which electrical current is passed to generate a magnetic field. The magnetic field induces electrical currents within the tissue in/around the ear of the subject to activate neural structures. In an aspect, neural stimulator 212 includes an ultrasonic stimulator, for example as described in Legon et al., "Pulsed Ultrasound Differentially Stimulates Somatosensory Circuits in Humans as Indicated by EEGand fMRI," PLOS ONE 7(12): e5177. Doi:10.01371/journal.pone.0051177, December 2012. In some aspects, other types of neural stimulators, such as optical or chemical stimulators are used. See, for example, stimulators described in U.S. Patent 8,170,658 to Dacey, Jr. et al.

In some aspects, circuitry for driving delivery of the neural stimulus is included fully or partially in wearable neural stimulation device 202. In some aspects, some or all of the circuitry for driving delivery of the neural stimulus are housed separately from wearable neural stimulation device 202, and a control signal for driving delivery of the neural stimulus by neural stimulator 212 is provided by personal computing device 208, or from remote system 216 via communication network 218.

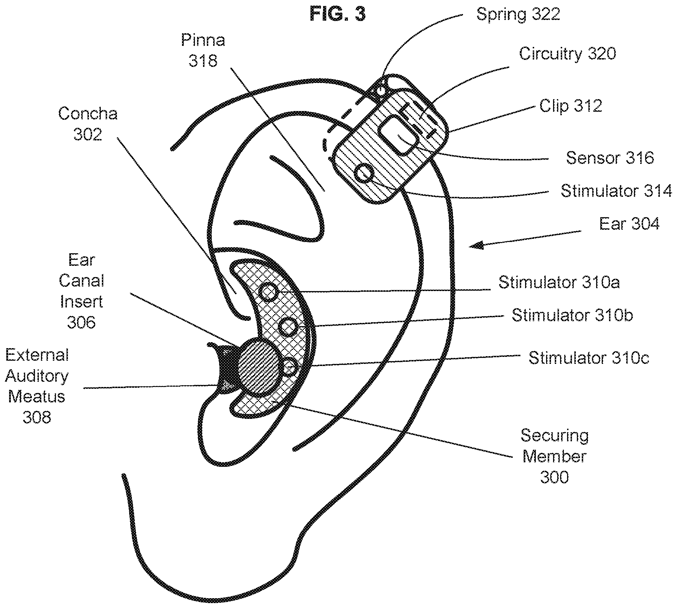

Various examples and embodiments of neural stimulation devices are described herein. In various aspects of neural stimulation systems described herein, neural stimulation devices are wearable, i.e. the device can be carried by or worn on the ear of a subject, secured by a securing member, in order to position one or more neural stimulator with respect to a portion of the ear of the subject, or in some cases, in the vicinity of the ear of the subject. Various types of securing members may be used, without limitation. A securing member may also serve to position one or more sensors on or in the vicinity of the ear of the subject and may also include or support other system components, such as electrical circuitry components. Examples of neural stimulation devices including different types of securing members are shown in FIGS. 3 - 6.

FIG. 3 depicts securing member 300, which is a concha-fitted member configured to fit into concha 302 of ear 304. In this example, securing member 300 has a size and shape sufficient to be retained in concha 302 by friction and/or tensioning of securing member 300 with respect to concha 302. Other system components may be attached to securing member 300, e.g., ear canal insert 306, which extends into external auditory meatus (ear canal) 308 and stimulators 310a, 310b, and 310c. In addition, system components may be built into or contained within securing member 300, e.g., control and/or communication circuitry (not shown) used to drive stimulators 310a, 310b, and 310c and/or provide for communication with e.g., a personal computing device (not shown). A battery can be provided in securing member 300 to power the device for wireless operation. FIG. 3 also depicts a second type of securing member, clip 312, for attaching stimulator 314 and/or sensor 316 to the pinna 318 of the subject. Circuitry 320 provides for wireless communication between stimulator 314/sensor 316 and circuitry on securing member 300 or a personal computing device or remote system. Spring 322 provides spring force to secure clip 312 onto pinna 318. Clip 312 may be formed of a resilient material or formed from two sections of rigid material, joined at a hinge.

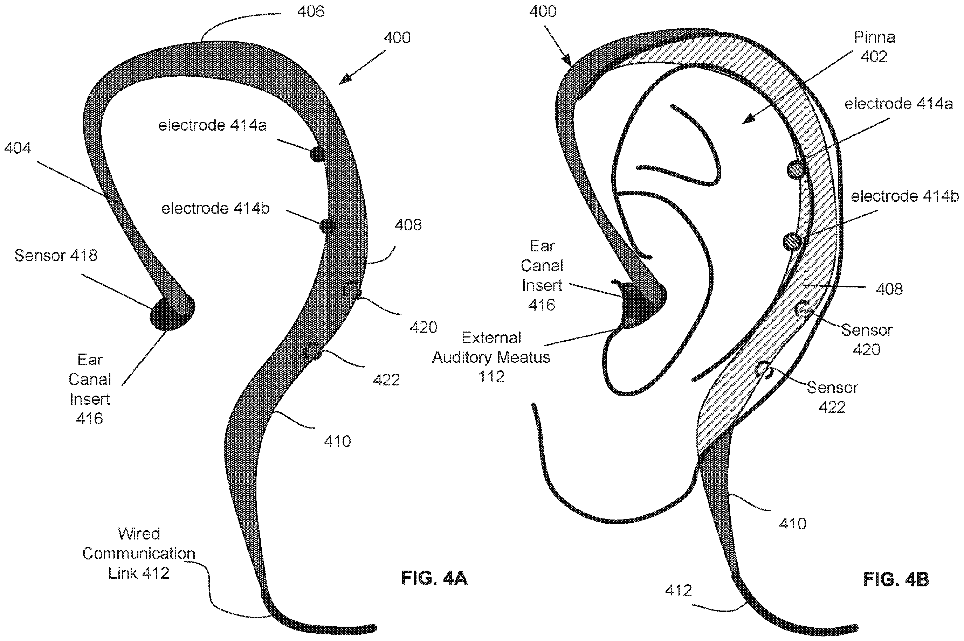

FIGS. 4A and 4B depict securing member 400 having a hanger-style configuration designed to hang on pinna 402. The hanger-style configuration is similar to the configuration used in certain types of headsets for listening to music. Securing member 400 includes anterior portion 404, which in use (shown in FIG. 4B) is positioned anterior to the ear of the subject (i.e. in front of pinna 402); over-ear portion 406, which arcs over and behind pinna 402; and posterior portion 408, which fits behind pinna 402. In an aspect, securing member 400 includes downward extension 410. In an aspect, wired communication link 412 (e.g., a cable) provides for connection of electrical components on securing member 400 to a remote computing device. For example, electrodes 414a and 414b on posterior portion 408 of securing member 400 are used to deliver electrical stimulation under control of a control signal delivered via wired communication link 412. Securing member 400 also includes ear canal insert 416, which fits into the external auditory meatus 112. A sensor 418 on ear canal insert 416 can be used to sense a physiological signal, which in some aspects is used to determine the stimulation delivered with electrodes 414a and 414b. Physiological sensor 418 may include, for example, an electrode for sensing a heart rate, or other physiological sensor as described in greater detail elsewhere herein. Additional sensors 420 and 422 are located on the aspect of posterior portion 408, facing and adapted to contact the surface of the head adjacent the pinna 402. In an aspect, sensors 420 and 422 are electrodes configured to detect an electroencephalographic (EEG) signal.

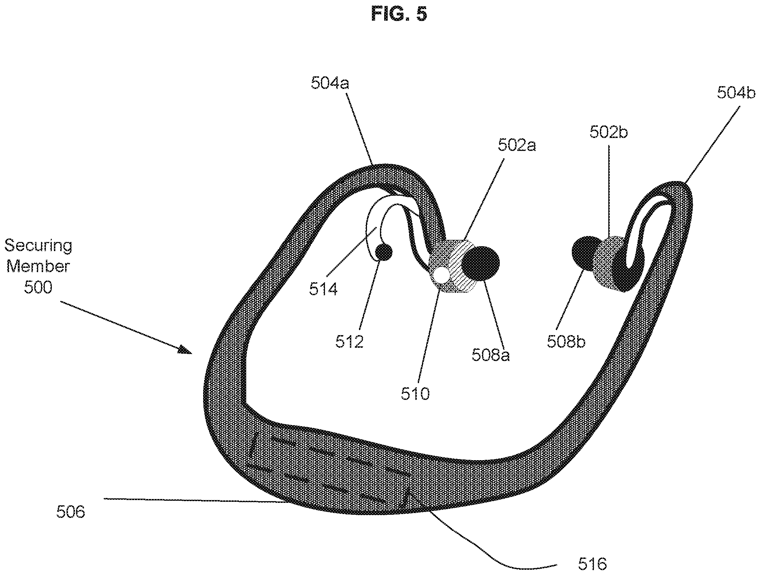

FIG. 5 depicts securing member 500 having a loop configuration of a type used for wireless headsets. Securing member 500 includes earpieces 502a and 502b, which fit into the left and right ears of a subject, respectively (e.g., fitting into one or both of the concha and external auditory meatus). Securing member 500 also includes arcs 504a and 504b, which fit over and behind the two ears of the subject, and connecting loop 506 which fits behind the head of the subject and connects earpieces 502a and 502b. In an aspect, securing member 500 is sufficiently rigid to maintain earpieces 502a and 502b in position in the ears of the subject while the subject moves about (e.g., walking or running). In an aspect, ear canal inserts 508a and 508b fit into the ear canals of the subject. A neural stimulator 510 may be positioned on earpiece 502a, as shown, or alternatively (or in addition) on ear canal extension 508a. A secondary neural stimulator 512 may be located on pinna extension 514. Extension 514 serves to position secondary neural stimulator 512 on the pinna of the subject at a desired location. In an aspect, extension 514 can be adjusted by elastic or plastic deformation to change the positioning of neural stimulator 512 on the pinna. In some aspects, extension 514 can include an adjustable linkage that provides for positioning of neural stimulator 512 with respect to the pinna.

FIG. 5 depicts a system in which neural stimulators 510 and 512 are positioned on securing member 500 so as to deliver stimulation to the left ear of the subject. Depending upon the desired application, neural stimulators can be positioned on one or both ears of the subject. In some aspects, stimulation is delivered to only one ear, while in other aspects, stimulation is delivered to both ears.

In some aspects, stimulator 512 located on pinna extension 514 can be used as the only, or primary neural stimulator, and stimulator 510 on earpiece 502a can be omitted. Earpieces 502a and 502b can function to hold securing member 500 in place with respect to the head of the subject, and, optionally, to deliver sound (such as a voice signal from a phone or music from an audio player) to the ears of the subject, independent of carrying stimulator 510. Circuitry 516 in securing member 506 includes communication circuitry for wirelessly communicating with other system components, for example a personal computing device (e.g., an audio player, a mobile phone, or a laptop computer). In addition, circuitry 516 may provide for wireless communication with a sensor located at a distance from securing member 500. For example, the wireless headset device depicted in FIG. 5 can be used in combination with sensors in one or more locations, not limited to sensors on securing member 500. Sensors include any type of physiological sensor located in, on or adjacent to the body of the subject (e.g., implanted sensors, sensors secured to the body, sensors in wearable items such as clothing, wristbands); remote sensors, environmental sensors, motion sensors, location sensors, and/or other types of sensors, without limitation.

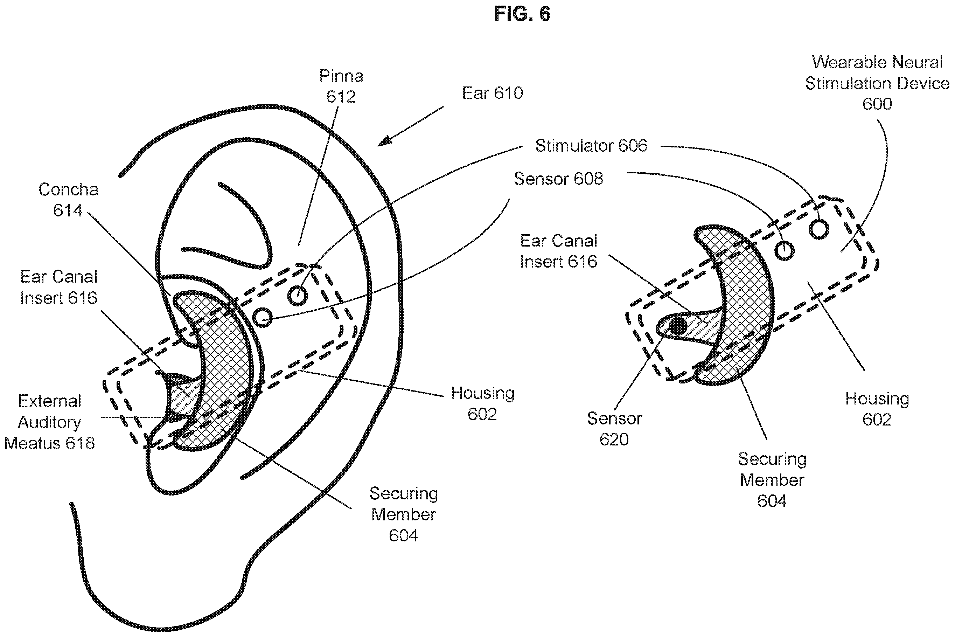

FIG. 6 depicts a further example of a wearable neural stimulation device 600 including a housing 602 attached to a securing member 604. Housing 602 is shown only in a dashed outline so that the position of stimulator 606 and sensor 608 with respect to ear 610 can be seen. Housing 602 is a thin, flat box-like structure, with stimulator 606 and sensor 608 mounted on the exterior of housing 602 on the side facing pinna 612. Housing 602 is fastened to or formed integrally with securing member 604. Securing member 604 fits into concha 614 to secure device 600 to ear 610. Ear canal insert 616 fits into external auditory meatus 618. Sensor 620 on ear canal insert 616 senses a physiological signal from external auditory meatus 618. Sensor 608 is an environmental sensor that senses light from the environment of the subject, e.g., to determine whether it is day or night.

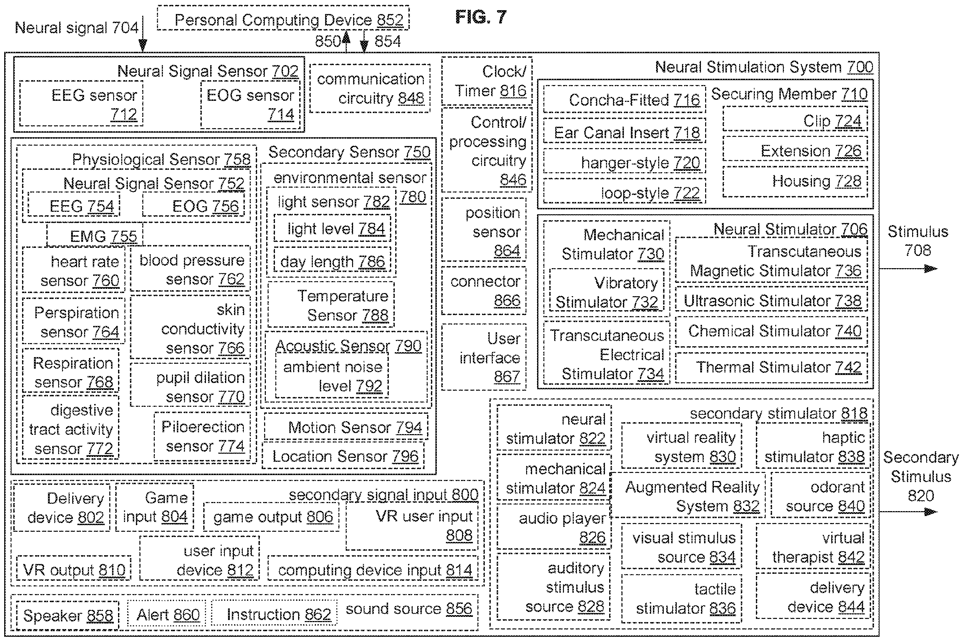

FIG. 7 is a block diagram of a neural stimulation system 700. Neural stimulation 700 system includes neural signal sensor 702, which is adapted to sense a neural signal 704 from a subject. Neural signal 704 may be an electroencephalographic (EEG) signal or electrooculographic (EOG) signal, and in an aspect is indicative of a physiological status of the subject. Neural stimulation system 700 also includes neural stimulator 706, which is adapted to produce a stimulus 708 responsive to sensed neural signal 704, stimulus 708 configured to activate at least one sensory nerve fiber innervating at least a portion of a pinna of the subject. Neural stimulation system 700 also includes securing member 710 configured to secure neural stimulator 706 to the pinna of the subject.

In various aspects, neural signal sensor 702 can be an electroencephalographic signal sensor 712 or electrooculographic signal sensor 714. Electroencephalographic signal sensor 712 can be configured to fit within an ear canal of a subject, e.g., on an ear canal insert as depicted in FIG. 4A (for example as described in U.S. Patent Publication 2003/0195588 to Fischell et al., or U.S. Patent Publication 2006/0094974 to Cain). EOG sensor 714 can be located on an extension (e.g., similar to extension 514 shown in FIG. 5) to position EOG sensor 714 on the subject's temple or side of the subject's head. An electromyographic signal sensor could be similarly placed. Physiological status of the subject, as indicated by neural signal 704, may include indications or symptoms of various types of physiological status, including various brain-related disorders or statuses, or other physiological statuses. Brain-related disorders include, for example, mental health disorders (e.g., psychological or psychiatric disorder), depression, post-traumatic stress disorder, seasonal affective disorder, anxiety, headache (e.g., primary headache, cluster headache, or migraine headache), or epilepsy). Neural signal sensor 704 may include other types of neural signal sensors, including external or implantable sensors, located in or on the ear or other part of the body. One or more neural signal sensors may be used.

In various aspects, securing member 710 is configured to secure neural stimulator 706 to different portions of the pinna of the subject. For example, in an aspect, securing member 710 includes a concha-fitted portion 716, configured to fit into the concha of the subject (e.g., as depicted in FIG. 3). In an aspect, securing member 710 includes an ear canal insert 718 configured to fit in the ear canal of the subject (e.g., as depicted in FIGS. 4A, 4B, and 5). In another aspect, securing member 710 is a hanger-style securing member 720, as depicted in FIGS. 4A and 4B. Hanger-style securing member 720 can be used to secure the neural stimulator to the back of the pinna, or to the surface of the head adjacent the pinna. In another aspect, securing member 710 is a loop-style securing member 722, (e.g., of the type depicted in FIG. 5). In another aspect, securing member 710 includes a clip 724 (e.g., of the type depicted in FIG. 3). A clip may be used to secure neural stimulator 706 to various parts of the front or back of the pinna, including the front or back of the ear lobe. In another aspect, securing member 720 includes an extension 726 (e.g., such as extension 514 depicted in FIG. 5). Such an extension can be used to position the neural stimulator in virtually any desired position on the pinna, or on the head adjacent to and above, below, in front of, or behind the ear. In an aspect, securing member 710 includes a housing 728. It should be noted that housing 710 may in some cases function as an extension. For example, housing 602 depicted in FIG. 6 also functions as an extension extending from securing member 604 to provide for placement and securing of stimulator 606 and sensor 608 on a portion of the pinna 612 not immediately adjacent securing member 604. Securing member 710 can be configured to secure the neural stimulator to the concha, tragus, front or back of the pinna, the helix, or various other parts of the pinna, e.g., the triangular fossa, antihelix, superior or inferior crus of the antihelix, antitragus, or tragus of the subject. In some aspects securing member 710 is permanently configured to position neural stimulator 706 in a particular position with respect to the ear of the subject, wherein in some aspects securing member 710 is adjustable such that the positioning of neural stimulator 706 can be selected by the subject. For example, a sensor or stimulator may be secured to a particular portion of the pinna by being pressed sufficiently firmly against the pinna by the securing member or extension to form a reliable mechanical or electrical contact with the pinna. In an aspect, securing member 710 includes a shape memory material. Various materials may be suitable for the construction of securing member 710, including but not limited to hard or soft, elastically or plastically deformable polymers, metals, ceramics, glasses, and composites formed therefrom. Flexible or stretchable electronic circuitry, formed from flexible materials or structures (e.g. conductors having, e.g., a serpentine design) or resilient conductive materials such as conductive polymers can be used in sensors and stimulators that conform to the pinna. While discussion herein has focused on positioning of the neural stimulator by securing member 710, it will be appreciated that securing member 710 can also be configured to position sensors with respect to the ear in a similar fashion. Several such examples are provided in FIGS. 3 - 6.

In an aspect, the neural stimulator 706 is positioned with respect to securing member 710 such that when securing member 710 is worn on the pinna, neural stimulator 706 is positioned (secured) over a specific region of the pinna, e.g., a region of the pinna innervated by a cranial nerve, e.g., the vagus nerve, the facial nerve, the trigeminal nerve, or the glossopharyngeal nerve. Such positioning may be selected based upon knowledge of the innervation of the pinna, for example, as provided in references texts such as Cranial Nerves in Health and Disease, by Linda Wilson-Pauwels, Elizabeth J. Akesson, Patricia A. Stewart, and Sian D. Spacey; BC Decker Inc.; 2 edition (January 1, 2002); ISBN-10: 1550091646/ISBN-13: 978-1550091649).

As noted above, neural stimulator 706 may be, for example, a mechanical stimulator 730 (e.g., a vibratory mechanical stimulator 732), a transcutaneous electrical stimulator 734, a transcutaneous magnetic stimulator 736, an ultrasonic stimulator 738, a chemical stimulator 740, a thermal stimulator 742, or other type of stimulator.

As shown in FIG. 7, in an aspect, neural stimulation system 700 includes at least one secondary sensor 750. In an aspect, neural signal sensor 702 is a primary neural signal sensor, and secondary sensor 750 is a secondary neural signal sensor 752, which may be, for example, an electroencephalographic (EEG) sensor 754, or electrooculographic (EOG) sensor 756. The secondary neural signal sensor 752 may be of the same or different type as primary neural signal sensor 702, and may be located at the same or different location on the body as primary neural signal sensor 702. In an aspect, secondary sensor 750 is a physiological sensor 758, for example, an electromyographic (EMG) sensor 755, a heart rate sensor 760 (which may be used to heart rhythm variability, as well as heart rate, and may include, but is not limited to, and EKG or pulse-oximeter based heart rate sensor), blood pressure sensor 762, perspiration sensor 764, skin conductivity sensor 766, respiration sensor 768, pupil dilation sensor 770, digestive tract activity sensor 772, or piloerection sensor 774. In another aspect, secondary sensor 750 is an environmental sensor, for example a light sensor 782, which may be configured to sense light level 784 and or day length 786. Environmental sensor 750 may include a temperature sensor 788, or an acoustic sensor 790, e.g., configured to sense ambient noise level 792. Other types of sensors for providing information regarding the state of the subject and his or her environment may be used, without limitation, including motion sensor 794 or location sensor 796, for example. A variety of physiological and environmental sensors are described in U. S. Patent 8,204,786 to LeBoueuf et al. Digestive tract activity may be sensed with external acoustical sensors, for example as described in "New disposable biosensor may help physicians determine which patients can safely be fed following surgery," MedicalXpress, August 7, 2014.

In an aspect, neural stimulation system 700 includes a secondary signal input 800. In various aspects, the signal received at secondary signal input 800 includes a signal from a delivery device 802 (indicative of delivery of a drug or nutraceutical to the subject), an input to a game 804 (e.g., a signal corresponding to the subjects input to a video game played by the subject), an output from a game 806 (e.g., a signal output by a game system indicative of a state of or an event in a game played by the subject), a user input to a virtual reality system 808, an output from a virtual reality system 810 (e.g., a signal output by the VR system indicative of an state of or an event in the VR system), a user input device 812 (e.g., a user input device of a computing device or a user input to the neural stimulation system), or a computing device input 814 (e.g., a data input). Inputs received via a user input device or computing device input may be indicative of intake of a food item, beverage, nutraceutical, or pharmaceutical by the subject, for example. Inputs received via a user input device may be provided by the subject, or by another user, e.g. a medical caregiver. Inputs may be provided spontaneously by the user, or in response to a prompt or query. In an aspect, inputs may be provided by the user in response to queries or prompts that form a part of a quiz, questionnaire, or survey, including, e.g. questions presented in yes/no or multiple choice response format. User responses provided in response to such prompts or queries may indicate the subject's mental or emotional state. Inputs received via a data input may include, for example, health-related information of the subject, including genome information or microbiome information of the subject, information from medical-records of the subject, or other information pertaining to the health of the subject.

In an aspect, neural stimulation system 700 includes a clock or timer 816. In various aspects, neural stimulator 706 is adapted to produce stimulus 708 based at least in part on a time of day indicated by clock/timer 816, and/or based at least in part on a date indicated by clock/timer 816.

Data drawn from one or more neural signals, physiological signals, environmental signals, or other secondary signals (e.g. obtained with secondary sensor 750 in FIG. 7) or secondary inputs (e.g. secondary signal input 800 in FIG. 7), as well as clock or timer information, can be correlated with a mental or emotional state of the subject, reported to a medical care provider or other party, and/or stored in the subj ect's medical or health records. In particular, values of any such parameters that are indicative of worsening mental or physical/physiological status of the subject can be reported to a medical care provider so that an appropriate intervention can be made, and/or used as a basis for modulating the delivery of neural stimulation.

In various aspects, neural stimulation system 700 includes at least one secondary stimulator 818 for delivery a secondary stimulus 820 to the subject. In an aspect, secondary stimulator 818 is a secondary neural stimulator 822, which may be any of the various types of neural stimulators described in connection with neural stimulator 706, and which may be of the same or different type as neural stimulator 706. Alternatively, secondary stimulator 818 may include a mechanical stimulator 824, an audio player 826, an auditory stimulus source 828, a virtual reality system 830, an augmented reality system 832, a visual stimulus source 834, a tactile stimulator 836, a haptic stimulator 838, an odorant source 840, a virtual therapist, or a delivery device 844, for delivering a drug or nutraceutical, for example.

In various aspects, neural stimulation system 700 includes control circuitry 846 carried by securing member 710 (either directly on securing member 710, or on an extension or housing connected to securing member 710, e.g., as depicted in FIGS. 3 - 6), the control circuitry 846 configured to control neural stimulator 706.

In an aspect, neural stimulation system 700 includes communication circuitry 848 carried by securing member 710 and configured for at least one of sending one or more signal 850 to a personal computing device 852 and receiving one or more signal 854 from personal computing device 852.

In an aspect, neural stimulation system 700 includes a sound source 856, for delivering an auditory signal to the subject. Sound source 856 may be, for example, a speaker 858. Sound source 856 may be configured (e.g., with appropriate electronic circuitry, not shown) to delivery an instruction 860 or alert 862 to the subject.

In an aspect, neural stimulation system 700 includes position sensor 864 for sensing the position of neural stimulator 706 with respect to the pinna of the subject. Position sensor 864 may detect the position of neural stimulator 706 with respect to the pinna by detecting electrical activity from a nerve, by detecting an image of the ear and determining the position based on landmarks in the image, or by detecting a temperature, pressure, or capacitive signal indicative of adequate contact of the stimulator with the ear, for example.

In an aspect, neural stimulation system 700 includes connector 866 for connecting the neural stimulator to a personal computing device. Connector 866 includes, for example, a jack or port for creating a wired (cable) connection with the personal computing device. In an aspect, neural stimulation system 700 includes user interface 867 for receiving input from the subject or presenting information to the subject. In an aspect, user interface 867 includes a small display, one or more indicator lights and simple user inputs, such as one or more buttons or dials for adjusting device setting and viewing and modifying system settings.

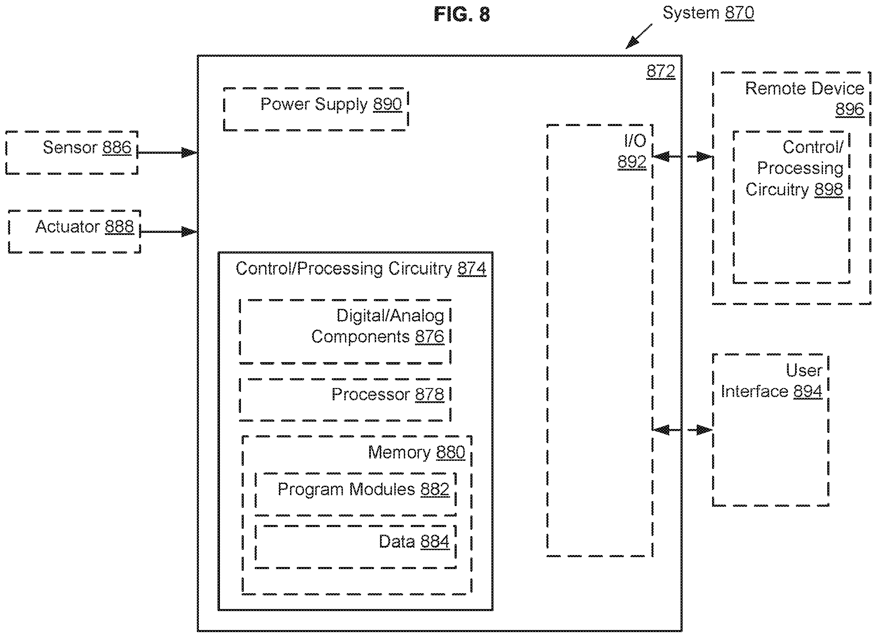

FIG. 8 illustrates a generalized form of circuitry-based systems as depicted in FIG. 7 and elsewhere herein. Although specific embodiments are described herein, those skilled in the art will appreciate that methods and systems as described herein can be implemented in various ways. Reference is made herein to various circuitry systems and subsystems (e.g., neural stimulation system 700 includes control/processing circuitry 846 in FIG. 7, which may be considered to be control/processing circuitry. As shown generically in FIG. 8, a system 870 includes a circuitry-based system 872. Circuitry-based system 872, which in some aspects is a computing device or computing subsystem, includes control/processing circuitry 874, which includes any or all of digital and/or analog components 876, one or more processor 878 (e.g., a microprocessor), and memory 880, which may store one or more program module 882 and/or data 884. In some aspects, control/processing circuitry provides for preliminary handling of data from one or more sensor 886, transfer of data to remote device 896, receipt of control signal from remote device 896, and actuation of actuator 888, which may be for example a neural stimulator (such as neural stimulator 706 as shown in FIG. 7). Systems as described herein may receive signals from various sensors (e.g., sensor 886 depicted in FIG. 8). System 870 may include other components as known to those skilled in the art, e.g., one or more power supply 890, I/O structure 892, clock, timer, data bus, etc. I/O structure 892 permits communication with various types of user interface devices (represented by user interface 894, which may include one or more input devices such as a keyboard, button, switch, computer mouse, or touchscreen or one or more output devices such as screen, sound source, alphanumeric display, Braille display, etc.) and communication with various types of remote device 896, e.g., remote system 216 in FIGS. 2A-2B, which may have control/ processing capability conferred by control/ processing circuitry 898.

In a general sense, the various embodiments described herein can be implemented, individually and/or collectively, by various types of electrical circuitry having a wide range of electrical components such as hardware, software, firmware, and/or virtually any combination thereof. Electrical circuitry (including control/processing circuitry 846 in FIG. 7, for example) includes electrical circuitry having at least one discrete electrical circuit, electrical circuitry having at least one integrated circuit, electrical circuitry having at least one application specific integrated circuit, electrical circuitry forming a computing device configured by a computer program (e.g., a computer configured by a computer program which at least partially carries out processes and/or devices described herein, or a microprocessor configured by a computer program which at least partially carries out processes and/or devices described herein), electrical circuitry forming a memory device, which may include various types of memory (e.g., random access, flash, read only, etc.)), electrical circuitry forming a communications device (e.g., communication circuitry 848 in FIG. 7) (e.g., a modem, communications switch, optical-electrical equipment, etc.), and/or any non-electrical analog thereto, such as optical or other analogs (e.g., graphene based circuitry). In an embodiment, the system is integrated in such a manner that the system operates as a unique system configured specifically for function of the neural stimulation system described herein. In an embodiment, one or more associated computing devices of the system operate as specific use computers for purposes of the claimed system, and not general use computers. In an embodiment, one or more of the associated computing devices of the system are hardwired with a specific ROM to instruct the one or more computing devices.

In a general sense, the various aspects described herein which can be implemented, individually and/or collectively, by a wide range of hardware, software, firmware, and/or any combination thereof can be viewed as being composed of various types of "electrical circuitry. "

At least a portion of the devices and/or processes described herein can be integrated into a data processing system. A data processing system generally includes one or more of a system unit housing, a video display, memory such as volatile or non-volatile memory, processors such as microprocessors or digital signal processors, computational entities such as operating systems, drivers, graphical user interfaces, and applications programs, one or more interaction devices (e.g., a touch pad, a touch screen, an antenna, etc.), and/or control systems including feedback loops and control motors (e.g., feedback for sensing position and/or velocity; control motors for moving and/or adjusting components and/or quantities). A data processing system may be implemented utilizing suitable commercially available components, such as those typically found in data computing/communication and/or network computing/communication systems.

In various embodiments, methods as described herein may be performed according to instructions implementable in hardware, software, and/or firmware. Such instructions may be stored in non-transitory machine-readable data storage media, for example. The state of the art has progressed to the point where there is little distinction left between hardware, software, and/or firmware implementations of aspects of systems; the use of hardware, software, and/or firmware is generally (but not always, in that in certain contexts the choice between hardware and software can become significant) a design choice representing cost vs. efficiency tradeoffs. There are various vehicles by which processes and/or systems and/or other technologies described herein can be effected (e.g., hardware, software, and/or firmware), and that the preferred vehicle will vary with the context in which the processes and/or systems and/or other technologies are deployed. For example, if an implementer determines that speed and accuracy are paramount, the implementer may opt for a mainly hardware and/or firmware vehicle; alternatively, if flexibility is paramount, the implementer may opt for a mainly software implementation; or, yet again alternatively, the implementer may opt for some combination of hardware, software, and/or firmware in one or more machines, compositions of matter, and articles of manufacture. Hence, there are several possible vehicles by which the processes and/or devices and/or other technologies described herein may be effected, none of which is inherently superior to the other in that any vehicle to be utilized is a choice dependent upon the context in which the vehicle will be deployed and the specific concerns (e.g., speed, flexibility, or predictability) of the implementer, any of which may vary. Optical aspects of implementations will typically employ optically-oriented hardware, software, and or firmware.

In some implementations described herein, logic and similar implementations may include software or other control structures. Electrical circuitry, for example, may have one or more paths of electrical current constructed and arranged to implement various functions as described herein. In some implementations, one or more media may be configured to bear a device-detectable implementation when such media hold or transmit device detectable instructions operable to perform as described herein. In some variants, for example, implementations may include an update or modification of existing software or firmware, or of gate arrays or programmable hardware, such as by performing a reception of or a transmission of one or more instructions in relation to one or more operations described herein. Alternatively or additionally, in some variants, an implementation may include special-purpose hardware, software, firmware components, and/or general-purpose components executing or otherwise invoking special-purpose components.

Implementations may include executing a special-purpose instruction sequence or invoking circuitry for enabling, triggering, coordinating, requesting, or otherwise causing one or more occurrences of virtually any functional operations described herein. In some variants, operational or other logical descriptions herein may be expressed as source code and compiled or otherwise invoked as an executable instruction sequence. In some contexts, for example, implementations may be provided, in whole or in part, by source code, such as C++, or other code sequences. In other implementations, source or other code implementation, using commercially available and/or techniques in the art, may be compiled/ /implemented/translated/converted into a high-level descriptor language (e.g., initially implementing described technologies in C or C++ programming language and thereafter converting the programming language implementation into a logic-synthesizable language implementation, a hardware description language implementation, a hardware design simulation implementation, and/or other such similar mode(s) of expression). For example, some or all of a logical expression (e.g., computer programming language implementation) may be manifested as a Verilog-type hardware description (e.g., via Hardware Description Language (HDL) and/or Very High Speed Integrated Circuit Hardware Descriptor Language (VHDL)) or other circuitry model which may then be used to create a physical implementation having hardware (e.g., an Application Specific Integrated Circuit).

This detailed description sets forth various embodiments of devices and/or processes via the use of block diagrams, flowcharts, and/or examples. Insofar as such block diagrams, flowcharts, and/or examples contain one or more functions and/or operations, each function and/or operation within such block diagrams, flowcharts, or examples can be implemented, individually and/or collectively, by a wide range of hardware, software, firmware, or virtually any combination thereof. In an embodiment, several portions of the subject matter described herein may be implemented via Application Specific Integrated Circuits (ASICs), Field Programmable Gate Arrays (FPGAs), digital signal processors (DSPs), or other integrated formats. However, some aspects of the embodiments disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, the mechanisms of the subject matter described herein are capable of being distributed as a program product in a variety of forms, and that an illustrative embodiment of the subject matter described herein applies regardless of the particular type of signal bearing medium used to actually carry out the distribution. Examples of a signal bearing medium include, but are not limited to non-transitory machine-readable data storage media such as a recordable type medium such as a floppy disk, a hard disk drive, a Compact Disc (CD), a Digital Video Disk (DVD), a digital tape, a computer memory, etc.. A signal bearing medium may also include transmission type medium such as a digital and/or an analog communication medium (e.g., a fiber optic cable, a waveguide, a wired communications link, a wireless communication link (e.g., transmitter, receiver, transmission logic, reception logic, etc.) and so forth).

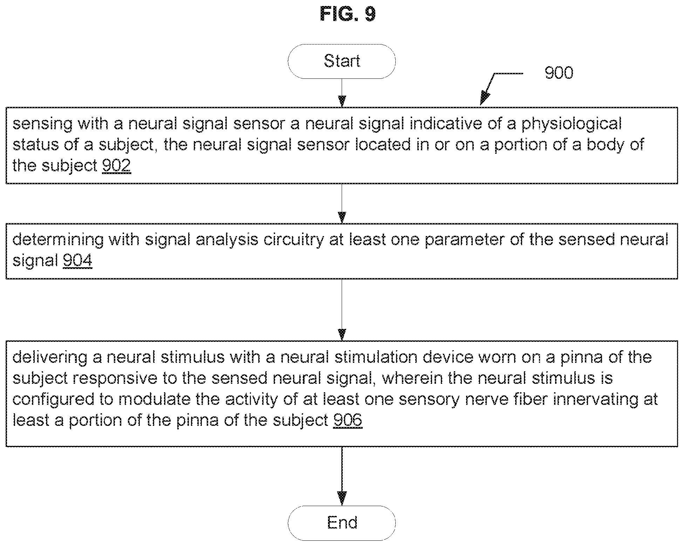

FIG. 9 is a flow diagram of a method 900 relating to use of a neural stimulation system as depicted in FIG. 7. Here and elsewhere, method steps outlined with dashed lines represent steps that are included in some, but not all method aspects, and combinations of steps other than those specifically depicted in the figures are possible as would be known by those having ordinary skill in the relevant art. Method 900 includes sensing with a neural signal sensor a neural signal indicative of a physiological status of a subject, the neural signal sensor located in or on a portion of a body of the subject, as indicated at 902; determining with signal analysis circuitry at least one parameter of the sensed neural signal, as indicated at 904; and delivering a neural stimulus with a neural stimulation device worn on a pinna of the subject responsive to the sensed neural signal, wherein the neural stimulus is configured to modulate the activity of at least one sensory nerve fiber innervating at least a portion of the pinna of the subject, as indicated at 906. In an aspect, the neural stimulus is of sufficient frequency and amplitude to modulate the activity of the at least one sensory nerve fiber innervating the at least a portion of the pinna of the subject. For example, in various aspects the neural stimulus has a frequency in the approximate range of 1 Hz -1000 Hz, 10 Hz - 500 Hz, 30 Hz - 40 Hz, 10 Hz - 50 Hz, 10 Hz - 80 Hz, 50 Hz - 100 Hz, or 200 - 300 Hz. In an aspect, the stimulus has a sinusoidal waveform. In other aspects, the stimulus may have a triangular, rectangular, square, trapezoidal, or other waveform, delivered cyclically, with cycle frequencies in the ranges listed above. It will be appreciated that depending on the stimulus waveform or pulse shape, or envelope shape, a given stimulus may include higher or lower frequencies. The neural stimulus may be delivered according to programmed pattern, which may be stored in memory on the neural stimulation device or on a personal computing device or other remote device in communication with the neural stimulation device. In various aspects, the neural stimulus is delivered continuously, intermittently, and/or in a time-varying fashion. The neural stimulus may be a pulsed stimulus.

In an aspect, the neural stimulus is delivered with a neural stimulation device and/or neural stimulus configured to activate a cranial nerve, such as the vagus nerve, facial nerve, trigeminal nerve, or glossopharyngeal nerve. The neural stimulation device can be configured to stimulate a particular nerve by one or both of positioning the neural stimulator on at least a portion of a receptive field of the nerve of interest, and selecting the amplitude and other stimulus parameters (e.g. frequency, waveform, duration) of the stimulus delivered to activate the nerve fibers in the nerve of interest.

In an aspect, the method includes delivering the neural stimulus responsive to the at least one parameter of the sensed neural signal. The at least one parameter may include, for example, a frequency content of an electroencephalographic signal, an amplitude of an electroencephalographic signal, a rate of eye movement determined from an electrooculogram, or a gaze direction determined from an electrooculogram. In some aspects, such parameters are indicative of a brain-related disorder, or symptoms thereof. In an aspect, method 900 includes delivering the neural stimulus in response to detection of symptoms of a brain-related disorder (which may be, for example, any mental health disorder (e.g., psychological or psychiatric disorder), depression, post-traumatic stress disorder, seasonal affective disorder, anxiety, headache (e.g., primary headache, cluster headache, or migraine headache), or epilepsy). In an aspect, the method includes delivering the neural stimulus until symptoms of the brain-related disorder are no longer detected.

In an aspect, method 900 includes sensing at least one secondary signal with a secondary sensor. In an aspect, delivery of the neural stimulus may be started, stopped, or modulated in response to the secondary signal. The secondary signal may be a secondary neural signal (of the same or different type and sensed from the same or from a different location than the primary neural signal), or it may another type of physiological signal, an environmental signal, a location signal, or a signal from a motion sensor, for example. Such secondary signals may provide additional information relevant for determining whether the neural stimulus should be applied, assessing the subject's response to the neural stimulus, identifying appropriate time of delivery of the neural stimulus, etc. The secondary signal may include other types of secondary signal, e.g., as received by secondary signal input 800 in FIG. 7. In an aspect, method 900 includes delivering at least one secondary stimulus to the subject in addition to the neural stimulus delivered with the neural stimulation device. The secondary stimulus may be any of various types of secondary stimulus, e.g., as delivered with secondary stimulator 818 as described in FIG. 7. In various aspects, method 900 includes controlling the neural stimulation device with control circuitry located at least in part on the neural stimulation device, or with control circuitry located at least in part on a personal computing device in communication with the neural stimulation device worn on the pinna of the subject. In an aspect, method 900 includes sending a signal from the neural stimulation device worn on the pinna of the subject to a personal computing device or receiving a signal from a personal computing device at the neural stimulation device worn on the pinna of the subject. In an aspect, method 900 includes delivering an auditory instruction or an auditory alert to the subject with a sound source operatively connected to the neural stimulation device. In an aspect, method 900 includes sensing a position of the neural stimulation device relative to the pinna of subject with a position sensor operatively connected to the neural stimulation device. If the neural stimulation device is not positioned properly positioned, the auditory instruction or alert may remind the subject to correct the positioning of the neural stimulation device. Alternatively, or in addition, visual alerts can be provided to the subject, in the form of one or more blinking light, graphic, or a text message, delivered via an LED or other light emitting element, an alphanumeric display, a screen, or other display element on the neural stimulation device or on the personal computing device.

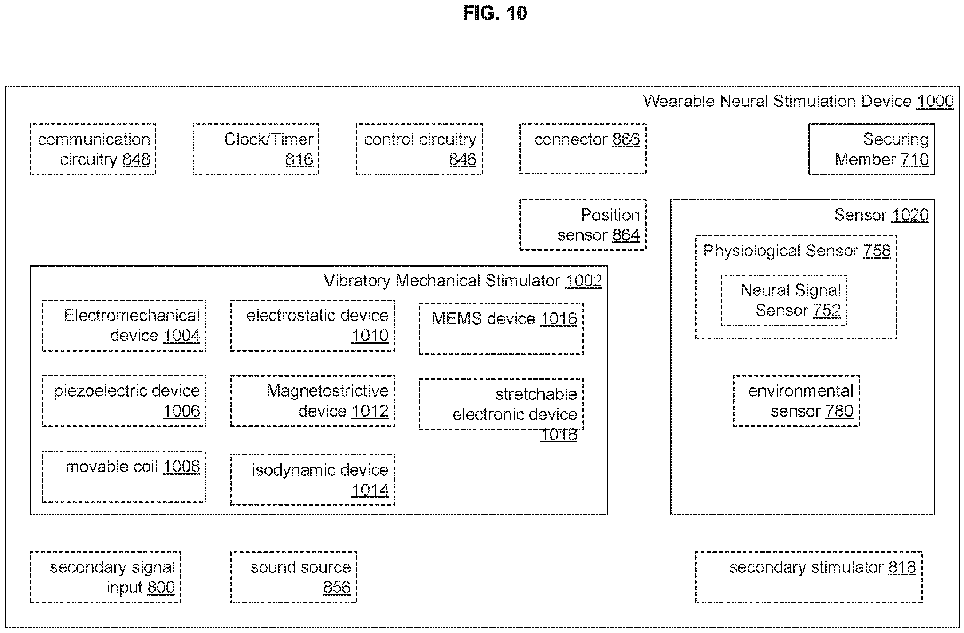

FIG. 10 depicts an embodiment of a wearable neural stimulation device 1000 that includes a vibratory mechanical stimulator 1002. Vibratory mechanical stimulator 1002 is adapted to produce a vibratory stimulus of sufficient frequency and amplitude to modulate the activity of at least one mechanoreceptor with a receptive field on at least a portion of a pinna of a subject, and a securing member 710 configured to secure vibratory mechanical stimulator 1000 to the pinna. Securing member 710 is as described herein above. Vibratory mechanical stimulator 1002 is a vibratory stimulator, such as vibratory stimulator 732 described generally in connection with FIG. 7. In various aspects, vibratory mechanical stimulator 1002 includes an electromechanical device 1004, piezoelectric device 1006, movable coil 1008, electrostatic device 1010, magnetostrictive device 1012, isodynamic device 1014, a MEMS device 1016, and/or a stretchable electronic device 1018.

In an aspect, neural stimulation device 1000 includes at least one sensor 1020, which may be any of the various types of sensors described in connection with secondary sensor 750 in FIG. 7, e.g., a physiological sensor 758, a neural signal sensor 752, an environmental sensor 780, a motion sensor 794 or a location sensor 796. In various aspects, neural stimulation device 1000 includes a secondary signal input 800, secondary stimulator 818, control circuitry 846 carried by securing member 710, communication circuitry 848, sound source 856, position sensor 864, and connector 866, all of which have been discussed in connection with FIG. 7.

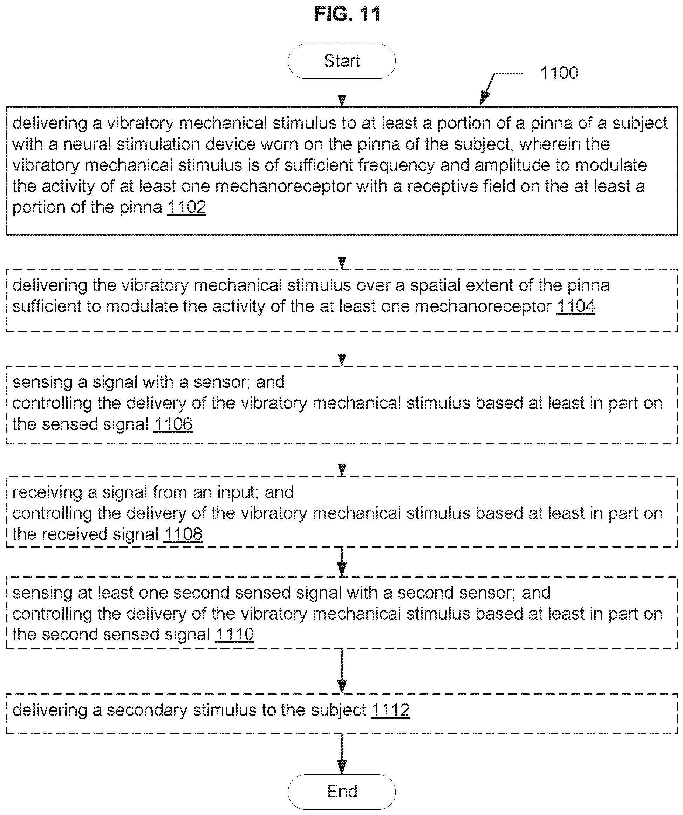

FIG. 11 is a flow diagram of a method 1100 relating to use of a neural stimulation system as depicted in FIG. 10. In an aspect, method 1100 includes delivering a vibratory mechanical stimulus to at least a portion of a pinna of a subject with a neural stimulation device worn on the pinna of the subject, wherein the vibratory mechanical stimulus is of sufficient frequency and amplitude to modulate the activity of at least one mechanoreceptor with a receptive field on the at least a portion of the pinna, as indicated at 1102. In an aspect, method 1100 includes delivering the vibratory mechanical stimulus over a spatial extent of the pinna sufficient to modulate the activity of the at least one mechanoreceptor, as indicated at 1104.

In an aspect, the vibratory mechanical stimulus has a waveform sufficient to modulate the activity of the at least one mechanoreceptor with a receptive field on the at least a portion of the pinna. For example, the vibratory mechanical stimulus may have a sinusoidal or other waveform. In some aspects, the vibratory mechanical stimulus is delivered according to programmed pattern, which may include delivering the vibratory mechanical stimulus either continuously or intermittently.

In an aspect, as indicated at 1106, method 1100 includes sensing a signal with a sensor and controlling the delivery of the vibratory mechanical stimulus based at least in part on the sensed signal. The sensed signal may be any of the various types of signal sensed with sensor 1018 in FIG. 10. In various aspects, controlling delivery of the vibratory mechanical stimulus based at least in part on the sensed signal includes modulating delivery of the neural stimulus in response to the sensed signal, or delivering the vibratory mechanical stimulus in response to the sensed signal. In an aspect, controlling the delivery of the vibratory mechanical stimulus based at least in part on the sensed signal includes initiating delivery of the vibratory mechanical stimulus in response to the sensed signal.

In an aspect, method 1100 includes receiving a signal from an input and controlling the delivery of the vibratory mechanical stimulus based at least in part on the received signal, as indicated at 1108. The received signal may be e.g., any of the various types of input signals received at secondary signal input 800 in FIG. 10.

In an aspect, method 1100 includes sensing at least one second sensed signal with a second sensor and controlling the delivery of the vibratory mechanical stimulus based at least in part on the second sensed signal, as indicated at 1110.

In an aspect, method 1100 also includes delivering a secondary stimulus to the subject, as indicated at 1112, which may include delivering a secondary stimulus with a secondary stimulator 818, as described in connection with FIG. 7.

As discussed in connection with method 900, the vibratory mechanical stimulus can be delivered in response to detection of symptoms of a brain-related disorder, which may include, for example, a mental health disorder, depression, post-traumatic stress disorder, seasonal affective disorder, anxiety, headache, or epilepsy. In an aspect, method 1100 includes delivering the vibratory mechanical stimulus until symptoms of the brain-related disorder are no longer detected.

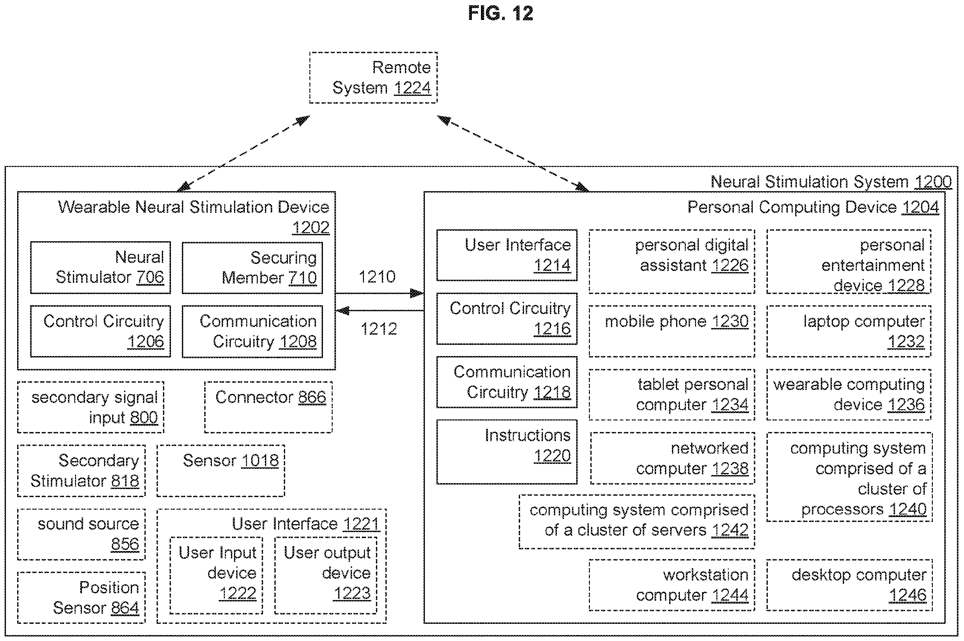

FIG. 12 depicts a neural stimulation system 1200 which includes a wearable neural stimulation device 1202 and personal computing device 1204. Personal computing device 1204 may be packaged separately from wearable neural stimulation device 1202, e.g., similar to the system depicted in FIGS. 2A and 2B. Wearable neural stimulation device 1202 includes neural stimulator 706, which is adapted to produce a stimulus for activating at least one sensory nerve fiber innervating at least a portion of a pinna of a subject, securing member 710 configured to secure the neural stimulator to the pinna, control circuitry 1206 for controlling operation of neural stimulator 706, and first communication circuitry 1208. Neural stimulator 706 and securing member 710 are as described herein above in connection with FIG. 7. Both control circuitry 1206 and first communication circuitry 1208 are incorporated into the wearable neural stimulation device 1202. First communication circuitry 1208 is operatively connected to control circuitry 1206 and is configured for at least one of sending a signal 1210 to and receiving a signal 1212 from personal computing device 1204. Other system components that may be included in or used in connection with wearable neural stimulation device 1202 include secondary signal input 800, secondary stimulator 818, sound source 856, position sensor 864 and connector 866, as described herein above in connection with FIG. 7, and sensor 1018 as described herein above in connection with FIG. 10. In an aspect, neural stimulation system 1200 includes user interface 1221, including user input device 1222 which is used to receive an input from the subject or other user, and user output device 1223. User input device 1222 may be any of various types of user input devices known to those of ordinary skill in the art, including but not limited to a button, keyboard, keypad, touchscreen, voice input, etc. In system 1200 and in other neural stimulation systems described herein, system components such as secondary signal input 800, secondary stimulator 818, sound source 856, position sensor 864, connector 866, sensor 1018, and user input device 1221 may in some cases be built into the wearable neural stimulation device (e.g., wearable neural stimulation device 1202) and in some cases be packaged separately but used in combination with the wearable neural stimulation device. For example, sensors may be located on the subject's body at a location other than the ear, or in the vicinity of the subject but not on the subject's body. In some cases, sensors may be implanted within the subject's body. Similarly, one or both of a secondary stimulator and a sound source can be located on the wearable neural stimulation device, on the subject's body distinct from the neural stimulation device, or in the vicinity of the subject but not on the subject's body.

Personal computing device 1202 includes a user interface 1214 for at least one of presenting information to and receiving information from a user, control circuitry 1216 operatively connected to user interface 1214, and second communication circuitry 1218 configured for at least one of sending a signal to and receiving a signal from the first communication circuitry 1208 carried by the housing of the wearable neural stimulation device. In addition, personal computing device 1202 includes instructions 1220 that when executed on personal computing device 1204 cause personal computing device 1204 to perform at least one of sending signal 1212 to and receiving signal 1210 from wearable neural stimulation device 1202 via second communication circuitry 1218.

Communication circuitry 1208 and communication circuitry 1218 provide for communication between wearable neural stimulation device 1202 and personal computing device 1204. In addition, in some aspects one or both of communication circuitry 1208 and communication circuitry 1218 provide for communication of wearable neural stimulation device 1202 or personal computing device 1204, respectively, with a remote system 1224. In some aspects, communication circuitry 1208 and communication circuitry 1218 provide for wired communication between wearable neural stimulation device and personal computing device 1204. Wired communication to wearable neural stimulation device may occur via connector 866. Alternatively, or in addition, a wireless communication link may be established between wearable neural stimulation device 1202 and personal computing device 1204, and/or between either wearable neural stimulation device 1202 or personal computing device 1204 and remote system 1224. In various aspects, a wireless communication link includes at least one of a radio frequency, wireless network, cellular network, satellite, WiFi, BlueTooth, Wide Area Network, Local Area Network, or Body Area Network communication link. Various types of communication links are suitable for providing communication between two remote locations. Communication between locations remote from each other may take place over telecommunications networks, for example public or private Wide Area Network (WAN). In general, communication between remote locations is not considered to be suitably handled by technologies geared towards physically localized networks, e.g., Local Area Network (LAN) technologies operation at Layer 1/2 (such as the forms of Ethernet or WiFi). However, it will be appreciated that portions (but not the entirety) of communication networks used in remote communications may include technologies suitable for use in physically localized network, such as Ethernet or WiFi.

In an aspect, personal computing device 1204 is personal digital assistant 1226, a personal entertainment device 1228, a mobile phone 1230, a laptop computer 1232, a tablet personal computer 1234, a wearable computing device 1236 (e.g., a fitness band, an item of clothing, attire, or eyewear incorporating computing capability), a networked computer 1238, a computing system comprised of a cluster of processors 1240, a computing system comprised of a cluster of servers 1242, a workstation computer 1244, and/or a desktop computer 1246. In various aspects, personal computing device 1204 includes one or more of a portable computing device, a wearable computing device, a mobile computing device, and a thin client computing device, for example.

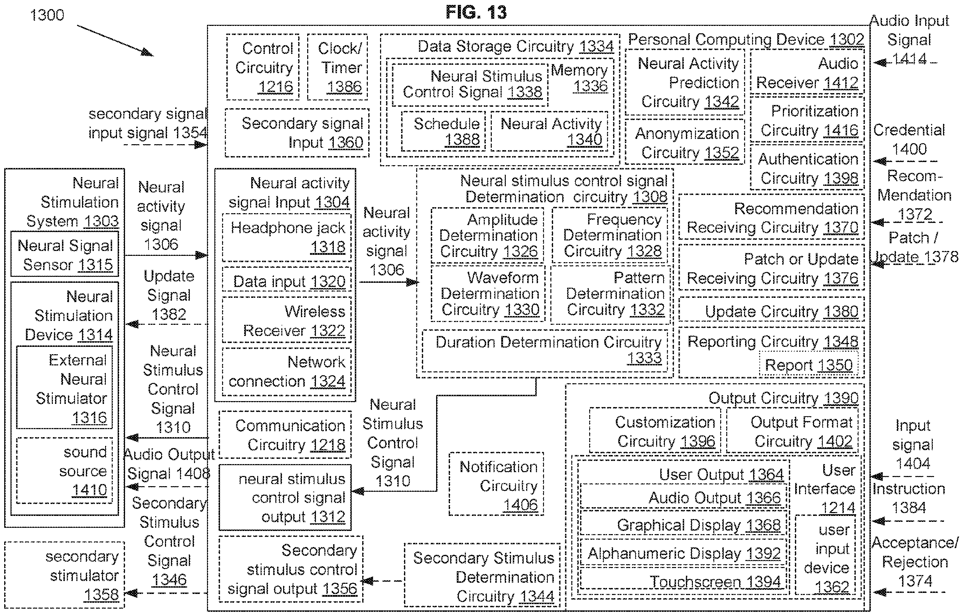

FIG. 13 depicts aspects of a system 1300 including personal computing device 1302, for use in connection with neural stimulation system 1303, which is a neural stimulation system such as described herein above. Personal computing device 1302 is as described generally in connection with FIG. 12. In an aspect, personal computing device 1302 includes circuitry 1304 for receiving a neural activity signal 1306, circuitry 1308 for determining a neural stimulus control signal 1310 based at least in part on neural activity signal 1306, and circuitry 1312 for outputting neural stimulus control signal 1310 to neural stimulation device 1314. In an aspect, neural activity signal 1306 is sensed by neural signal sensor 1315, and is indicative of a physiological status of a subject. Neural activity signal 1306 may be an unprocessed neural signal, or neural activity signal 1306 may have been subjected to various types and amounts of signal processing, and/or analysis (including, but not limited to filtering, amplification, analog to digital conversion, signal averaging, conversion from time to frequency domain, feature extraction, and so forth). Neural activity signal 1306 may include neural activity sensed from one or more neural signal sensors 1315 (which may be electroencephalographic sensors or electrooculographic sensors, for example). Neural activity signal 1306 may include information derived from or associated with the sensed neural signal, and may include or be accompanied by additional information that identifies the type of signal, type of processing to which the signal has been subject, data formatting, device settings used during acquisition of the neural signal, etc.. Neural signal sensor 1315 is a component of neural stimulation system 1303, and may be a component of neural stimulation device 1314, or used in association therewith, as described herein above. Neural stimulation device 1314 includes external neural stimulator 1316, which is configured to be carried on a pinna of the subject. Neural stimulus control signal 1310 is configured to control delivery of a neural stimulus by external neural stimulator 1316, the neural stimulus configured to activate at least one sensory nerve fiber innervating at least a portion of the pinna.

Neural activity signal input 1304 (the circuitry for receiving neural activity signal 1306) includes, for example, a headphone jack 1318, data input 1320, wireless receiver 1322, or network connection 1324. In various aspects neural activity signal input 1304 includes circuitry for receiving a signal from a body area network, a local area network, or a wide area network.

Neural stimulus control signal determination circuitry 1308 includes one or more of amplitude determination circuitry 1326 for determining a neural stimulus amplitude, frequency determination circuitry 1328 for determining a neural stimulus frequency, waveform determination circuitry 1330 for determining a neural stimulus waveform, pattern determination circuitry 1332 for determining a neural stimulus pattern, or duration determination circuitry 1333 for determining a neural stimulus duration. In an aspect, personal computing device 1302 includes data storage circuitry 1334 for storing data on the data storage device, including memory 1336 and circuitry for accessing data stored therein. Memory 1336 may contain stored preprogrammed stimulus patterns and waveforms as well as neural stimulus parameter values from which neural stimuli can be computed. In an aspect, system 1300 includes data storage circuitry 1334 for storing data on personal computing device 1302 representing neural stimulus control signal 1338. In an aspect, system 1300 includes data storage circuitry 1334 for storing data on personal computing device 1302 representing previous neural activity 1340. In an aspect, neural activity prediction circuitry 1342 predicts a future neural activity signal based on a previous neural activity signal.

In an aspect, system 1300 includes secondary stimulus determination circuitry 1344 for determining a secondary stimulus based on neural activity signal 1306. In an aspect, secondary stimulus determination circuitry 1344 determines the secondary stimulus control signal 1346 based on previous neural activity signal 1340.

In an aspect, system 1300 includes reporting circuitry 1348 for providing a report 1350 to at least one recipient. Reporting circuitry 1348 may cause report 1350 to be provided via a user interface 1214 (as described in connection with FIG. 12) or via a computing network (accessed via communication circuitry 1218). In an aspect, report 1350 is provided to the subject using the neural stimulation device 1314. In another aspect, report 1350 is provided to other parties, for example, a medical care provider, an insurance company, a service provider (e.g., a business or other entity that provides services related to the neural stimulation device or related to monitoring use of the neural stimulation device). In an aspect, report 1350 is provided to at least one social media contact (or 'friend'), or to a peer of the subject, e.g., via a social network. In an aspect, the recipient is a computing system, e.g. a computing system used for storing and/or processing healthcare information. In various aspects, anonymization circuitry 1352 is used to provide the report in anonymized form (e.g., with information identifying the subject removed therefrom). Reporting circuitry 1326 may include circuitry for including various information in report 1350, e.g., information relating to one or more of neural activity signal 1306 or information derived therefrom, neural stimulus control signal 1310, settings for neural stimulation device 1314 or personal computing device 1302, stored neural activity data 1340, secondary input signal 1354, and secondary stimulus control signal 1346. In an aspect, system 1300 includes secondary stimulus control signal output circuitry 1356 for delivering secondary stimulus control signal 1346 to secondary stimulator 1358. Secondary stimulator 1358 can be any type of stimulator, for example such as secondary stimulator 818 described in connection with FIG. 7.

In an aspect, system1300 includes secondary signal input 1360 for receiving a secondary input signal 1354 at personal computing device 1302. In an aspect, neural stimulus control signal determination circuitry is configured to determine neural stimulus control signal 1310 based at least in part on secondary input signal 1354. Secondary input signal may be representative of a physiological parameter of the subject or an environmental parameter of the subject, and may include a signal sensed from a sensor on or associate with neural stimulation device 1314, or a sensor in the environment of the subject, and/or parameters or values derived from such sensed signals. In an aspect, the secondary input signal is indicative of a user input provided by the subject. In an aspect, secondary input signal 1354 may be received via user input 1362 in user interface 1214.

In an aspect, system 1300 includes circuitry for presenting a recommendation to the subject. The recommendation may be presented to the subject via user output 1364 of user interface 1214, e.g., via audio output 1366 and/or graphical display 1368 or transmitted to neural stimulation device 1303 and presented via a user interface on neural stimulation device 1303. In an aspect, system 1300 includes recommendation receiving circuitry 1370 for receiving recommendation 1372 at personal computing device 1302. For example, in an aspect recommendation receiving circuitry 1370 receives recommendation 1372 via a computing network. In various aspects, recommendation 1372 is received from a medical care provider, from an insurance company, a service provider, an advisor, a computation-based system (including, e.g. an artificial intelligence), or a social media source, for example. In various aspects, recommendation receiving circuitry 1370 is configured to receive recommendations from particular sources, e.g. by receiving along with the recommendation a code indicating the source of the recommendation (e.g., a specific medical care provider, a medical care provider as opposed to a social media source), and to recognize a source of the recommendation and respond differently depending upon the source of the recommendation. Recommendation receiving circuitry 1370 may be configured such that recommendations from more credible sources may presented to the subject more promptly or more prominently, whereas recommendations from undesirable sources may be blocked, for example. Recommendation 1372 may relate to a configuration of neural stimulus control signal 1319 or secondary stimulus control signal 1346. In other aspects, recommendation 1372 relates to one or more of a consumer product, a service, a user experience, a user activity, or an organization that may be of interest to the subject, e.g., because the recommendations would enhance or be compatible with the effects of the neural stimulation received by the subject, or in some other manner relate to the neural stimulation or the condition which it is intended to treat. For example, the recommendation might be for software for storing, presenting, sharing, or reporting stimulation data or health data or for an organization that provides counseling to individuals with a particular condition. In an aspect, user input 1362 is configured to receive acceptance/rejection signal 1374 from the subject regarding acceptance or rejection of recommendation 1372.

In an aspect, system 1300 includes patch or update receiving circuitry 1376 for receiving patch/update 1378 at personal computing device 1302. Patch/update 1378 includes a software patch or update for software residing on personal computing device 1302 or neural stimulation device 1314 and may be received, for example, from the manufacturer of neural stimulation device 1314, from a service provider, or the like. In an aspect, personal computing device 1302 includes update circuitry 1380 for applying the patch or update to software installed on personal computing device 1302 or to software installed on neural stimulation device 1314, by sending update signal 1382 to neural stimulation system 1303. In an aspect, update circuitry 1380 also provides for updating a configuration of at least one of the neural stimulation device and the personal computing device, the configuration relating to operation of the neural stimulation device. In an aspect, update circuitry 1380 can be configured to update the configuration of at least one of the neural stimulation device and the personal computing device based on historical data (e.g., as stored in memory 1336). In another aspect, update circuitry 1380 is configured to update the configuration based on at least one instruction 1384. In an aspect, instruction 1384 is received via user input 1362 of personal computing device 1302. In another aspect, instruction 1384 is received from a computing network, (e.g., from a remote device or system, via a data input such as I/O 892 depicted in FIG. 8). In various aspects, instruction 1384 is received from a medical care provider, an insurance company, or a service provider, for example.

In another aspect, update circuitry 1380 is configured to update the configuration of at least one of the neural stimulation device and the personal computing device based on at least one recommendation 1372. As discussed herein above, recommendation 1372 is received by recommendation receiving circuitry 1370, and can be received from an advisor, from a computation-based system (e.g., an artificial intelligence, machine learning system, or search engine based on a data-driven technique), or from a social media source (for example, in various aspects, the recommendation is based on the at least one preference of at least one social media contact, peer, or role model of the subject). In addition, acceptance/rejection input 1374 is received from the subject by user interface 1214 regarding acceptance or rejection of the recommendation, and update circuitry 1380 updates the configuration responsive to acceptance of the recommendation by the subject (if the recommendation is rejected, no update is made in response to the recommendation). As an alternative, acceptance or rejection of the recommendation can be provided by a caregiver of the subject regarding received via either user interface 1214 or via a data input from a remote device or system. Update circuitry 1380 updates the configuration responsive to acceptance of the recommendation by the caregiver of the subject. In another aspect, update circuitry 1380 is configured to update the configuration of at least one of the neural stimulation device and the personal computing device based on an environmental parameter (based in a secondary input signal 1354 received at secondary signal input 1360. In another aspect, update circuitry 1380 is configured to update the configuration of at least one of the neural stimulation device and the personal computing device automatically. For example, in an aspect, the configuration is updated automatically according to a schedule, for example when the time and/or date indicated by clock/timer 1386 matches an update time/date in schedule 1388 stored in memory 1336.

In an aspect, neural activity signal input 1304 includes circuitry for receiving neural activity signal 1306 via a secure connection. In an aspect, neural control signal output 1312 includes circuitry for outputting neural stimulus control signal 1346 via a secure connection. The secure connection may include be provided through the use of an encrypted signal, for example.

In an aspect, system 1300 includes output circuitry 1390 for presenting information to the subject via user interface 1214, including e.g., audio output 1366, graphical display 1368, alphanumeric display 1392, touchscreen 1394, or other user interface devices, as known to those of ordinary skill in the art.

In an aspect, system 1300 includes customization circuitry 1396. Customization circuitry 1396 customizes for the subject one or both of the information, or the formatting of the information, that is presented to via user interface 1214, based on user preferences, for example.

In an aspect, system 1300 includes authentication circuitry 1398 for receiving a credential 1400 showing that the subject is an authorized user. In an aspect, output circuitry 1390 presents information to the subject via user interface 1214 only following receipt of credential 1400 showing that the subject is an authorized user. In various aspects, authentication circuitry 1398 receives a password, a personal identification number, a biometric feature, or a card authentication, for example.

In an aspect, output circuitry 1390 includes output format circuitry 1402 for presenting the information to the subject via user interface 1214 in a graphical format that mimics the graphical format of an audio player, in a graphical format that mimics the graphical format of a mobile phone, or in any other graphical format that mimics the graphical format of a familiar user interface. This permits the subject to use the neural stimulation device discretely, and present to observers the impression that the personal computing device is functioning as a mobile phone or audio player rather than being used in connection with a neural stimulation device. In an aspect, output circuitry 1390 changes or discontinues the presenting of information to the subject via the user interface in response to an input signal 1404. For example, output circuitry 1390 switches between a first graphical format and a second graphical format on user interface 1214 in response to input signal 1404. For example, the first graphical format may present information relating to the neural stimulus, while the second graphical format may mimic the format of a mobile phone or audio player. In an aspect, input signal 1404 is a user input signal, received for example via user interface 1214. In another aspect, input signal 1404 is a sensed environmental signal indicative of presence of another person (e.g., an audio input signal containing the detected voice of the other person, received via secondary input signal 1354). In an aspect, input signal 1404 is indicative of a time (e.g., a signal received from clock/timer 1386 on personal computing device 1302).

In an aspect, neural stimulus control signal determination circuitry 1308 modulates neural stimulus control signal 1310 in response to an override signal. For example, in an aspect override signal is input signal 1404 received via user input 1362. In an aspect, override signal is secondary input signal 1354, received via secondary signal input 1360. In an aspect, the override signal originates from a sensor that senses a physiological parameter, such as heart rate. In the event that the physiological parameter indicates an unsafe condition (e.g., the heart rate is too high or too low), the neural stimulus control signal determination circuitry 1308 modulates neural stimulus control signal 1310 to discontinue production of the neural stimulus. For example, in various aspects, the override signal originates from a sensor responsive to sensing a presence of a person other than the subject in the vicinity of the subject or responsive to sensing that the external neural stimulator is not properly positioned on the pinna of the subject. In an aspect, neural stimulus control signal determination circuitry 1308 modulates neural stimulus control signal 1310 to discontinue production of the neural stimulus. In an aspect, neural stimulus control signal determination circuitry 1308 modulates neural stimulus control signal 1310 to change an intensity of the neural stimulus. In addition to modulating or discontinuing the neural stimulus in response to an override condition (e.g., physiological parameter indicative of an unsafe condition, improper positioning of the external neural stimulator, etc.), a notification may be sent to the subject and/or to a medical care provider or other party regarding the override condition, to prompt the recipient of the notification to take corrective action, or for inclusion of the information in the subject's medical records.

In an aspect, secondary signal input 1360 is adapted to receive a position signal indicative of a position of the external neural stimulator with respect to the pinna of the subject. In connection therewith, system 1300 may also include notification circuitry 1406 for delivering a notification to the subject indicating that the external neural stimulator should be repositioned. In an aspect, notification circuitry 1406 includes circuitry for delivering the notification via a graphical display 1368 of personal computing device 1302. In an aspect, notification circuitry 1406 includes circuitry for delivering an auditory alert, either via audio output 1366 of personal computing device, or by generating an appropriate audio output signal 1408 for driving production of the auditory alert by a sound source 1410 on neural stimulation device 1314. In an aspect, notification circuitry 1406 includes circuitry for delivering a voice message (e.g., a preset message retrieved from memory 1336). In a further aspect, notification circuitry 1406 includes circuitry for storing information indicating that stimulator 1316 is improperly positioned in a data storage location (e.g., memory 1336) in personal computing device 1302. In another aspect, notification circuitry 1406 provides for storing information indicating that stimulator 1316 is improperly positioned in a data storage location in neural stimulation device 1314 (e.g., by transmitting such information to neural stimulation device 1314.

In an aspect, system 1300 includes circuitry for outputting an audio output signal, either via an audio output 1366 of personal computing device 1302 or via sound source 1410 of neural stimulation device 1314, where the audio output signal drives delivery of sound to the ear of the subject via a sound source. In an aspect, output circuitry 1390 is used to output the audio output signal via audio output 1366 of the personal computing device. In an aspect, communication circuitry 1218 is used for transmitting audio output signal 1408 to a sound source 1410 on neural stimulation device 1314. Alternatively, communication circuitry 1218 can be used to deliver an audio output signal to sound source distinct from the neural stimulation device (e.g., a sound source included in a device used by the subject, but not included in the neural stimulation device). In an aspect, output circuitry 1390 retrieves an audio signal from a data storage location (e.g., memory 1336) on personal computing device 1302, and generate audio output signal based on the retrieved audio signal. In another aspect, system 1300 includes audio receiver 1412 for receiving audio input signal 1414 from a telecommunication network. For example, in various aspects, audio input signal 1414 is a broadcast radio signal, a webcast audio signal, or a mobile phone signal.

In an aspect, system 1300 includes prioritization circuitry 1416 for prioritizing delivery of the neural stimulus control signal relative to the audio output signal (either audio output signal 1408 for delivery to sound source 1410, and/or an audio output signal delivered via audio output 1366 on personal computing device 1302). In an aspect, prioritization circuitry 1416 automatically discontinues outputting of the neural stimulus control signal 1310 and starts outputting of the audio output signal in response to receipt of audio input signal 1414. In another aspect, prioritization circuitry 1416 automatically declines audio input signal 1414 if the neural stimulus is currently being delivered. In another aspect, prioritization circuitry 1416 provides for circuitry for outputting the audio output signal simultaneously with neural stimulus control signal 1310. In another aspect prioritization circuitry 1416 provides for switching between outputting the audio output signal and outputting neural stimulus control signal 1346. Switching may occur in response to a user input received via user input 1362, or in response to sensor input received, for example, via secondary signal input 1360. In an aspect, prioritization circuitry 1416 performs switching between outputting the audio output signal and outputting neural stimulus control signal 1310 according to a schedule (stored, e.g., in memory 1336) in response to input from clock/timer 1386. In an aspect, prioritization circuitry 1416 switches between outputting the audio output signal and outputting the neural stimulus control signal responsive to receipt of the audio input signal 1414 from a telecommunication network. Prioritization circuitry 1416 may be configured to give higher priority to outputting of the neural stimulus control signal than to outputting of the audio output signal, or to give higher priority to outputting of the audio output signal than to outputting of the neural stimulus control signal. The priority of the signals may be determined by the preference of the subject. For example, the subject may consider it a higher priority to receive a phone call via his or her mobile phone than to continue received of a neural stimulation, and therefore may configure system 1300 so that neural stimulation is discontinued when a phone call is received. Alternatively, the subject may prefer that a neural stimulation session not be interrupted, and may configure system 1300 such that no phone calls will be received while neural stimulation is taking place. In other aspects, the subject may provide an input at user interface 1214 (e.g., by pressing a button) to switch between receiving neural stimulation and listening to music, as preferred. In another aspect, system 1300 is configured to deliver neural stimulation in combination with music.

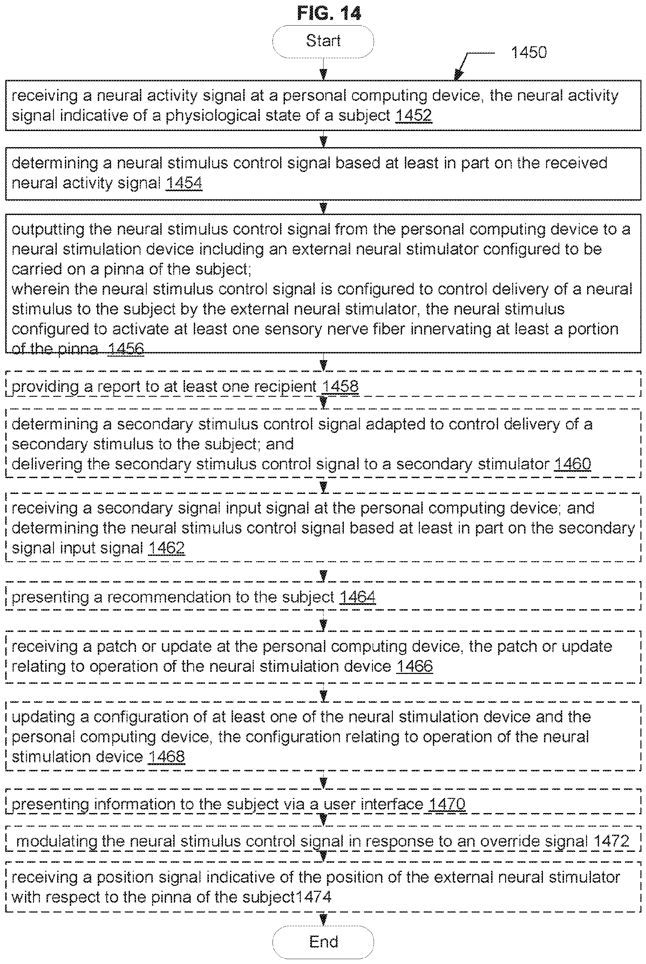

FIG. 14 is a flow diagram of a method 1450 relating to use of a system including a personal computing device, as illustrated in FIG. 13. Method 1450 includes receiving a neural activity signal at a personal computing device, the neural activity signal indicative of a physiological status of a subject, as indicated at 1452. In addition, method 1450 includes determining a neural stimulus control signal based at least in part on the neural activity signal, as indicated at 1454, and outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, as indicated at 1456. In an aspect, determining the neural stimulus control signal includes determining a stimulation pattern. In various aspect, method 1450 includes additional steps, relating to the system functions described in greater detail in connection with FIG. 13. For example, in an aspect, method 1450 includes providing a report to at least one recipient, as indicated at 1458. In an aspect, method 1450 includes determining a secondary stimulus control signal adapted to control delivery of a secondary stimulus to the subject, and delivering the secondary stimulus control signal to a secondary stimulator, as indicated at 1460. For example, in an aspect, the secondary stimulator includes a game device, and the secondary stimulus control signal controls operation of the game device. In another aspect, the secondary stimulator includes computing system configured to deliver a virtual therapist experience, and the secondary stimulus control signal controls operation of the virtual therapist. In another aspect, the secondary stimulus includes an interactive activity delivered via a computing device, and the secondary stimulus control signal controls operation of the computing device.

In an aspect, method 1450 includes receiving a secondary input signal at the personal computing device and determining the neural stimulus control signal based at least in part on the secondary input signal, as indicated at 1462. For example, in an aspect the secondary input signal is indicative of a user input provided spontaneously by subject. Other secondary input signals are described herein above.

In an aspect, method 1450 includes presenting a recommendation to the subject, as indicated at 1464. Method 1450 may also include receiving the recommendation at the personal computing device, as described above in connection with FIG. 13.

In an aspect, method 1450 includes receiving a patch or update at the personal computing device, the patch or update relating to operation of the neural stimulation device, as indicated at 1466. In an aspect, the patch or update is for software installed on the personal computing device. In another aspect, the patch or update is for software installed on the neural stimulation device, in which case method 1450 may also include sending the patch or update to the neural stimulation device.

In an aspect, method 1450 includes updating a configuration of at least one of the neural stimulation device and the personal computing device, the configuration relating to operation of the neural stimulation device, as indicated at 1468. As discussed above, the configuration is updated based on at least one instruction. In another aspect, the configuration is updated based on at least one recommendation, responsive to receipt of an input regarding acceptance of the recommendation by the subject or a caregiver of the subj ect.

In an aspect, method 1450 includes presenting information to the subject via a user interface, as indicated at 1470. The method may also include changing or discontinuing the presenting of information to the subject via the user interface in response to an input signal. In an aspect, method 1450 includes modulating the neural stimulus control signal in response to an override signal, as indicated at 1472.

In an aspect, method 1450 includes receiving a position signal indicative of the position of the external neural stimulator with respect to the pinna of the subj ect, as indicated at 1474. Method 1450 may also include delivering a notification to the subject indicating that external neural stimulator should be repositioned. Other method aspects are discussed in connection with FIG. 13.

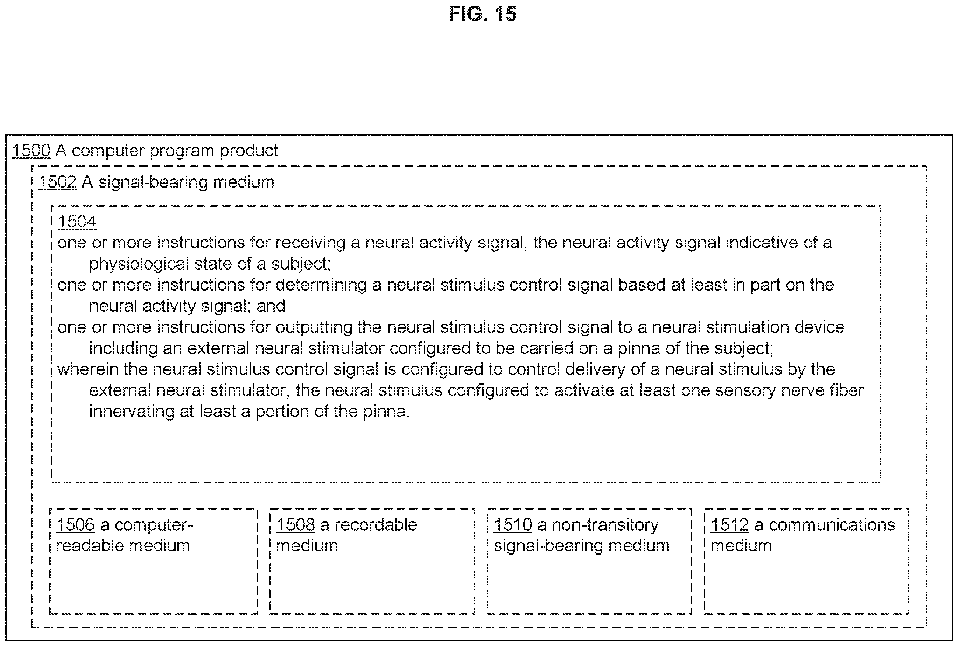

FIG. 15 is a block diagram of a computer program product 1500 for implementing a method as described in connection with FIG. 14. Computer program product 1500 includes a signal-bearing medium 1502 bearing one or more instructions for receiving a neural activity signal, the neural activity signal indicative of a physiological status of a subject; one or more instructions for determining a neural stimulus control signal based at least in part on the neural activity signal; and one or more instructions for outputting the neural stimulus control signal to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, as indicated at 1504. Signal-bearing medium 1502 may be, for example, a computer-readable medium 1506, a recordable medium 1508, a non-transitory signal-bearing medium 1510, or a communications medium 1512, examples of which are described herein above.

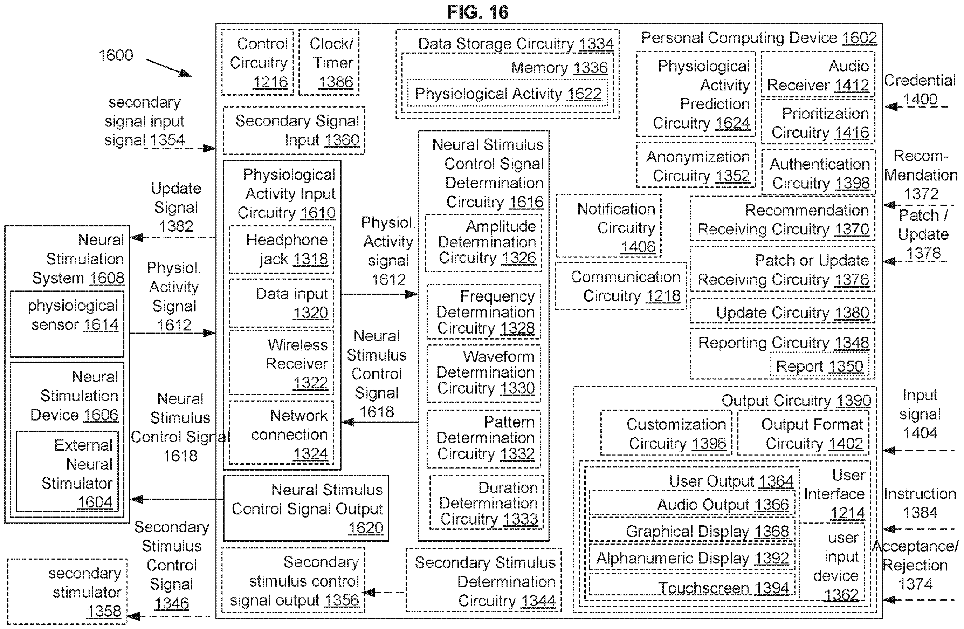

FIG. 16 is a block diagram of a system 1600 including a personal computing device 1602 and external neural stimulator 1604, which comprises a part of neural stimulation device 1606 and neural stimulation system 1608. Personal computing device 1602 is as described generally in connection with FIG. 12. In an aspect, a system 1600 includes personal computing device 1602 including physiological activity input circuitry 1610 for receiving a physiological activity signal 1612 at personal computing device 1062. Physiological activity signal 1612 is sensed by physiological sensor 1614 in neural stimulation system 1608, and is indicative of a physiological status of a subject. Physiological sensor 1614 can be any of various types of physiological sensors, e.g., as described in connection with physiological sensor 758 in FIG. 7. In various aspects, physiological activity signal 1612 is representative of a heart rate (and in some cases heart rate rhythm variability), a blood pressure, perspiration, skin conductivity, respiration, pupil dilation, digestive tract activity, or piloerection. In some aspects, physiological activity signal 1612 is a neural activity signal, such as an electroencephalographic or electrooculographic signal. Physiological activity signal 1612 may be an electromyographic signal (indicative of muscle activity of the subject) or an electrocardiographic signal (indicative of cardiac activity of the subject). Physiological activity signal 1612 may be an unprocessed physiological signal, or physiological activity signal 1612 may have been subjected to various types and amounts of signal processing, and/or analysis (including, but not limited to filtering, amplification, analog to digital conversion, signal averaging, conversion from time to frequency domain, feature extraction, and so forth). Physiological activity signal 1612 may include activity sensed from one or more physiological sensors 1614. Physiological activity signal 1612 may include information derived from or associated with the sensed physiological signal, and may include or be accompanied by additional information that identifies the type of signal, type of processing to which the signal has been subject, data formatting, device settings used during acquisition of the physiological signal, etc.. Personal computing device 1602 also includes neural stimulus control signal determination circuitry 1616 for determining neural stimulus control signal 1618 based at least in part on physiological activity signal 1612. Neural stimulus control signal 1618 is configured to control delivery of a neural stimulus by external neural stimulator 1604. In an aspect, the neural stimulus is configured to activate at least one sensory nerve fiber innervating at least a portion of the pinna. Personal computing device 1602 also includes neural stimulus control signal output circuitry 1620 for outputting neural stimulus control signal 1618 from personal computing device 1602 to neural stimulation device 1606. Neural stimulation device 1606 includes external neural stimulator 1604 configured to be carried on a pinna of the subject. Personal computing device 1602 also includes output circuitry 1390 for presenting information to the subject via user interface 1364 (as described herein above in connection with FIG. 13). Various elements of system 1600 are the same as like-numbered elements of the systems shown in FIGS. 12 or 13, and accordingly will not be discussed in detail again in connection with FIG. 16. However, some components of system 1600 include different and/or additional features. For example, data storage circuitry 1334 is also adapted for storing physiological activity data 1622 representing physiological activity signal 1612 in memory 1336. In an aspect, physiological activity prediction circuitry 1624 predicts a future physiological activity signal based on a previous physiological activity signal. In addition, neural stimulus control signal determination circuitry 1616 determines the neural stimulus based on a previous physiological activity signal. Secondary stimulus determination circuitry 1344 is adapted to determine the secondary stimulus based on physiological activity signal 1612 or a previous physiological activity signal (e.g., stored in memory 1336). As noted above in connection with FIG. 13, in an aspect, secondary input signal 1354 is a physiological signal. It will be appreciated that secondary input signal 1354 in this context will be a secondary physiological signal, and physiological activity signal 1612 will be a primary physiological signal. In an aspect, physiological activity input circuitry 1610 includes circuitry for receiving physiological activity signal 1612 via a secure connection. In an aspect, neural stimulus control signal output 1620 includes circuitry for outputting neural stimulus control signal 1618 via a secure connection.

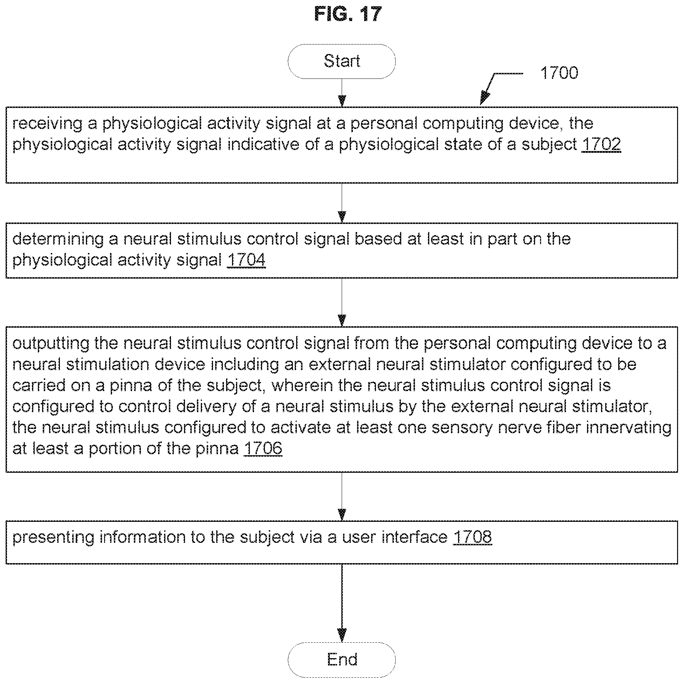

FIG. 17 is a flow diagram of a method 1700 relating to use of a system as depicted in FIG. 16. In an aspect, method 1700 includes receiving a physiological activity signal at a personal computing device, the physiological activity signal indicative of a physiological status of a subject, as indicated at 1702; determining a neural stimulus control signal based at least in part on the physiological activity signal, as indicated at 1704; outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, as indicated at 1706; and presenting information to the subject via a user interface, as indicated at 1708. Other method aspects are discussed in connection with FIGS. 14 and 16.

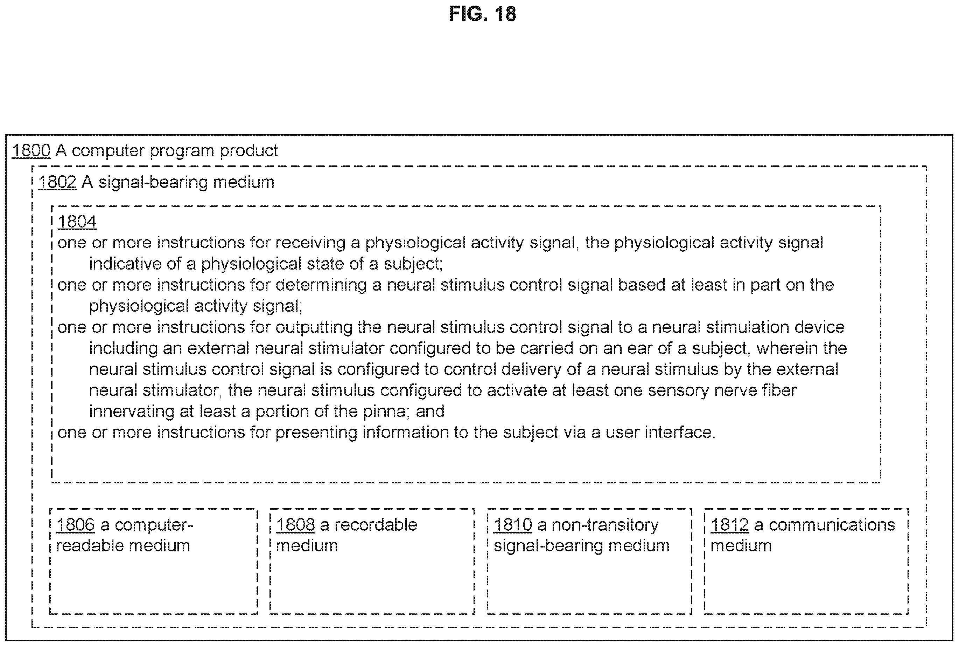

FIG. 18 is a block diagram of a computer program product 1800 for implementing a method as described in connection with FIG. 17. Computer program product 1800 includes a signal-bearing medium 1802 bearing one or more instructions for receiving a physiological activity signal, the physiological activity signal indicative of a physiological status of a subject; one or more instructions for determining a neural stimulus control signal based at least in part on the physiological activity signal; one or more instructions for outputting the neural stimulus control signal to a neural stimulation device including an external neural stimulator configured to be carried on an ear of a subject, wherein the neural stimulus control signal is configured to control delivery of a neural stimulus by the external neural stimulator, the neural stimulus configured to activate at least one sensory nerve fiber innervating at least a portion of the pinna; and one or more instructions for presenting information to the subject via a user interface, as indicated at 1804. Signal-bearing medium 1802 may be, for example, a computer-readable medium 1806, a recordable medium 1808, a non-transitory signal-bearing medium 1810, or a communications medium 1812, examples of which are described herein above.

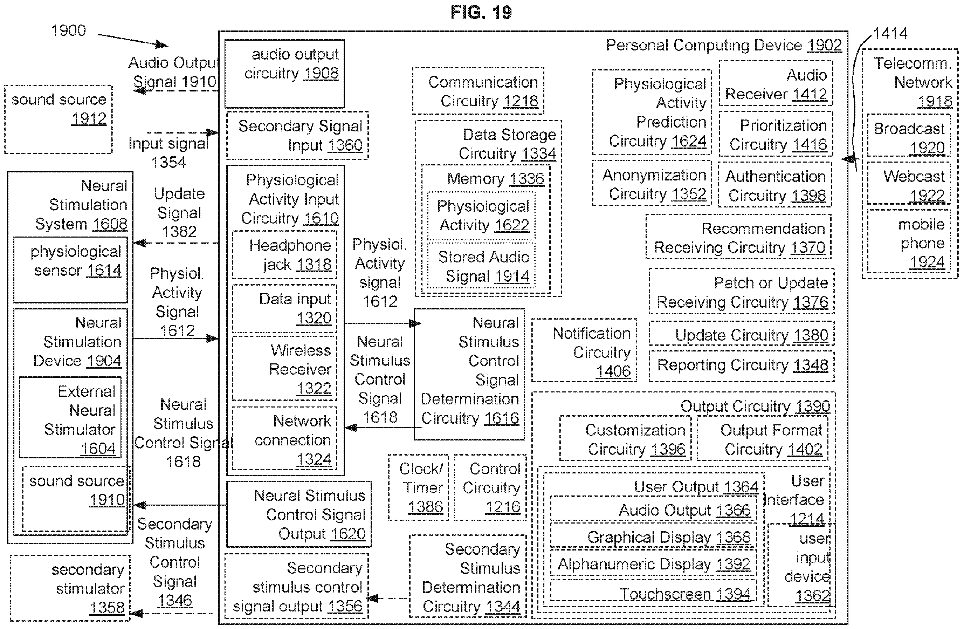

FIG. 19 is a block diagram of a system 1900. FIG. 19 is similar to the system depicted in FIGS. 13 and 16, and like-numbered system components described in connection with these figures will not be described again in connection with FIG. 19. In an aspect, system 1900 includes a personal computing device 1902 including physiological activity input circuitry 1610 for receiving a physiological activity signal at personal computing device 1902, the physiological activity signal 1612 indicative of a physiological status of a subject. System 1900 also includes neural stimulus control signal determination circuitry 1616 for determining a neural stimulus control signal 1618 based at least in part on physiological activity signal 1612. In addition, system 1900 includes neural stimulus control signal output circuitry 1620 for outputting neural stimulus control signal 1618 from personal computing device 1902 to neural stimulation device 1904. Neural stimulation device 1904 includes external neural stimulator 1604 configured to be carried on a pinna of the subject, wherein neural stimulus control signal 1618 is configured to control delivery of a neural stimulus by the external neural stimulator, the neural stimulus configured to activate at least one sensory nerve fiber innervating at least a portion of the pinna. System 1900 also includes audio output circuitry 1908 for outputting an audio output signal 1910 via an audio output 1366 of personal computing device 1902. In an aspect, system 1900 includes circuitry for delivering the audio output signal to sound source 1910 on neural stimulation device. In another aspect, system 1900 includes circuitry for delivering audio output signal 1910 to sound source 1912 that is distinct from neural stimulation device 1904. For example, sound source 1912 may be a sound source in the environment of the subject but not on the neural stimulation device, including but not limited to a sound source on, built into, or associated with personal computing device 1902. In an aspect, system 1900 includes data storage circuitry 1334 for retrieving stored audio signal 1914 from a data storage location (memory 1336) on personal computing device 1902. In an aspect, system 1900 includes audio receiver 1412 for receiving the audio input signal from telecommunication network 1918. For example, in various aspects, the audio input signal is a broadcast radio signal 1920, a webcast audio signal 1922, or a mobile phone signal 1024.

In an aspect, system 1900 includes prioritization circuitry 1416 which prioritizes between delivery of neural stimulus and delivery of the audio output signal, based upon system settings and/or preferences of the subject. For example, prioritization circuitry 1416 provides for automatically discontinuing outputting of the neural stimulus control signal and starting outputting of the audio output signal in response to receipt of the audio input signal, automatically declining the audio input signal if the neural stimulus is currently being delivered, or outputting the audio output signal simultaneously with the neural stimulus control signal. In other aspects, prioritization circuitry 1416 provides switching between outputting the audio output signal and outputting the neural stimulus control signal, for example in response to a user input or a sensor input, according to a schedule, or in response to receipt of an audio input signal (e.g., a phone call) from a telecommunication network. Depending on preference of the subject or other considerations, prioritization circuitry 1416 can be configured to give higher priority to outputting of the neural stimulus control signal than to outputting of the audio output signal, or to give higher priority to outputting of the audio output signal than to outputting of the neural stimulus control signal.

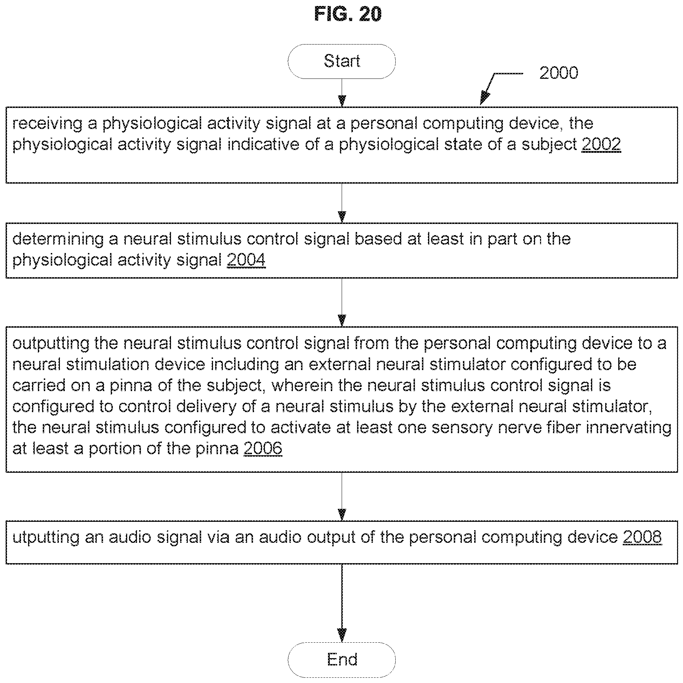

FIG. 20 is a flow diagram of a method 2000 relating to use of a system as depicted in FIG. 19. In an aspect, method 2000 includes receiving a physiological activity signal at a personal computing device, the physiological activity signal indicative of a physiological status of a subject, as indicated at 2002; determining a neural stimulus control signal based at least in part on the physiological activity signal, as indicated at 2004; outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, as indicated at 2006; and outputting an audio output signal via an audio output of the personal computing device, as indicated at 2008. Other method aspects are discussed in connection with FIGS. 14 and 19.

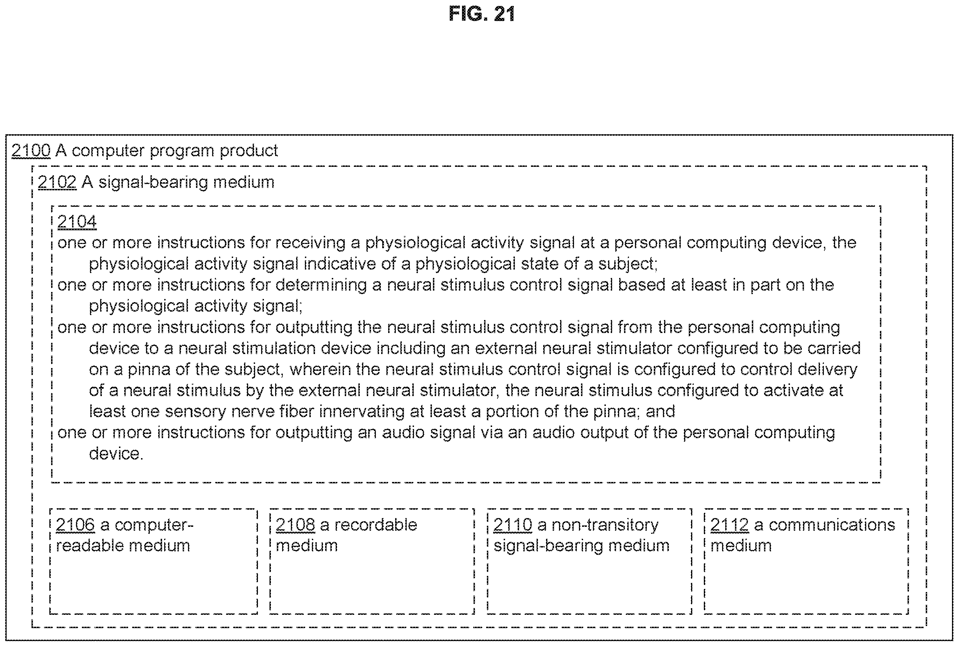

FIG. 21 is a block diagram of a computer program product 2100 for implementing a method as described in connection with FIG. 20. Computer program product 2100 includes a signal-bearing medium 2102 bearing one or more instructions for receiving a physiological activity signal at a personal computing device, the physiological activity signal indicative of a physiological status of a subject, one or more instructions for determining a neural stimulus control signal based at least in part on the physiological activity signal, one or more instructions for outputting the neural stimulus control signal from the personal computing device to a neural stimulation device including an external neural stimulator configured to be carried on a pinna of the subject, wherein the neural stimulus control signal is configured to control delivery of a neural stimulus by the external neural stimulator, the neural stimulus configured to activate at least one sensory nerve fiber innervating at least a portion of the pinna, and one or more instructions for outputting an audio output signal via an audio output of the personal computing device, as indicated at 2104. Signal-bearing medium 2102 may be, for example, a computer-readable medium 2106, a recordable medium 2108, a non-transitory signal-bearing medium 2110, or a communications medium 2112, examples of which are described herein above.

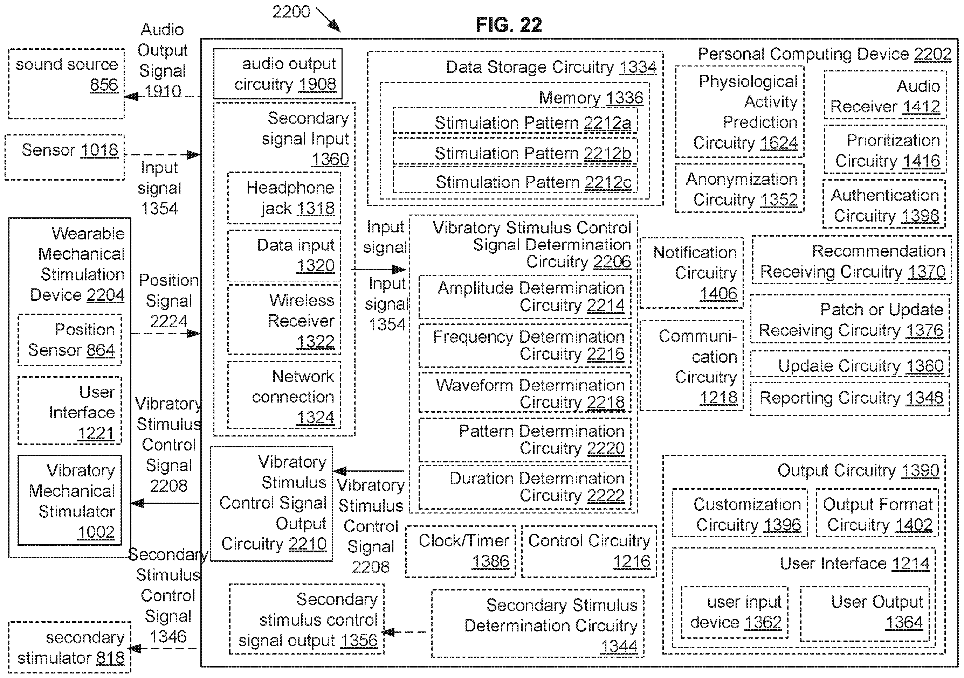

FIG. 22 is a block diagram of a system 2200, which includes a personal computing device 2202 for use in combination with a wearable mechanical stimulation device 2204. FIG. 22 is similar to the systems depicted in FIGS. 13, 16 and 19 and like-numbered system components described in connection with these figures will not be described again in connection with FIG. 22. Personal computing device 2202 includes vibratory stimulus control signal determination circuitry 2206 for determining a vibratory stimulus control signal 2208, and vibratory stimulus control signal output circuitry 2210 for outputting vibratory stimulus control signal 2208 to wearable mechanical stimulation device 2204. Wearable mechanical stimulation device 2204 includes a vibratory mechanical stimulator 1002 configured to be carried on a pinna of a subject, wherein the vibratory stimulus control signal is configured to control delivery of a vibratory stimulus by the vibratory mechanical stimulator 1002, the vibratory stimulus configured to activate at least one mechanoreceptor with a receptive field on at least a portion of the pinna. In an aspect, wearable mechanical stimulation device 2204 is a wearable neural stimulation device 1000 of the type discussed in connection with FIG. 10, and can be considered a variant of wearable neural stimulation device 1202 depicted and discussed in connection with FIG. 12. In addition, in various aspects system 2200 includes additional components such as are included in neural stimulation system 1200 described in connection with FIGS. 7, 10 and/or 12, including, but not limited to, sensor 1018 for detecting input signal 1354, user interface 1221, position sensor 864, secondary stimulator 818, and sound source 856. Personal computing device 2202 can be any of the various types of personal computing devices described in connection with FIG. 12, for example, a personal digital assistant, a personal entertainment device, a mobile phone, a laptop computer, a table personal computer, a wearable computing device, a networked computer, a computing system comprised of a cluster of processors, a computing system comprised of a cluster of servers, a workstation computer, or a desktop computer. Data storage circuitry 1334 including memory 1336 on personal computing device 2202 can be used to store data, instructions, parameters, as described elsewhere herein, including but not limited to stimulation patterns 2212a, 2212b, and 2212c representing vibratory mechanical stimuli to be delivered under the control of vibratory stimulus control signal 2208. In an aspect, vibratory stimulus control signal 2208 is configured to cause delivery of one of a plurality of pre-programmed stimulation patterns, e.g., selected from stimulation patterns 2212a, 2212b, and 2212c stored in memory 1336. In an aspect, vibratory stimulus control signal 2208 is determined by vibratory stimulus control signal determination circuitry 2206. In various aspects, vibratory stimulus control signal determination circuitry 2206 includes amplitude determination circuitry 2214, frequency determination circuitry 2216, waveform determination circuitry 2218, pattern determination circuitry 2220, or duration determination circuitry 2222 for determining various aspects of the vibratory stimulus control signal 2208, which determines the mechanical stimulus delivered by vibratory mechanical stimulator 1002. If position signal 2224 from position sensor 864 indicates that vibratory mechanical stimulator 1002 is not properly positioned on the ear of the subject, a notification is provided to the subject, e.g., via notification circuitry 1406, instructing the subject to reposition vibratory mechanical stimulator 1002.

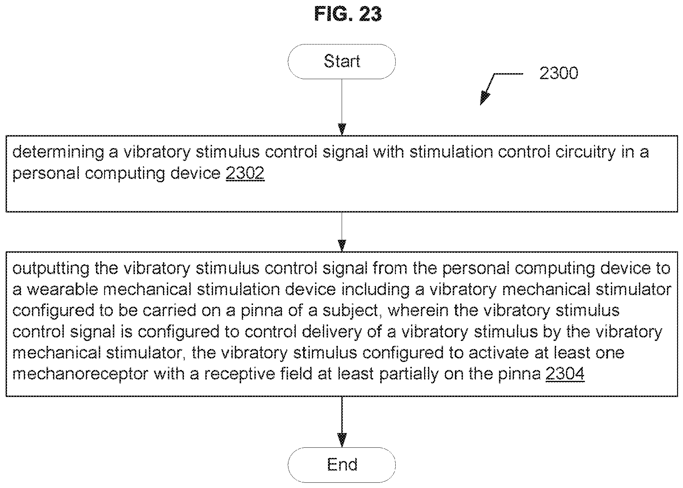

FIG. 23 is a flow diagram of a method 2300 involving the use of a system as depicted in FIG. 22. In an aspect, method 2300 includes determining a vibratory stimulus control signal with stimulation control circuitry in a personal computing device, as indicated at 2302; and outputting the vibratory stimulus control signal from the personal computing device to a wearable mechanical stimulation device including a vibratory mechanical stimulator configured to be carried on a pinna of a subject, wherein the vibratory stimulus control signal is configured to control delivery of a vibratory stimulus by the vibratory mechanical stimulator, the vibratory stimulus configured to activate at least one mechanoreceptor with a receptive field on at least a portion of the pinna, as indicated at 2304.

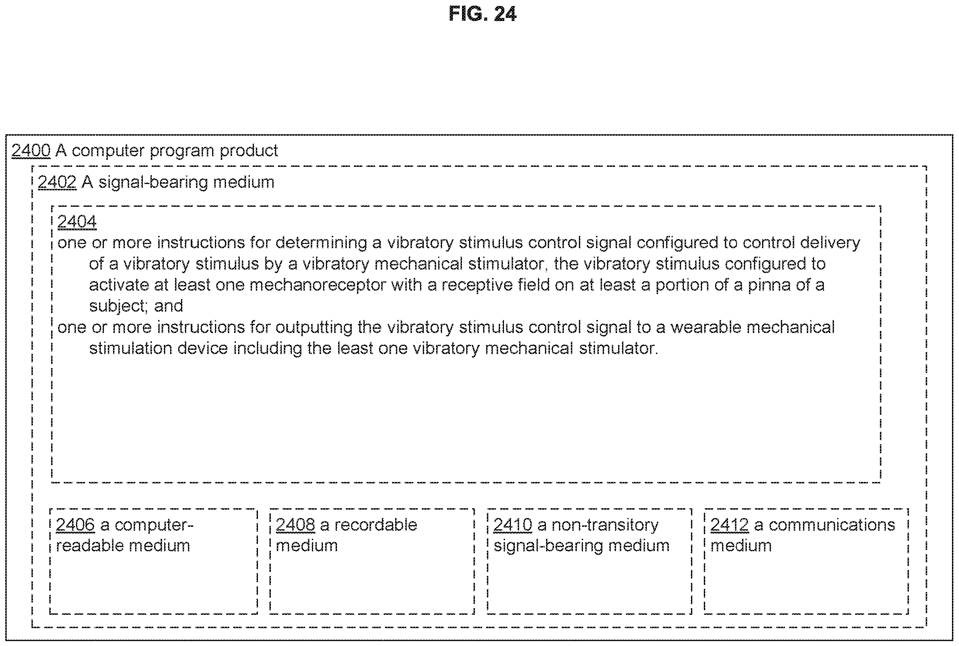

FIG. 24 is a block diagram of a computer program product 2400 for implementing a method as described in connection with FIG. 23. Computer program product 2400 includes a signal-bearing medium 2402 bearing one or more instructions for determining a vibratory stimulus control signal configured to control delivery of a vibratory stimulus by a vibratory mechanical stimulator, the vibratory stimulus configured to activate at least one mechanoreceptor with a receptive field on at least a portion of a pinna of a subject, and one or more instructions for outputting the vibratory stimulus control signal to a wearable mechanical stimulation device including the least one vibratory mechanical stimulator, as indicated at 2404. Signal-bearing medium 2402 may be, for example, a computer-readable medium 2406, a recordable medium 2408, a non-transitory signal-bearing medium 2410, or a communications medium 2412, examples of which are described herein above.

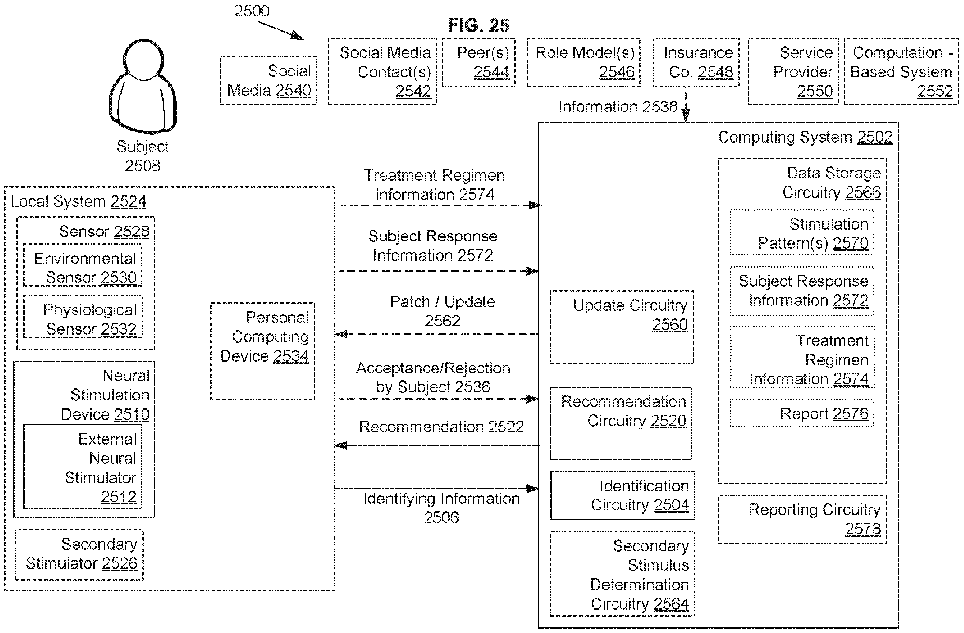

In some aspects, wearable neural stimulation devices and systems as described herein above are used in combination with remote systems. For example, FIGS 2A and 2B illustrate a neural stimulation system used in combination with remote system 26, via communication network 218. FIG. 12 depicts communication between wearable neural stimulation device 1202 and/or personal computing device 1204, which form neural stimulation system 1200, and remote system 1224. In addition, as shown in FIG. 13, information may be transmitted to personal computing device 1302 from a remote system, including, for example, recommendation 1372, patch/update 1374, or instruction 1384. FIG. 25 provides greater detail regarding such a remote system 2500. Remote system 2500 includes computing system 2502. Computing system 2502 includes identification circuitry 2504 for receiving identifying information 2506 identifying at least one of a subject 2508 and a neural stimulation device 2510 associated with subject 2508. Neural stimulation device 2510 is a neural stimulation device configured to be carried on an ear of a subject and including an external neural stimulator 2512. System 2502 includes recommendation circuitry 2520 for providing a recommendation 2522 relating to a treatment regimen to subject 2508, where the treatment regimen includes delivery of a neural stimulus to the subject with external neural stimulator 2512, the neural stimulus configured to activate at least one sensory nerve fiber innervating skin on or in the vicinity of the ear of the subject. In an aspect, recommendation circuitry 2520 uses a database to generate recommendations for combinations of treatments in the treatment regimen, for example in a manner similar to that described in U.S. Patent 7,801,686 granted Sept. 21, 2010 to Hyde et al.; U.S. Patent 7,974,787 granted July 5, 2011 to Hyde et al.; U.S. Patent 8,876,688 granted Nov. 4, 2014 to Hyde et al.; U.S. Patent Publication 20090269329 to Hyde et al., dated Oct. 29, 2009; U.S. Patent Publication 20090271009 to Hyde et al. dated Oct. 29, 2009; and U.S. Patent Publication 20090271375 to Hyde et al. dated Oct. 29, 2009.

In various aspects, neural stimulation device 2510 is a neural stimulation device of any of the various types described herein, e.g., in connection with any of FIGS. 7, 10, or 12. In an aspect recommendation 2522 is sent to, and identifying information 2506 is received from, a local system 2524. Local system 2524 includes neural stimulation device 2510 and other components at the location of subject 2508, including but not limited to a secondary stimulator 2526, at least one sensor 2528 (e.g., an environmental sensor 2530, a physiological sensor 2532, or other sensor as discussed herein above). In an aspect, local system 2524 includes personal computing device 2534. Personal computing device 2534 may include, for example, at least one of a personal digital assistant, a personal entertainment device, a mobile phone, a laptop computer, a tablet personal computer, a wearable computing device, a networked computer, a workstation computer, and a desktop computer, as discussed herein above. In an aspect, recommendation 2522 is presented to subject 2508 via a user interface of personal computing device 2534, for example, and acceptance or rejection of the recommendation entered via a user interface of personal computing device 2534 and transmitted as acceptance/rejection signal 2536 to remote computing system 2502.

Secondary stimulator 2526, sensor 2528, and personal computing device 2534 are as described herein above, e.g., in connection with at least FIGS. 7 and 12. Signals containing information, instructions, data, etc. may be sent between neural stimulation device 2510 and computing system 2502 directly, or information may be sent between computing system 2502 and personal computing device 2534, and then between personal computing device 2534 and neural stimulation device 2510. Transmission of signals (information, instructions, data, etc.) between computing system 2502 and local system 2524 may be via wired or wireless communication links, e.g., via computer or communication networks. In an aspect, computing system 2502 is part of a computing network from which it receives information 2536 from various parties and/or entities, including but not limited to social media 2540, social media contacts 2542, peers 2544, or role models 2546 of subject 2508, insurance companies, service providers (e.g., medical care providers or companies providing various health or wellness related services), and computation-based system associated with such service providers, for example.

Computing system 2502 includes one or more computing device, as described generally in connection with FIG. 8. In an aspect, computing system 2502 includes update generation circuitry 2560 for generating patch/update 2562 which is sent to local system 2524, for updating software on either personal computing device 2534 or neural stimulation device 2510. In an aspect, computing system 2504 includes secondary stimulus determination circuitry 2564 for determining a secondary stimulus to be delivered in combination with the neural stimulus, e.g., by secondary stimulator 2526. The secondary stimulus may be any of various types of stimuli, as described herein above. In an aspect, computing system 2502 includes data storage circuitry 2566, which in various aspects stores information regarding, e.g., one or more stimulation patterns 2570, subject response information 2572 received, e.g., from local system 2524, treatment regimen information 2574, or one or more report 2576. In an aspect, report 2576 is generated by reporting circuitry 2578 and stored in data storage circuitry 2566 in addition to, or as an alternative to, providing report 2576 to a recipient.

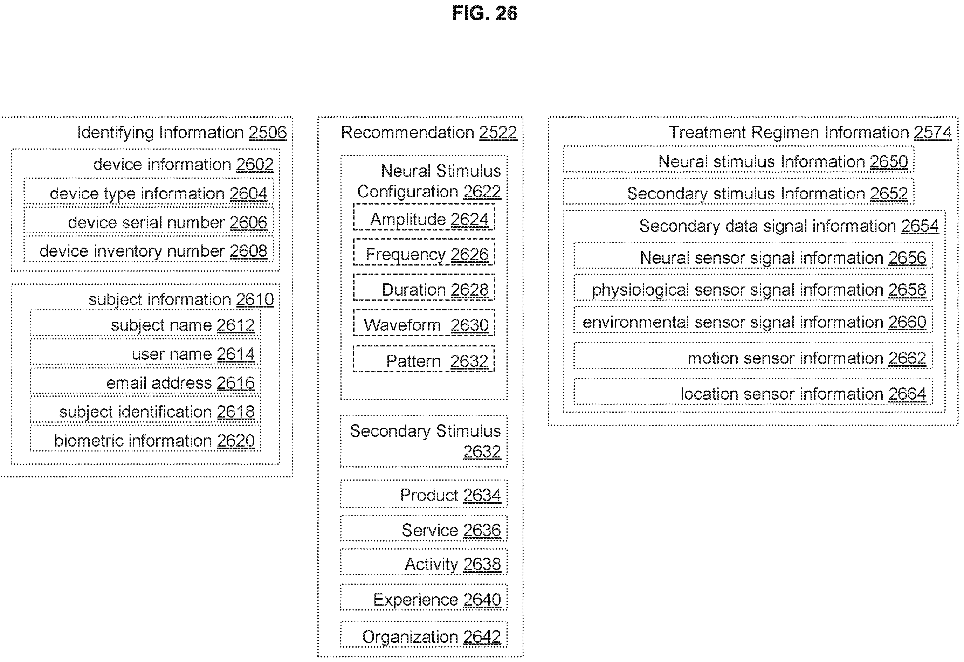

FIG. 26 provides greater detail regarding several aspects of FIG. 25 of information handled by system 2500, specifically information included in identifying information 2506, recommendation 2522, and treatment regimen information 2574.

In various aspects, identifying information 2506 includes device information 2602 pertaining to the neural stimulation device 2510, or subject information 2610 pertaining to the subject. Device information 2602 includes, for example, device type information 2604, device serial number 2606, or device inventory number 2608). Subject information 2610 includes, for example, a name of the subject 2612, a user name 2614 associated with the subject, an email address 2616 associated with the subject, a subject identification 2618 (e.g., identification number, code or the like), or biometric information 2620 associated with the subject. In various aspects, subject identification 2618 can be input by the subject via a user input, read with a bar-code or RFID reader, received with an RF receiver, etc.

Recommendation 2522 may include one or more recommendations for various aspects of device and system configuration for delivery of neural stimulation, and for one or more additional stimuli or experiences to be presented to or experienced by the subject in association with the neural stimulus. In various aspects, recommendation 2522 is for a configuration of the neural stimulus 2622 (e.g., stimulus amplitude 2624, frequency 2626, duration 2628, waveform 2630, or delivery pattern 2632). In various aspects, recommendation 2522 is for a secondary stimulus 2632 to be delivered in association with the neural stimulus. In various aspects, secondary stimulus 2632 includes music, an auditory stimulus, a video stimulus, a tactile stimulus, a haptic stimulus, an olfactory stimulus, a pharmaceutical, a nutraceutical, a secondary neural stimulus, an experience (including, but not limited to a virtual reality experience, a game experience, a virtual therapist experience, an augmented reality experience, and/or an interactive experience). In various aspects, recommendation 2522 is for a product 2634, a service 2636, an activity 2638, an experience 2640, or an organization 2642. The recommendation may be for multiple experiences. In an aspect, the recommendation specifies a pattern of delivery of the experience(s). It will be appreciated that not all secondary stimuli recommended for use in conjunction with a neural stimulus are delivered by the neural stimulation system. Recommendations (e.g., for a product, service, experience, or organization) can be presented to the subject via the personal computing device in the form of a link to a relevant website, so that the subject may conveniently access the recommended product, service, experience, or organization, which the subject does, as desired.

Treatment regimen information 2574 includes, for example, neural stimulus information 2650 regarding the neural stimulus, secondary stimulus information 2652 regarding a secondary stimulus delivered in association with the neural stimulus, information 2654 regarding a secondary data signal, which may specifically include neural sensor signal information 2656, physiological sensor signal information 2658, environmental sensor signal information 2660, motion sensor information 2662 or location sensor information 2664.

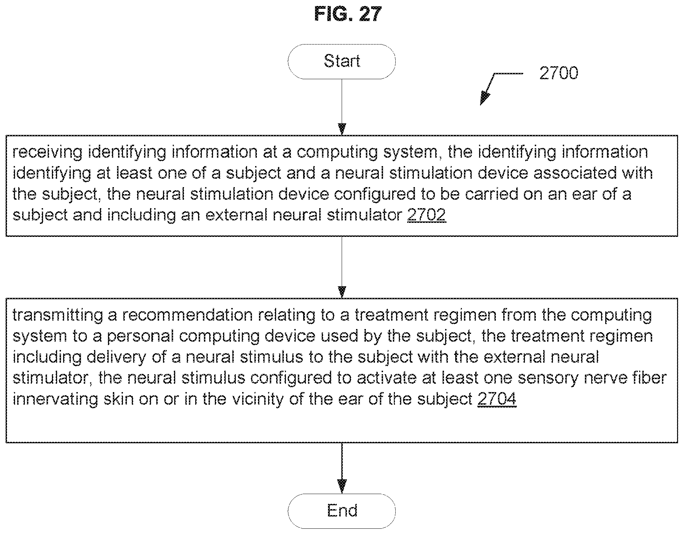

FIG. 27 is a flow diagram of a method 2700 carried out in connection with a system as depicted in FIG. 25 for providing recommendations to a subject. In an aspect, a method 2700 includes receiving identifying information at a computing system (e.g., computing system 2502 in FIG. 25), the identifying information identifying at least one of a subject and a neural stimulation device associated with the subject, the neural stimulation device configured to be carried on an ear of a subject and including an external neural stimulator, as indicated at 2702; and transmitting a recommendation relating to a treatment regimen from the computing system to a personal computing device used by the subject (e.g., personal computing device , the treatment regimen including delivery of a neural stimulus to the subject with the external neural stimulator, the neural stimulus configured to activate at least one sensory nerve fiber innervating skin on or in the vicinity of the ear of the subject, as indicated at 2704.

In an aspect, receiving the identifying information at the computing system includes receiving information transmitted from the personal computing device. In an aspect, receiving the identifying information at the computing system includes receiving information transmitted via a computing network. In an aspect, receiving the identifying information at the computing system includes receiving information transmitted via a wireless network. In an aspect, providing the recommendation relating to the treatment regimen to the subject includes transmitting the recommendation to a personal computing device, e.g., via a computing network or a wireless network.

In an aspect, the recommendation is received at the computing system from a medical care provider. In another aspect, the recommendation is generated at the computing system, e.g., by recommendation circuitry 2520 as shown in FIG. 25. The recommendation can be generated based on various types of information: for example, information regarding a response of the subject to a past treatment regimen (subject response information 2572 in FIG. 25); information obtained via social media (e.g., information 2536 in FIG. 25) which may include, for example, information regarding one or more preferences of one or more social media contacts, peers, or role models of the subject); information from an insurance company; or information from a service provider. In an aspect, generating the recommendation includes generating the recommendation with a computation-based system 2552 (e.g., an artificial intelligence, machine learning system, or search engine based on a data-driven technique). In an aspect, generating the recommendation includes generating the recommendation based on a predicted response of the subject to a treatment regimen.

In an aspect, method 2700 includes receiving information regarding whether the subject has accepted or rejected the recommendation. In an aspect, method 2700 includes receiving a credential showing that the subject is an authorized user of the personal computing device. For example, the credential may include a password, a PIN, a biometric feature, or a card authentication, and/or a credential showing that the personal computing device is an authorized device.

In an aspect, method 2700 includes storing at least one parameter of the neural stimulus in a data storage location associated with the computing system (e.g., with data storage circuitry 2566 of computing system 2502).

In aspect, the recommendation relates to at least one parameter of the neural stimulus, for example, an amplitude, frequency, waveform, or duration of delivery of the neural stimulus, or stimulation pattern for delivery of the neural stimulus. The stimulation pattern may be, for example, a preprogrammed pattern, a continuous pattern, an intermittent pattern, a time-varying pattern, and/or a pulsed pattern. In an aspect, the recommendation specifies a selection of one of multiple stimulation patterns.

In an aspect, receiving the identifying information at the computing system includes receiving information transmitted from the personal computing device.

In an aspect, method 2700 includes transmitting a report relating to the treatment regimen to at least one recipient. In an aspect, the at least one recipient includes, for example, the subject, a caregiver of the subject, at least one social media contact of the subject, at least one peer of the subject, at least one medical care provider, or at least one insurance provider. In an aspect, the recipient is a computing system, e.g. a computing system used for storing and/or processing healthcare information. In some cases the report is anonymized, e.g., to preserve the privacy of the subject. The report may include demographic information pertaining to the subject, but not personal identifying information pertaining to the subject, for example. In an aspect, transmitting the report includes transmitting the report to the personal computing device. The report may include, for example, a neural stimulus control signal, a determined compliance of the subject with the treatment regimen, a determined efficacy of the treatment regimen, one or more system settings for controlling delivery of the neural stimulus, data retrieved from a data storage location associated with the computing system, and/or information regarding a secondary stimulus delivered in association with the neural stimulus. Compliance of the subject and/or efficacy of the treatment regimen may be determined by questioning the subject directly, by questioning another party, such as a caregiver, or by making a determination from measured physiological parameters of the subject.

In an aspect, method 2700 includes receiving a report relating to the treatment regimen from the personal computing device. In an aspect, method 2700 includes storing information relating to the treatment regimen in a data storage location associated with the computing system, e.g., treatment regimen information 2574 as described in connection with FIGS. 15 and 26.

In an aspect, method 2700 includes receiving information at the computing system regarding a previously delivered treatment regimen. In addition, the method may include receiving information at the computing system regarding a response of subject to the previously delivered treatment regimen.

In an aspect, method 2700 includes sending a patch or update to a personal computing device from the computing system. The patch or update may be for software installed on the personal computing device, or for software installed on the external neural stimulator.

In an aspect, method 2700 includes generating an update for the configuration of the neural stimulus. This may be done based on a response of the subject to a previous treatment regimen, based on an environmental factor, or based on motion or location of the subject. In an aspect, the update is generated automatically e.g., when it is determined that an update is needed (based on a subject response or sensed environmental factor). In another aspect, the update is generated based upon acceptance of a recommendation for the update by the subj ect.

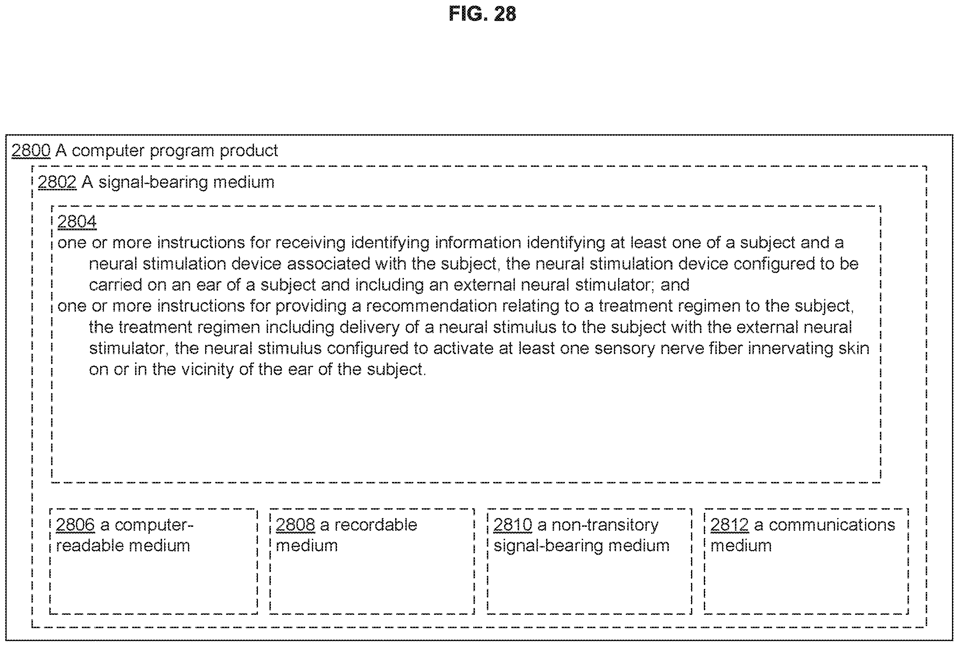

FIG. 28 is a block diagram of a computer program product 2800 for implementing a method as described in connection with FIG. 27. Computer program product 2800 includes a signal-bearing medium 2802 bearing one or more instructions for receiving identifying information identifying at least one of a subject and a neural stimulation device associated with the subject, the neural stimulation device configured to be carried on an ear of a subject and including an external neural stimulator, and one or more instructions for providing a recommendation relating to a treatment regimen to the subject, the treatment regimen including delivery of a neural stimulus to the subject with the external neural stimulator, the neural stimulus configured to activate at least one sensory nerve fiber innervating skin on or in the vicinity of the ear of the subject, as indicated at 2804. Signal-bearing medium 2802 may be, for example, a computer-readable medium 2806, a recordable medium 2808, a non-transitory signal-bearing medium 2810, or a communications medium 2812, examples of which are described herein above.

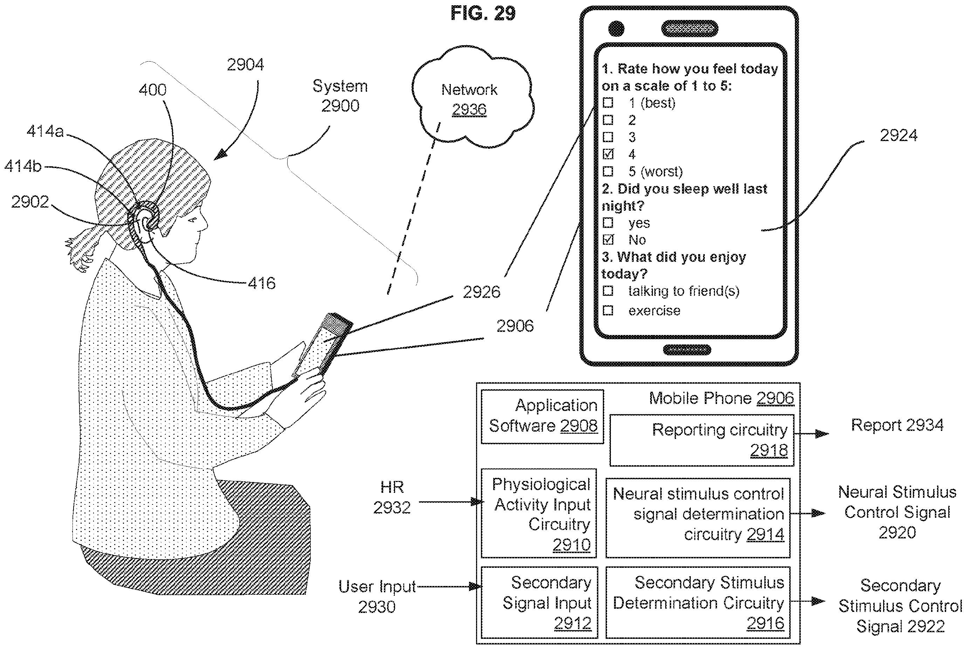

FIG. 29 depicts an embodiment of a system 2900 for delivering neural stimulation in combination with providing a therapeutic secondary stimulus. System 2900 includes securing member 400, of the type depicted in FIGS. 4A and 4B, with an ear canal insert 416 including a heart rate sensor (not shown in FIG. 29), and stimulating electrodes 414a and 414b, positioned to stimulate pinna 2902 of subject 2904. System 2900 also includes mobile phone 2906, configured with application software 2908. Mobile phone 2906 and application software 2908 together form at least physiological activity input circuitry 2910, secondary signal input 2912, neural stimulus control signal determination circuitry 2914, secondary stimulus determination circuitry 2916, and reporting circuitry 2918. Mobile phone 2906 along with application software 2908 form a personal computing device, which includes a variety of circuitry (not all of which is depicted in FIG. 29), e.g. as depicted and described in connection with FIG. 16.

Neural stimulus control signal determination circuitry 2914 is used to generate neural stimulus control signal 2920, which drives delivery of a neural stimulus via electrodes 414a and 414b. Secondary stimulus determination circuitry 2916 is used to generate secondary stimulus control signal 2922, which controls delivery of the therapeutic secondary stimulus while subject 2904 is receiving stimulation delivered to pinna 2902. In the example of FIG. 29, the therapeutic secondary stimulus is provided via digital media, in the form of a therapy application that provides cognitive training and therapy. The therapy application also performs mental health monitoring. In an aspect, the therapy application includes an interactive survey 2924 displayed on touchscreen 2926 of mobile phone 2906. The survey asks subject 2904 questions designed, for example, to assess the subject's mental or emotional state ("Rate how you feel today"), identify factors contributing to or relating to the subject's mental or emotional state ("Did you sleep well last night?"), and guide the subject toward positive and/or constructive thought patterns ("What did you enjoy today?"). Subject 2904 provides responses (user input 2930) to the queries via touchscreen 2926, which are received by secondary signal input 2912. In addition, or as an alternative, the therapy application may provide a therapeutic secondary stimulus that includes music or guided meditation, delivered via touchscreen 2926 and/or a speaker in ear canal insert 416.

Heart rate 2932, sensed with a heart rate sensor (for example an ECG sensor or pulse oximeter sensor) in ear canal insert 416, is provided to physiological activity input circuitry 2910. The subject's heart rate is monitored during delivery of neural stimulation in combination with the therapeutic secondary stimulus, to track the effect of the stimulation and therapy over time. Amount of heart rate variability and duration of heart variability and/or changes in heart rate variability over time may be monitored. Heart rate variability is an indicator of the balance between sympathetic and parasympathetic tone. Increased heart rate variability is associated with reduced inflammation and anxiety. In addition, the physiologic data can be coupled with how the subject interacts with the program. In an aspect, one or both of neural stimulus control signal 2920 and secondary stimulus control signal 2922 are modified (by neural stimulus control signal determination circuitry 2914 and secondary stimulus determination circuitry 2916, respectively), in response to heart rate 2932 and user input 2930. Physiological data regarding the subject's heart rate as well as data regarding interaction of subject 2904 with application software 2908 can be included in report 2934 which can be sent to the subject's medical care provider or psychologist via network 2936. Detection of a heart rate indicative of an unsafe condition due to the neural stimulation results in discontinuation or modulation of stimulation, and transmittal of a notification to the subject's medical care provider.

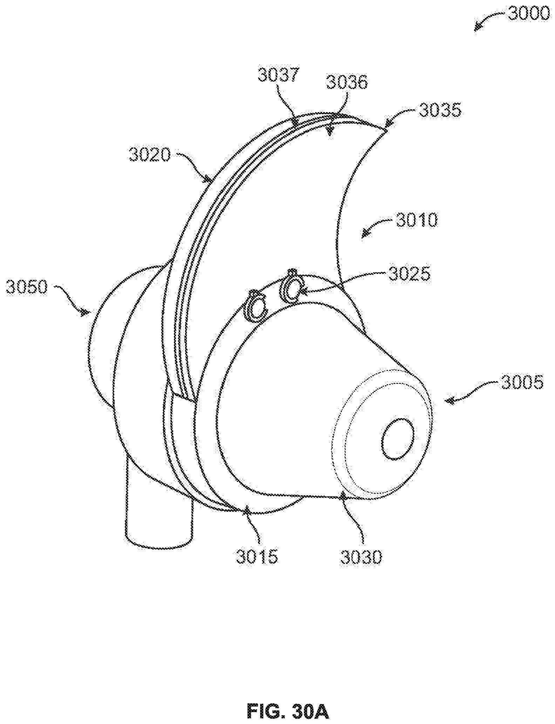

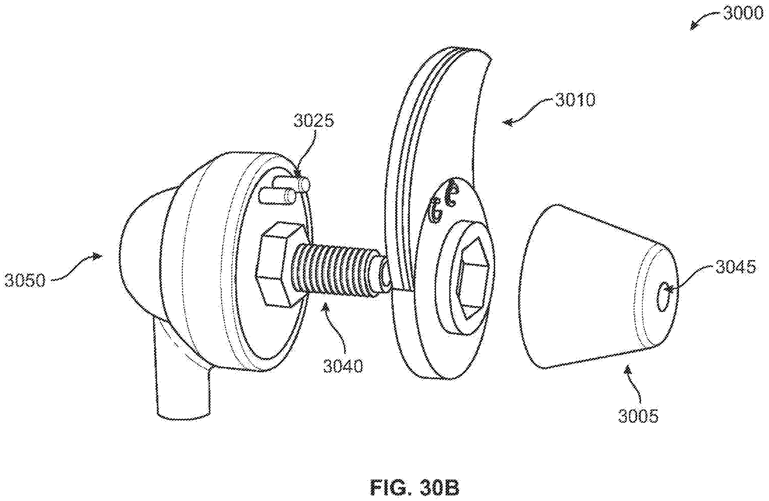

FIG. 30A depicts a nerve stimulation earpiece 3000. The nerve stimulation earpiece 3000 may be used to implement at least some of the embodiments described herein. For example, the nerve stimulation earpiece 3000 may be used to implement the wearable neural stimulation device 202 depicted in Figure 2A. The nerve stimulation earpiece 3000 includes an ear canal insert 3005, a concha insert 3010, a first electrical connector 3040 (not visible in FIG. 30A, see FIG. 30B), and a second electrical connector 3025. In some embodiments, the nerve stimulation earpiece 3000 may include or may be configured to couple to a body portion of an audio headphone 3050.

The ear canal insert 3005 may be adapted to fit into an ear canal of a human subject. The ear canal insert 3005 includes a first electrode 3030 to electrically contact skin within the ear canal of the subject. The first electrical connector 3040 may connect the electrode 3030 of the ear canal insert to a first electrical current source. The concha insert 3010 may be adapted to fit within a concha of the subject. The concha insert 3010 may include a base portion 3015 configured to fit within the cavum of the concha of the subject and a wing portion 3020 configured to fit within the cymba of the concha of the subject. In some embodiments, the concha insert 3010 may include a second electrode 3035 to electrically contact at least a portion of the concha of the subject. Although the electrode 3035 is shown on the wing portion 3020 in the aspect depicted in FIG. 30A, in other aspects, the electrode 3035 may be located on the base portion 3015 or located on both the base portion 3015 and the wing portion 3020. The second electrical connector 3025 may connect the electrode 3035 of the concha insert to a second electrical current source. In some embodiments, the first and second electrical current sources may be a common electrical current source.

In some embodiments, the nerve stimulation earpiece 3000 may include or otherwise be formed a disposable material. For example, the electrode 3030 or electrode 3035 may be disposable electrodes. In another example, a portion of or the entire ear canal insert 3005 and/or concha insert 3010 may be disposable. In some embodiments, the wing portion 3020 of the concha insert 3010 may include a soft, deformable, compliant, flexible, and/or resilient material. In some embodiments, the wing portion of 3020 the concha insert 3010 may include a rigid material. In some embodiments, the concha insert 3010 may include both a rigid material and a soft, deformable, compliant, flexible, and/or resilient material. In some embodiments, the base portion of the concha insert 3010 may include a hard material. In some embodiments, the base portion and the wing portion 3020 of the concha insert may be integrally formed with each other. In some embodiments, one or more components of the nerve stimulation earpiece 3000 may be constructed by a three-dimensional (3D) printer.

In some embodiments, at least a portion of the nerve stimulation earpiece 3000 (e.g., concha insert 3010 and/or ear canal insert 3005) may be formed for a specific subject. For example, at least a portion of the nerve stimulation earpiece 3000 may be custom made for a specific subject. In some embodiments, the nerve stimulation earpiece 3000 may include a material that may be initially compliant to mold to a subject's ear and then subsequently retains its shape. For example, the material may be a plastic that is softened by heat, placed in a subject's concha and/or ear canal to mold to the subject's ear. Once cooled, the plastic may retain its molded shape. In another example, the material may be an air-activated material that is initially compliant and hardens after exposure to air. In some embodiments, an impression of a subject's ear may be taken and a mold formed from the impression. The mold may be used to form the nerve stimulation earpiece 3000. In some embodiments, a 3D scan may be taken of the subject's ear and a mold may be generated from the 3D scan for forming the nerve stimulation earpiece 3000. In another embodiment, the nerve stimulation earpiece 3000 may be constructed directly from the 3D scan (e.g., 3D printer, milling machine).

In some embodiments, the first electrode 3030 may have an electrical contact area between about 190 mm² and about 380 mm². In some embodiments, the second electrode 3035 may have an electrical contact area between about 100 mm² and about 220 mm². All or a portion of the electrical contact area of the first electrode 3030 and/or second electrode 3035 may contact the skin of a subject. In some embodiments, the first electrode 3030 and/or second electrode 3035 may include a silver/silver chloride, platinum, tungsten, stainless steel, and/or gold component, conductive gel, hydrogel, conductive polymer, conductive foam, and/or fabric. In some embodiments, the first electrode 3030 and/or second electrode 3035 may include a layered structure including a hydrogel layer and a conductive polymer layer. In the example depicted in FIG. 30A, the second electrode 3035 includes a conductive polymer layer 3037 adjacent to a surface of the wing portion 3020 of the concha insert 3010 and a hydrogel layer 3036 adjacent to the polymer layer 3037. When the nerve stimulation earpiece 3000 is inserted into the ear of the subject, at least a portion of the hydrogel layer 3036 may contact the skin of the concha of the subject. In some embodiments, the first electrode 3030 and/or second electrode 3035 may include two or more materials, for example, a combination of two or more of the material described herein.

FIG. 30B depicts an exploded view of the nerve stimulation earpiece 3000 shown in FIG. 30A. In some embodiments, the ear canal insert 3005 may include a sound delivery portion 3045 adapted to deliver sound to the ear canal of the subject. In some embodiments, the sound delivery portion 3045 of the ear canal insert 3005 may include a channel through the ear canal insert to permit passage of sound through the ear canal insert 3005 to the ear canal of the subject from a speaker (not visible in FIGS. 30A-B) in the audio headphone 3050. In some embodiments, the sound delivery portion 3045 may include the speaker in the audio headphone 3050. In some embodiments, the nerve stimulation earpiece 3000 may include a hearing aid and/or the nerve stimulation earpiece 3000 may be included in a hearing aid. In some embodiments, the sound delivery portion 3045 may be a portion of the hearing aid.

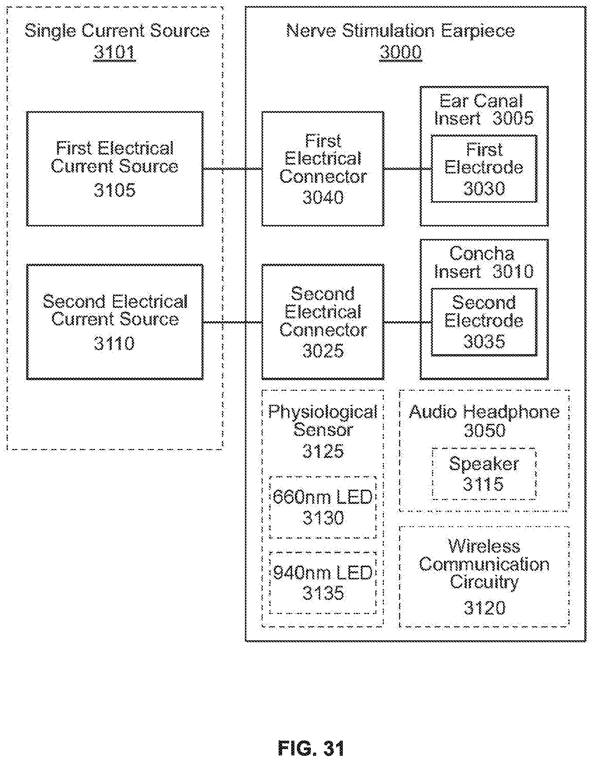

FIG. 31 depicts a block diagram of the nerve stimulation earpiece 3000 shown in FIGS. 30A-B. Although the audio headphone 3050, including a speaker 3115, is shown as a component of the nerve stimulation earpiece 3000 in FIG. 31, in some embodiments, the audio headphone 3050 may be a separate component operably coupled to the nerve stimulation earpiece 3000. As discussed previously in reference to FIG. 30A, the first electrical connector 3040 may connect the electrode 3030 of the ear canal insert to a first electrical current source 3105. The second electrical connector 3025 may connect the electrode 3035 of the concha insert 3010 to a second electrical current source 3110. In some embodiments, the first electrical current source 3105 and second electrical current source 3110 may be included in a separate device operably coupled to the nerve stimulation earpiece 3000 as shown in FIG. 31. In some embodiments, the first electrical current source 3105 and second electrical current source 3110 may be included in the nerve stimulation earpiece 3000. In some embodiments, the first electrical current source 3105 and the second electrical current source 3110 are the first and second terminals of a single electrical current source 3101. In some embodiments, the single electrical current source 3101 is located on the nerve stimulation earpiece 3000. In some embodiments, the single electrical current source 3101 is connected to the first electrical connector 3040 and the second electrical connector 3025 via a wired connection.

In some embodiments, the nerve stimulation earpiece 3000 may include wireless communication circuitry 3120. The wireless communication circuitry 3120 may be adapted to receive an audio signal. The wireless communication circuitry 3120 may transmit or receive a data signal. The audio signal and/or data signal may be transmitted and/or received from an audio device (e.g., CD player, mp3 player) and/or a personal computing device (e.g., tablet computer, mobile phone, smart watch, laptop). The wireless communication circuitry 3120 may include Bluetooth® communication circuitry in some embodiments.

In some embodiments, the nerve stimulation earpiece 3000 may include a physiological sensor 3125. In some embodiments, the physiological sensor 3125 may be a separate device operably coupled to the nerve stimulation earpiece 3000. The physiological sensor 3125 may receive and/or transmit a signal indicative of a physiological status of the subject. In some embodiments, a nerve stimulus provided by the nerve stimulation earpiece 3000 may be based, at least in part, on the signal received and/or transmitted by the physiological sensor 3125. The physiological sensor 3125 may include at least one of an electroencephalogram (EEG) sensor, a heart rate sensor, a moisture sensor, a temperature sensor, a bio sensor, a chemical sensor, electrocardiograph (ECG), motion sensor (e.g., accelerometer and/or gyroscope), electromyogram (EMG), pulse oximeter, galvanic response sensor, or a photoplethysmograph probe. Other physiological sensors may also be used to implement the physiological sensor 3125. In some embodiments, the nerve stimulation earpiece 3000 may include multiple physiological sensors 3125. In some embodiments, when the physiological sensor 3125 is implemented with a photoplethysmograph probe, the photoplethysmograph probe may include a 660nm red wavelength LED 3130 and a 940nm infrared wavelength LED 3135.

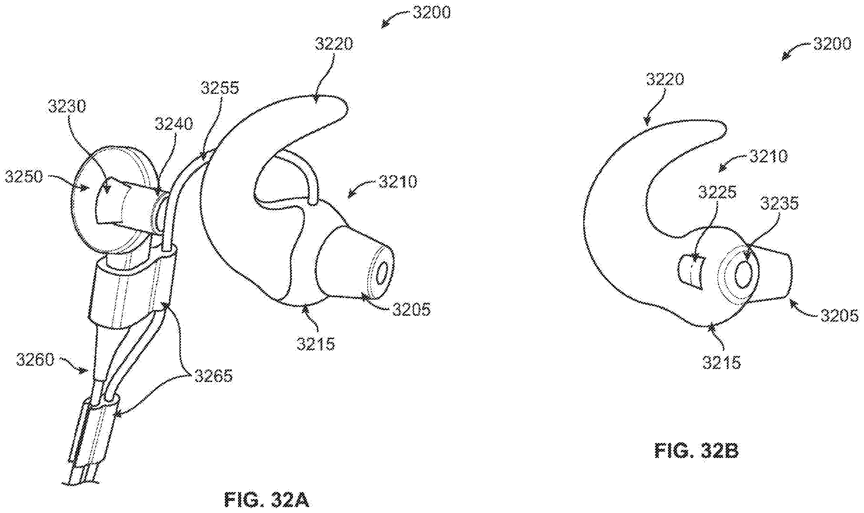

In some embodiments, the never stimulation earpiece includes an integral audio headphone for delivery of audio signals to a subject. In some embodiments, the nerve stimulation earpiece may include at least one mounting structure to physically mount the concha insert and/or the ear canal insert to a body structure of an audio headphone. The audio headphone may be a commercially available audio headphone (e.g., Bose® SoundSport®, Adidas® Monster® Sport, Jaybird X2) and the mounting structure of the nerve stimulation earpiece may be configured to mate with the commercially available audio headphone. In some embodiments, the audio headphone may be adapted to mate with the mounting structure of the nerve stimulation earpiece. FIGS. 32A-34 depict example configurations of the mounting structure included with the nerve stimulation earpiece and/or audio headphone.

FIGS. 32A-B depict a nerve stimulation earpiece 3200 including an example mounting structure. FIG. 32A depicts an audio headphone 3250 and the nerve stimulation earpiece 3200. A portion of the nerve stimulation earpiece 3200 that may contact the ear of a subject is visible in FIG. 32A. FIG. 32B depicts a portion of the nerve stimulation earpiece 3200 that may contact the audio headphone 3250 in some embodiments. The nerve stimulation earpiece 3200 may include an ear canal insert 3205 and a concha insert 3210 having base portion 3215 and a wing portion 3220. In some embodiments, the mounting structure may include a recess 3225 in the base portion 3215 of the concha insert 3210. The recess 3225 may receive a projecting portion 3230 of the body structure of an audio headphone 3250. In some embodiments, the mounting structure may include a recess coaxial 3235 within the ear canal insert 3205 to receive a projecting portion 3240 of the body structure of the audio headphone 3050. The recess 3235 may be formed in the interior of the ear canal insert 3205 in some embodiments. The mounting structure may include a resilient conductive element extending inward along at least a portion of the interior circumference of the recess 3225 and/or recess 3235. The mounting structure may make an electrical and/or a mechanical connection between the nerve stimulation earpiece 3200 and the audio headphone 3250 in some embodiments. In some embodiments, the one or more conductive elements of the mounting structure may be used to implement the first and/or second electrical connectors of the nerve stimulation earpiece.

In some embodiments, the nerve stimulation earpiece 3200 may include a wired connection 3255. The audio headphone 3250 may include a wired connection 3260 in some embodiments. The wired connection 3255 and the wired connection 3260 may be held together by one or more clips 3265. The clips may prevent or reduce tangling of the wired connections 3255, 3260. In some embodiments, the nerve stimulation earpiece 3200 and/or audio headphone 3250 may include wireless connections.

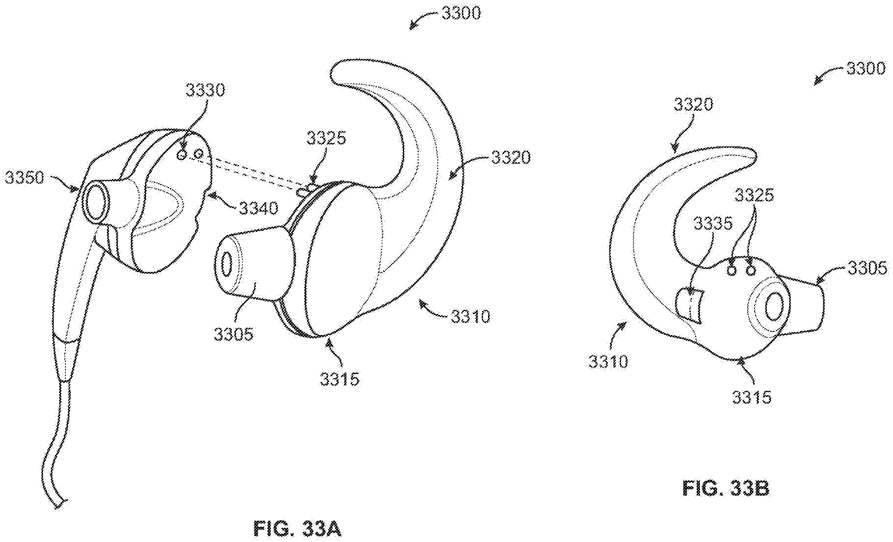

FIGS. 33A-B depict an example nerve stimulation earpiece 3300 including an example mounting structure. FIG. 33A depicts an audio headphone 3350 and the nerve stimulation earpiece 3300. A portion of the nerve stimulation earpiece 3300 that may contact the ear of a subject is visible in FIG. 33A. FIG. 33B depicts a portion of the nerve stimulation earpiece 3300 that may contact or interface with the audio headphone 3350 in some embodiments. The nerve stimulation earpiece 3300 may include an ear canal insert 3305 and a concha insert 3310 having base portion 3315 and a wing portion 3320. In some embodiments, the mounting structure may include at least one pin and/or socket 3325 on the base portion 3315 of the concha insert 3310 that may mate with a complementary socket and/or pin 3330 on the body structure of the audio headphone 3350. The pin and/or socket 3325 of the base portion 3315 on the concha insert 3310 may provide both electrical and mechanical connection of the base portion 3315 of the concha insert 3310 to the body structure of the audio headphone 3350. In some embodiments, the pin and/or socket elements of the mounting structure may be used to implement the first and/or second electrical connectors of the nerve stimulation earpiece. In some embodiments, the base portion 3315 of the concha insert 3310 may include a projecting portion 3335 that may be accepted by a recess 3340 on a body portion of the audio headphone 3350.

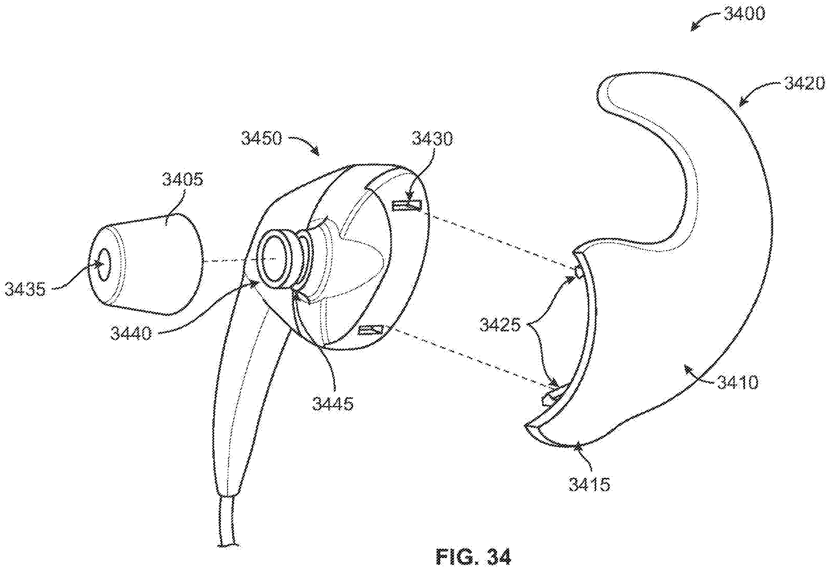

FIG. 34 depicts a nerve stimulation earpiece 3400 including an example mounting structure and an audio headphone 3450. The nerve stimulation earpiece 3400 may include an ear canal insert 3405 and a concha insert 3410 having base portion 3415 and a wing portion 3420. In some embodiments, the mounting structure may include at least one clip and/or socket 3425 on the base portion 3415 of the concha insert 3410 that may mate with a complementary socket and/or clip 3430 on the body structure of the audio headphone 3450. The clip and/or socket 3425 on the base portion 3415 of the concha insert 3410 may provide both electrical and mechanical connection of the base portion 3415 of the concha insert 3410 to the body structure of the audio headphone 3450. In some embodiments, the mounting structure may include at least one clip element that may mate with a complementary clip element on the body structure of the audio headphone. In some embodiments, the mounting structure may include a recess coaxial 3435 within the ear canal insert 3405 to receive a projecting portion 3440 of the body structure of the audio headphone 3450. The recess 3435 may be formed in the interior of the ear canal insert 3405 in some embodiments. The mounting structure may include a resilient conductive element extending inward along at least a portion of the interior circumference of the recess 3435. The conductive element may snap into a ring groove 3445 around an exterior circumference of the projecting portion 3440 of the body structure of the audio headphone 3450 to make electrical contact with a circular conductive element in the ring groove 3445 while making a mechanical connection with the ring groove 3445. In some embodiments, the clip, socket, and/or ring groove elements of the mounting structure may be used to implement the first and/or second electrical connectors of the nerve stimulation earpiece.

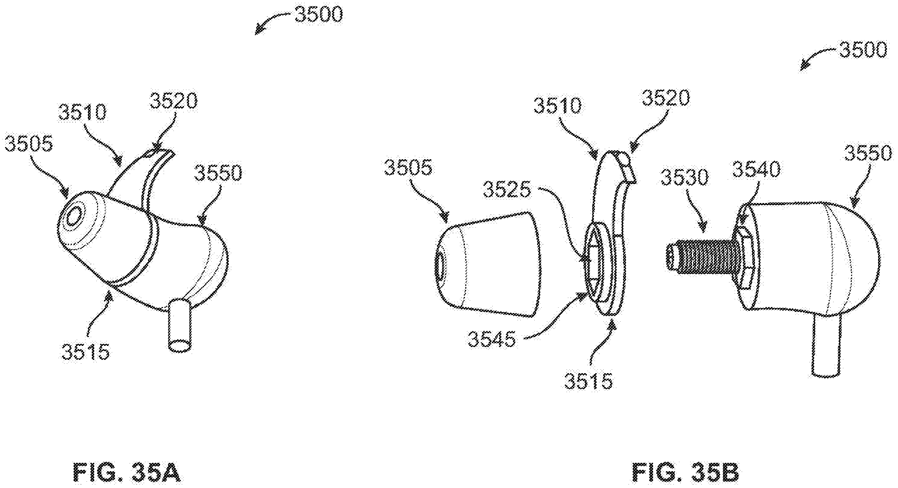

FIG. 35A depicts a nerve stimulation earpiece 3500 and an audio headphone 3550. In some embodiments, the audio headphone 3550 may be a component of the nerve stimulation earpiece 3500. The nerve stimulation earpiece 3500 may include an ear canal insert 3505 and a concha insert 3510 having base portion 3515 and a wing portion 3520. FIG. 35B depicts exploded view of the nerve stimulation earpiece 3500 and audio headphone 3550.

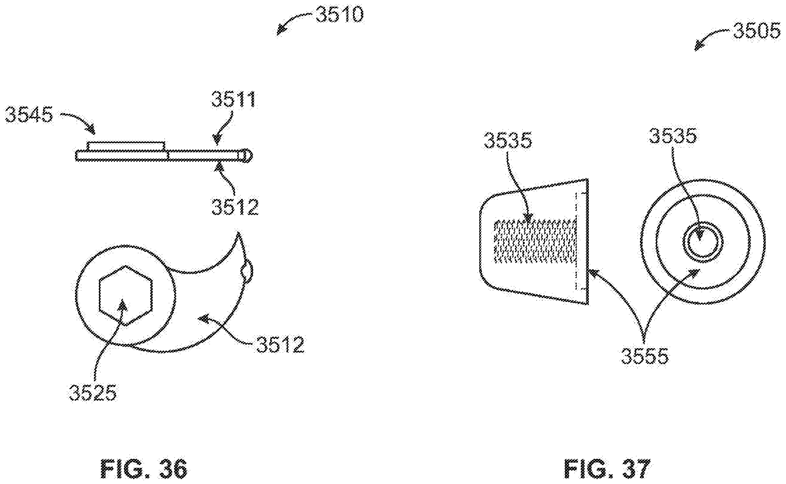

FIG. 36 depicts side and top plan views of the concha insert 3510. FIG. 37 depicts side and end views of the ear canal insert 3505. In some embodiments, the base portion 3515 of the concha insert 3510 may include a throughhole 3525. The body structure of the audio headphone 3550 and/or the ear canal insert 3505 may include a projection 3530 (FIG. 35B) configured to fit through the throughhole 3525 to mate with a complementary portion 3535 of the body structure of the other to secure the ear canal insert 3505 and the concha insert 3510 to the body structure of the audio headphone 3550. In other words, the ear canal insert 3505 and audio headphone 3550 may engage each other via the throughhole 3525 of the concha insert 3510 to secure the ear canal insert 3505 and the concha insert 3510 to the audio headphone 3550. In the example depicted in FIGS. 35A-37, the projection 3530 is included with the audio headphone 3550 and the complementary portion 3535 is included with the ear canal insert 3505. In some aspects, the projection 3530 and the complementary portion 3535 mate via a threaded connection. In some embodiments, the projection 3530 and the complementary portion 3535 mate via a friction fit. In some embodiments, the projection 3530 and the complementary portion 3535 mate via a snap fit.

In some embodiments, the throughhole 3525 has a non-circular shape and at least a portion 3540 of the projection 3530 has a non-circular shape complementary to the shape of the throughhole 3525. When the projection 3530 is fit into the throughhole 3525, the concha insert 3510 may be prevented from rotating with respect to the body structure of the audio headphone 3550. In some embodiments, the concha insert 3510 and the body structure of the audio headphone 3550 may have other or additional complementary mating features that may prevent rotation of the concha insert 3510 with respect to the body structure of the audio headphone 3550.

In some embodiments, the concha insert 3510 has a first face 3511 to face toward the concha of the subject and a second face 3512 to face away from the concha of the subject and toward the body structure of the audio headphone 3550. In some embodiments, the base portion 3515 of the concha insert 3510 and the ear canal insert 3505 may include complementary mating features 3545, 3555 that may permit assembly of the concha insert 3510 with the body structure of the audio headphone 3550 with the second face 3512 facing toward the body structure of the audio headphone 3550 and may prevent assembly of the concha insert 3510 to the body of the audio headphone with the first face 3511 facing toward the body structure of the audio headphone 3550. In some embodiments, the base portion 3515 of the concha insert 3510 and the body structure of the audio headphone 3550 may include complementary mating features that may permit assembly of the concha insert 3510 with the body structure of the audio headphone with the second face 3512 facing toward the body structure of the audio headphone 3550 and may prevent assembly of the concha insert 3510 to the body of the audio headphone 3550 with the first face 3511 facing toward the body structure of the audio headphone 3550. In some embodiments, the portion 3540 of the projection 3530 and the complementary mating feature 3545 may be complementary features.

Although FIGS. 32A-35B depict different embodiments of nerve stimulation earpieces, combinations of the embodiments may be used in some embodiments to implement the nerve stimulation earpiece 3000. For example, the ring groove 3445 of the nerve stimulation earpiece 3400 depicted in FIG. 34 may be implemented in combination with the pin and socket structure of the nerve stimulation earpiece 3300 depicted in FIG. 33A. The example nerve stimulation earpieces depicted in FIGS. 32A-35B or combinations thereof may be used to implement the nerve stimulation earpiece 3000 depicted in FIGS. 30A-B and 31 in some embodiments.

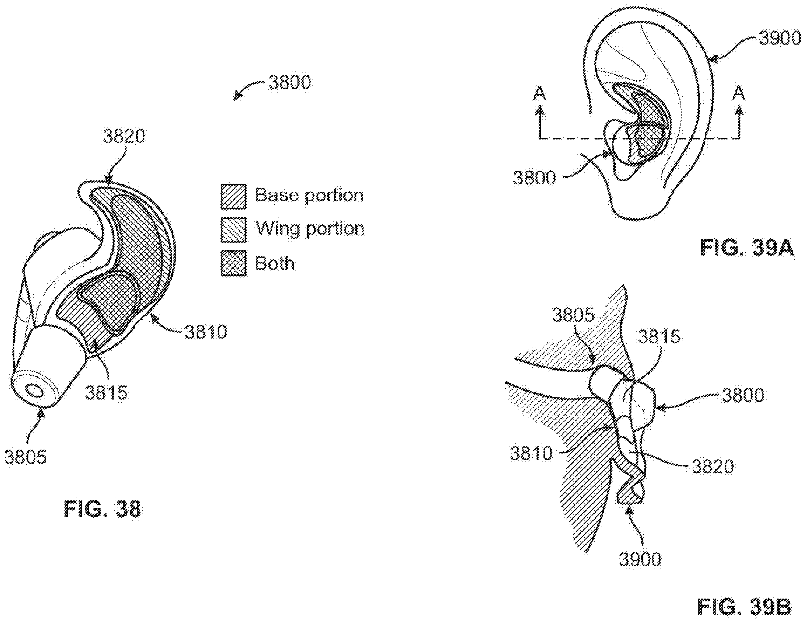

FIG. 38 depicts a nerve stimulation earpiece 3800. The nerve stimulation earpiece 3800 may be used to implement nerve stimulation earpiece 3000 in some embodiments. The nerve stimulation earpiece 3800 may include an ear canal insert 3805 and a concha insert 3810 having base portion 3815 and a wing portion 3820. The shading of different portions of the concha insert 3810 depicts an example of a boundary between the base portion 3815 and wing portion 3820. In some embodiments, an electrode may be included on at least a portion of the base portion 3815, at least a portion of the wing portion 3820, and/or at least a portion of both the base portion 3815 and the wing portion 3820. In some embodiments, the base portion 3815 and wing portion 3820 may be implemented using different materials. In some embodiments, the base portion 3815 and wing portion 3820 may be implemented using the same material.

FIGS. 39A-39B depict the nerve stimulation earpiece 3800 in the ear 3900 of a subject. FIG. 39A depicts an external side view of the nerve stimulation earpiece 3800 and ear 3900. FIG. 39B depicts a sectional view along the plane defined by line A-A. In some embodiments, the ear canal insert 3805 and concha insert 3810 together are configured to fit within one of the right ear or the left ear of the subject and not the other of the right ear or left ear of the subject. In some embodiments, the concha insert 3810 is shaped to fit within the concha of one of the right ear or the left ear of the subject and to not fit in the concha of the other of the right ear or the left ear of the subject. In other words, the ear canal insert 3805 and concha insert 3810 together and/or the concha insert 3810 alone may be designed specifically to fit in only either the left or right ear of the subject. In some applications, this may provide for a more comfortable fit for the subject. In some applications, this may provide improved electrical contact with the concha of the subject.

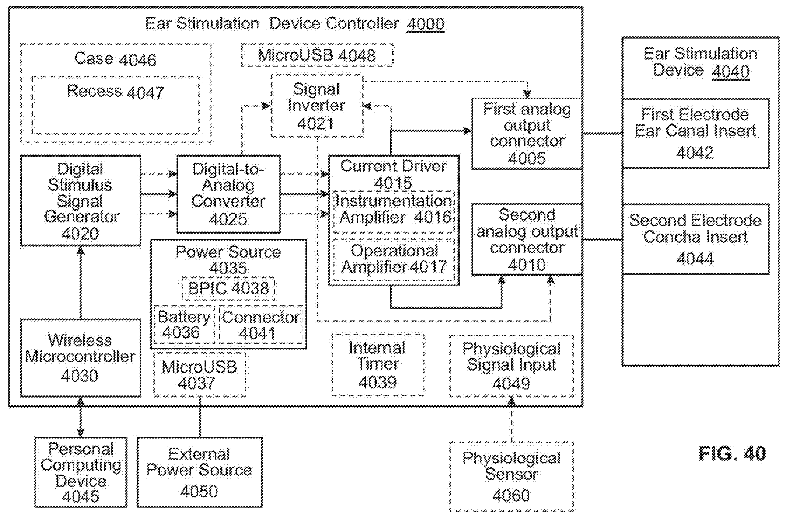

FIG. 40 depicts a block diagram of an ear stimulation device controller 4000 that may be employed to control any of the ear stimulation devices disclosed herein. The ear stimulation device controller 4000 may include a first analog output connector 4005, a second analog output connector 4010, a wireless microcontroller 4030, a digital stimulus signal generator 4020, a digital-to-analog converter (DAC) 4025, a current driver 4015, and a power source 4035. The first analog output connector 4005 may connect a first current signal to a first electrode 4042 of an ear stimulation device 4040, for example, the nerve stimulation earpiece 3000 depicted in Figure 30A. In some embodiments, the first electrode 4042 may be located on an ear canal insert of the ear stimulation device 4040. The second analog output connector 4010 may connect a second current signal to a second electrode 4044 of the ear stimulation device 4040. In some embodiments, the second electrode 4044 may be located on a concha insert of the ear stimulation device 4040.

The wireless microcontroller 4030 may control wireless communication between the ear stimulation device controller 4000 and a personal computing device 4045 to receive one or more stimulation parameters from the personal computing device 4045. Example personal computing devices include, but are not limited to, a smart phone, a mobile phone, a tablet computer, and an mp3 player. The digital stimulus signal generator 4020 may generate a digital stimulus signal based, at least in part, on the one or more stimulation parameters received from the personal computing device 4045. The DAC 4025 may convert the digital stimulus signal from the digital stimulus signal generator 4020 to an analog voltage waveform. The current driver 4015 may be operably connected to the DAC 4025 and generate a controlled current stimulus waveform responsive to the analog voltage waveform. The controlled current stimulus waveform may be provided to the ear stimulation device 4040 via the first analog output connector 4005 and the second analog output connector 4010. The power source 4035 may be operably connected to the wireless microcontroller 4030, digital stimulus signal generator 4020, the DAC 4025, and/or the current driver 4015.

In some embodiments, the wireless microcontroller 4030 may be a CC2650 microcontroller. An example of a wireless microcontroller that may be used to implement the wireless microcontroller 4030 is Texas Instruments CC2650 SimpleLink multi-standard ultra-low power wireless microcontroller unit for Bluetooth® communication. The wireless microcontroller 4030 may be compatible with a JTAG standard debugging interface in some embodiments. The wireless microcontroller 4030 may include a plurality of general purpose input/output pins in some embodiments. The wireless microcontroller 4030 may include a configurable serial peripheral interface in some embodiments. The wireless microcontroller 4030 may be a Bluetooth® controller in some embodiments.

The DAC 4025 may include two or more output channels in some embodiments. One or more of the output channels may produce an inverted signal relative to another of the output channels. The DAC 4025 may be an 8-bit, 10-bit, 12-bit, 14-bit, or 16-bit DAC. Other bit value converters may also be used to implement the DAC 4025. The DAC 4025 may be implemented as a single-channel or a multi-channel DAC. In some embodiments, the DAC 4025 may be implemented with a DAC7760. An example of a DAC that may be used to implement the DAC 4025 is Texas Instruments DAC7760 12-bit, single-channel, programmable current/voltage output DAC. Another example of a DAC that may be used to implement DAC 4025 is Texas Instruments Dual, Low Power, Ultra-low Glitch, Buffered Voltage Output DAC8163.

In some embodiments, the ear stimulation device controller 4000 may include a signal inverter 4021 to invert the analog voltage waveform output by the DAC 4025. The inverted signal may be provided by the signal inverter 4021 to the current driver 4015. In some embodiments, the ear stimulation device controller 4000 may include a signal inverter 4021 to invert the analog current wave form output by the current driver 4015. The signal inverter 4021 may provide the inverted signal to the first analog output connector 4005 and the second analog output connector 4010.

In some embodiments, the current driver 4015 may generate a controlled current output unaffected by load impedance. In some embodiments, the current driver 4015 may be implemented as an XTR300 analog current/voltage output driver. An example of a current driver that may be used to implement the current driver 4015 is Texas Instruments XTR300 Industrial Analog Current/Voltage Output Driver. The current driver 4015 may provide biphasic current stimulation in some embodiments. In some embodiments, the current driver 4015 may provide a current output between -100mA and 100mA. In some embodiments, the current driver 4015 may provide a current output between -20mA and +20mA. In some embodiments, the current output may be at a maximum voltage of +/-40V. In some embodiments, the current output may be at a maximum voltage of +/-10V. In some embodiments, the current driver 4015 may receive a power input of +/-15V. In some embodiments, the current driver 4015 may receive an input signal voltage of +/- VDD-3 and an external reference voltage. The current driver 4015 may supply over temperature, overcurrent, and common-mode over-range error signals in some embodiments.

In some embodiments, the current driver 4015 may include an internal instrumentation amplifier 4016 to supply a copy of the stimulating current through a set resistor via an IA channel. In some embodiments, the current driver 4015 may include an internal operational amplifier 4017 configured to supply a 1/10^{th} current copy. In some embodiments, the current driver 4015 may provide current to two or more stimulus channels that are electrically isolated from each other.

In some embodiments, the current driver 4015 may generate current pulses that fall within safety ranges and/or comply with safety regulations (e.g., FDA regulations). The current driver 4015 may generate current pulses of no more than 200Hz, 500Hz, 1kHz, or another maximum frequency. The current driver 4015 may generate current pulses having a pulse duration of no more than 2ms in some embodiments. The current driver 4015 may generate current pulses having a voltage of no more than +/- 12V. In some embodiments, the current driver 4015 may generate current pulses having a voltage compliance of no more than +/-10V. In some embodiments, the current driver 4015 may generate current pulses with a maximum average current of no more than 10mA. The current driver 4015 may generate current pulses with a maximum primary depolarizing phase duration of no more than 500ms in some embodiments. The current driver 4015 may generate current pulses with a maximum direct current of no more than 100mA during a non-pulse or device failure in some embodiments.

In some embodiments, the power source 4035 includes a battery 4036. The battery 4036 may be implemented as a 3.7V Li-Polymer battery. The ear stimulation device controller 4000 may include a microUSB port 4037 connected to the battery 4036. The microUSB port 4037 may be configured to connect the battery 4036 to an external power source 4050 for recharging. The power source 4035 may supply 3.3V in some embodiments. The power source 4035 may include a battery protection integrated circuit 4038 in some embodiments. An example of a battery protection integrated circuit that may be used to implement the battery protection integrated circuit 4038 is the Texas Instruments BQ2970 voltage and current protection integrated circuit. Another example of a battery protection integrated circuit that may be used to implement the battery protection integrated circuit 4038 is the Microchip MCP7383X Li-Ion System Power Path Management Reference. The power source 4035 may provide voltage and current protection in the event of erratic behavior, overcharging, or energy depletion in the battery. The power source 4035 may automatically shut off current flow upon detection of an overcharge, over-discharge, or a short circuit. The power source 4035 may resume operation when an internal timer expires. In some embodiments, the ear stimulation device controller may include an internal timer 4039.

In some embodiments, the power source may include a connector 4041. In some embodiments, the connector 4041 is a microUSB connector configured to connect to a power output of a mobile phone via a microUSB port. In some embodiments, the connector 4041 is an audio jack connector configured to connect a power output of a mobile phone via a phone audio jack. In some embodiments, the power output of the mobile phone is provided by a battery included in the mobile phone.

In some embodiments, the personal computing device 4045 may be a mobile phone. The mobile phone may include a microUSB connection, an audio jack, and/or wireless connections. In some embodiments, the wireless microcontroller 4030 may communicate one or more data signals between the ear stimulation device controller 4000 and a mobile phone via a microUSB connection. In some embodiments, the wireless microcontroller 4030 may communicate one or more data signals between the ear stimulation device controller 4000 and a mobile phone via a 2.4 GHz Bluetooth® connection. The wireless microcontroller 4030 may communicate one or more data signals between the ear stimulation device controller 4000 and the mobile phone via an audio jack of the phone. The wireless microcontroller 4030 may communicate one or more data signals between the ear stimulation device controller 4000 and the mobile phone via a wireless connection.

In some embodiments, the ear stimulation device controller 4000 may include a case 4046 that includes a recess 4047 to receive a personal computing device. The recess may be configured to receive a mobile phone or a tablet computer. The ear stimulation device controller 4000 may include an attachment means other than the recess 4047 for attaching the case 4046 to the personal computing device in some embodiments. In some embodiments, the case 4046 may be a cell phone case that includes recess 4047 to receive a cell phone. The ear stimulation device controller 4000 may include a microUSB connector 4048 to mate with a microUSB port of the cell phone.

In some embodiments, the ear stimulation device controller 4000 may include a physiological signal input 4049 that may receive a physiological signal. The physiological signal input 4049 may receive a signal indicative of a physiological status of the user. In some embodiments, the output of the digital stimulus signal generator 4020 and/or current driver 4015 may be based, at least in part, on the signal received by the physiological signal input 4049. The first and second output connectors 4005, 4010 may be connected to the ear stimulation device 4040 located on a first ear of the user and the physiological signal input may receive the physiological signal from a physiological sensor 4060 located on a second ear of the user.

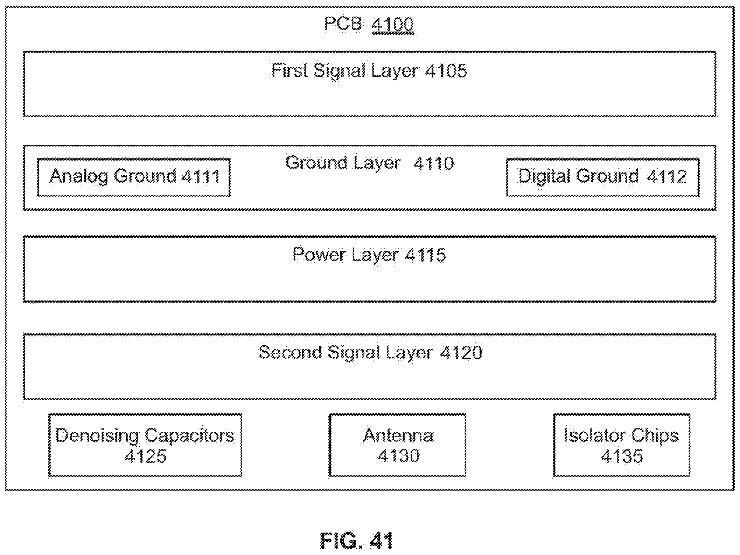

FIG. 41 depicts a block diagram of a printed circuit board (PCB) 4100 of the ear stimulation device controller 4000. In some embodiments, the ear stimulation device controller 4000 may include and/or be implemented as PCB 4100. In some embodiments, the PCB 4100 may have components mounted on one or more sides. In some embodiments, the PCB may include a standard FR-4 substrate. The PCB 4100 may include a first signal layer 4105, a ground layer 4110, a power layer 4115, and a second signal layer 4120. In some embodiments, the PCB 4100 may have components inset into one or more of the layers. The PCB 4100 may include separate analog ground 4111 and digital ground 4112 areas, which may avoid or reduce the introduction of noise. The PCB 4100 may include one or more denoising capacitors 4125. The denoising capacitors 4125 may be included in a separate layer and/or in one or more of the layers of the PCB 4100. The PCB 4100 may include an antenna 4130. The antenna 4130 may be included in a separate layer and/or in one of the layers of the PCB 4100. The PCB may include one or more isolator chips 4135 located between the wireless microcontroller 4030 and the DAC 4025. The wireless microcontroller 4030 and DAC 4025 may be located in the first signal layer 4105, the second signal layer 4120, and/or one may be located in the first signal layer 4105 and the other in the second signal layer 4120. The one or more isolator chips 4135 may be located in the same layer as the wireless microcontroller 4030 and/or the DAC 4025 and/or in a different layer of the PCB 4100. Example isolator chips that may be used to implement the one or more isolator chips 4135 are Texas Instruments ISO7631FC 4kVpk Low Power Triple-Channel 25 Mbps digital isolator and Texas Instruments ISO7310FC ECM Low Power Single-Channel 1/0 digital isolator.

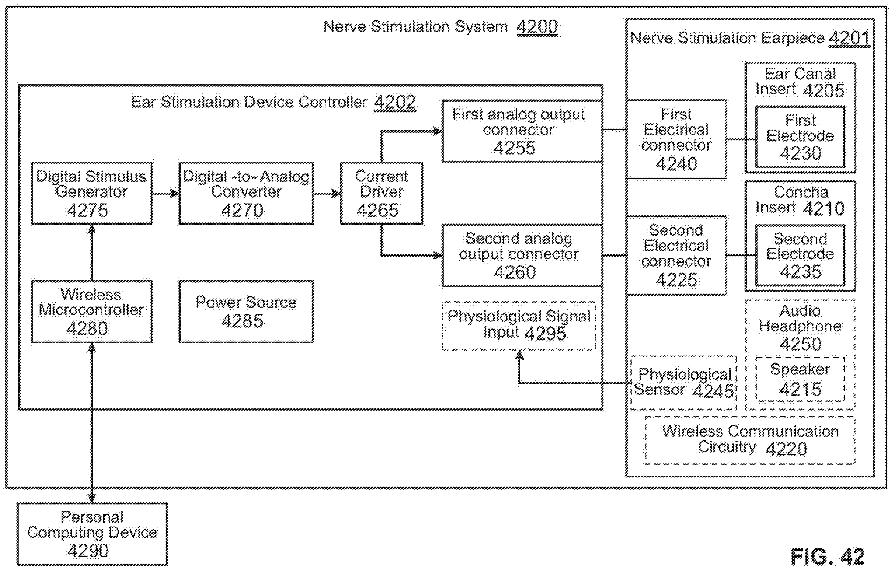

FIG. 42 depicts a block diagram of a nerve stimulation system 4200 including a nerve stimulation earpiece 4201 operably coupled to an ear stimulation device controller 4202. The nerve stimulation earpiece 4201 may include an ear canal insert 4205, a concha insert 4210, a first electrical connector 4240, and a second electrical connector 4225. In some embodiments, the nerve stimulation earpiece 4201 may include or may be configured to couple to an audio headphone 4250, which may include a speaker 4215.

The ear canal insert 4205 may be adapted to fit into an ear canal of a human subject. The ear canal insert 4205 may include a first electrode 4230 to electrically contact skin within the ear canal of the subject. The first electrical connector 4240 may connect the electrode 4230 of the ear canal insert 4205 to a first analog output connector 4255 of an ear stimulation device controller 4202. The concha insert 4210 may be adapted to fit within a concha of the subject. The concha insert 4210 may include a base portion configured to fit within the cavum of the concha of the subject and a wing portion configured to fit within the cymba of the concha of the subject. In some embodiments, the concha insert 4210 may include a second electrode 4235 to electrically contact at least a portion of the concha of the subject. The second electrical connector 4225 may connect the electrode 4235 of the concha insert to a second analog output connector 4260 of the ear stimulation device controller 4202.

In some embodiments, the nerve stimulation earpiece 4201 may include wireless communication circuitry 4220. The wireless communication circuitry 4220 may be adapted to receive an audio signal. The wireless communication circuitry 4220 may transmit or receive a data signal. The audio signal and/or data signal may be transmitted and/or received from an audio device (e.g., CD player, mp3 player), a personal computing device 4290 (e.g., tablet computer, mobile phone, smart watch, laptop), and/or the ear stimulation device controller 4202.

In some embodiments, the nerve stimulation earpiece 4201 may include a physiological sensor 4245. In some embodiments, the physiological sensor 4245 may be a separate device operably coupled to the nerve stimulation earpiece 4201. The physiological sensor 4245 may transmit and/or receive a signal indicative of a physiological status of a subject. The physiological sensor 4245 may include at least one of an electroencephalogram (EEG) sensor, a heart rate sensor, a moisture sensor, a temperature sensor, a bio sensor, a chemical sensor, electrocardiograph (ECG), motion sensor (e.g., accelerometer and/or gyroscope), electromyogram (EMG), pulse oximeter, galvanic response sensor, or a photoplethysmograph probe. Other physiological sensors may also be used to implement the physiological sensor 4245. In some embodiments, the nerve stimulation earpiece 4201 may include multiple physiological sensors 4245. The physiological sensor may be coupled to a physiological signal input 4295 of the ear stimulation device controller 4202 in some embodiments. The physiological signal input 4295 may receive the signal from the physiological sensor 4245. In some embodiments, an output of a digital stimulus signal generator 4275 and/or current driver 4265 of the ear stimulation device controller 4202 may be based, at least in part, on the signal received by the physiological signal input 4295 from the physiological sensor 4245.

The ear stimulation device controller 4202 may include a first analog output connector 4255, a second analog output connector 4260, a wireless microcontroller 4280, a digital stimulus signal generator 4275, a digital-to-analog converter (DAC) 4270, a current driver 4265, and a power source 4285. The first analog output connector 4255 may connect a first current signal to the first electrode 4230 of nerve stimulation earpiece 4201 via first electrical connector 4240. The second analog output connector 4260 may connect a second current signal to the second electrode 4235 of nerve stimulation earpiece 4201 via second electrical connector 4225.

The wireless microcontroller 4280 may control wireless communication between the ear stimulation device controller 4202 and the personal computing device 4290 to receive one or more stimulation parameters from the personal computing device 4290. Example personal computing devices include, but are not limited to, a smart phone, a mobile phone, a tablet computer, and an mp3 player. The digital stimulus signal generator 4275 may generate a digital stimulus signal based, at least in part, on the one or more stimulation parameters received from the personal computing device 4290. The DAC 4270 may convert the digital stimulus signal from the digital stimulus signal generator 4275 to an analog voltage waveform. The current driver 4265 may be operably connected to the DAC 4270 and generate a controlled current stimulus waveform responsive to the analog voltage waveform. The controlled current stimulus waveform may be provided to the nerve stimulation earpiece 4201 via the first analog output connector 4255 and the second analog output connector 4260. The power source 4285 may be operably connected to the wireless microcontroller 4280, digital stimulus signal generator 4275, the DAC 4270, and/or the current driver 4265.

In some embodiments, the nerve stimulation earpiece 4201 may be implemented with nerve stimulation earpiece 3000. In some embodiments, the ear stimulation device controller 4202 may be implemented with ear stimulation device controller 4000.

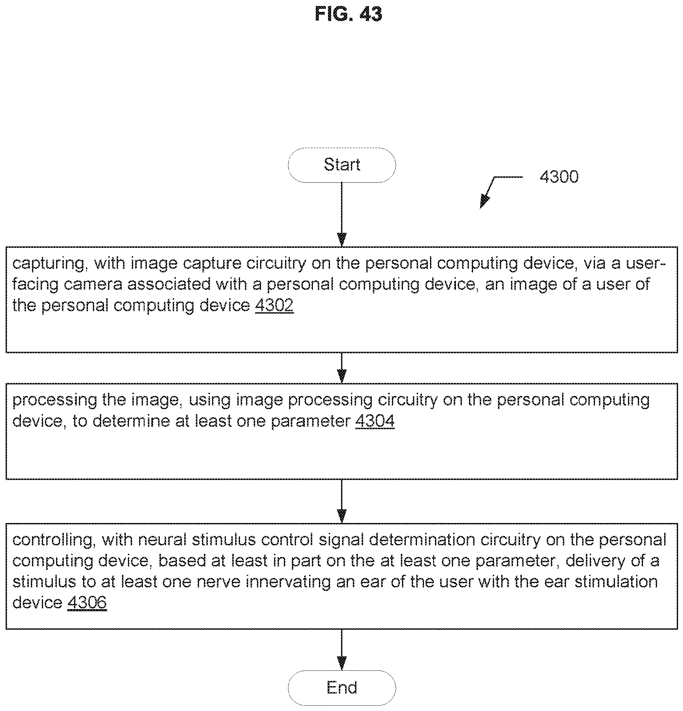

FIG. 43 is a flow diagram of a method of controlling an ear stimulation device with a personal computing device. The ear stimulation device is a wearable neural stimulation device as described elsewhere herein (e.g. wearable ear stimulation device 202 controlled with personal computing device 208, as depicted in FIGS. 2A and 2B), for delivering a stimulus to an ear of a user of the personal computing device to stimulate at least one nerve innervating the ear. In an aspect, the ear stimulation device includes an earpiece that incorporates a positioning element and a neural stimulator, various examples of which are described and depicted herein. In an aspect, the ear stimulation device is part of a system that is used for delivering sound (e.g., music), and the earpiece includes a speaker or other sound source. In an aspect, the system includes a pair of earpieces. In an aspect, only one of the earpieces includes a neural stimulator, but both earpieces include a speaker or other sound source. In another aspect, both earpieces include both neural stimulator and sound source. In another aspect, one earpiece includes a sound source and the other includes a neural stimulator. In an aspect, method 4300 in FIG. 43 includes capturing, with image capture circuitry on the personal computing device, via a user-facing camera associated with a personal computing device, an image of a user of the personal computing device, as indicated at 4302; processing the image, using image processing circuitry on the personal computing device, to determine at least one parameter as indicated at 4304; and controlling, with neural stimulus control signal determination circuitry on the personal computing device, based at least in part on the at least one parameter, delivery of a stimulus to at least one nerve innervating an ear of the user with the ear stimulation device, as indicated at 4306.

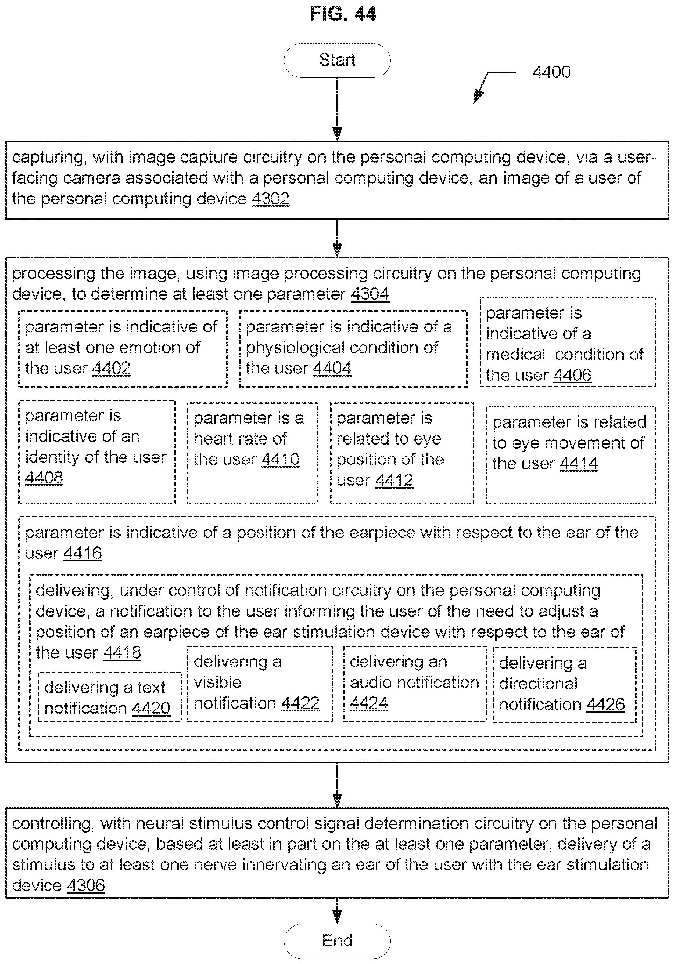

FIGS. 44 - 46 depict further aspects of the method of FIG. 43, wherein steps 4302, 4304, and 4306 are as depicted and described in connection with FIG. 43. As depicted in FIG. 44, in further aspects of method 4400, the at least one parameter is indicative of at least one emotion of the user 4402, is indicative of a physiological condition of the user 4404, is indicative of a medical condition of the user 4406, is indicative of an identity of the user 4408, is a heart rate of the user 4410, is related to eye position of the user 4412, is related to eye movement of the user 4414 of the user, or is indicative of a position of the earpiece with respect to the ear of the user 4416. Various schemes for identifying or classifying emotions have been devised, and the meaning of the term, as used herein, is not tied to any specific scheme. Examples of emotions include, but are not limited to, e.g. depression, anxiety, agitation, happiness, sadness, excitement, fear, and anger. In various aspects, a physiological condition of the subject is indicative of a medical condition of the subject. Medical conditions of the subject, include, for example, muscle spasm, seizure, epilepsy (e.g., seizure, spasm, staring), drowsiness, lethargy, fatigue, pain, fever, hypertension (e.g., sweating, flushing), hypotension, or mental state.

Determining a parameter indicative of a position of the earpiece with respect to the ear of the user can include, for example, determining a distance of one or more portion of the earpiece with respect to various anatomical features of the ear, e.g., the ear canal, the tragus, the helix, the lobe, etc., to determine whether the earpiece is positioned on the appropriate portion of the pinna or inserted far enough into the ear canal, for example. In an aspect, method 4400 further includes delivering, under control of notification circuitry on the personal computing device, a notification to the user informing the user of the need to adjust a position of an earpiece of the ear stimulation device with respect to the ear of the user, as indicated at 4418. In various aspects, delivering a notification includes delivering a text notification 4420, delivering a visible notification 4422, or delivering an audio notification 4424. The notification can be specific (e.g., a text or audio notification instructing the user to "push the earpiece further into the ear canal" or "move the earpiece higher up on the pinna") or non-specific (e.g., a flashing light or beeping sound that indicates the need to reposition the earpiece without providing detail on how specifically it should be repositioned). In an aspect, delivering a notification includes delivering a directional notification 4426. As used herein, the term "directional notification" refers a notification that provides information to the user regarding the direction of movement needed to move the earpiece to the proper position. For example, in an aspect, the notification includes a text or audio notification as described above, which instructs the user to "push the earpiece further into the ear canal" or "move the earpiece higher up on the pinna." In another aspect, the notification includes a tone that changes in pitch as the earpiece moves toward or away from the proper location, or a click or other pulsed sound that is repeated at a frequency that changes as the earpiece moves toward or away from the proper location. In an aspect, the tone can change (i.e. changing to another tone, or stopping entirely) when the earpiece is in the proper location.

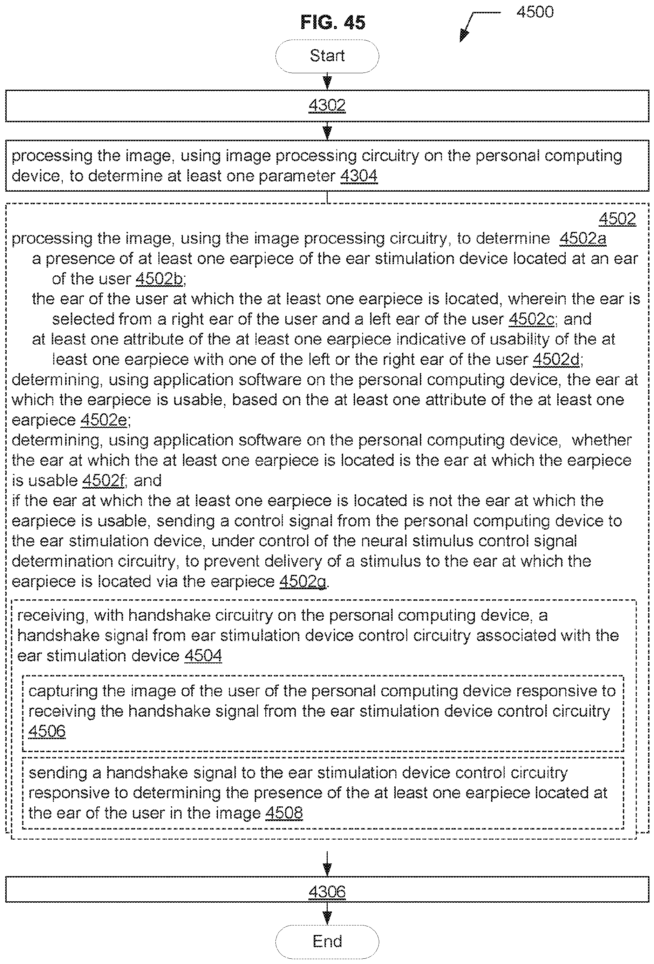

The vagus innervation of the ears is not strictly symmetrical; for example, the right ear, unlike the left ear, is innervated by a branch of the vagus nerve that, when stimulated, influences heart rate. Accordingly, in some circumstances it may be preferred to stimulate the left, but not the right ear, to avoid affecting the heart rate of the user. Therefore, if the system includes two earpieces (e.g., for the purpose of delivering music or other audio to both the left and right ear), in an aspect, only one of the earpieces includes a neural stimulator. For example, the earpiece with the neural stimulator is then considered to be usable on the left ear, but not the right ear. In some aspects, the two earpieces are shaped differently such that one fits the left, but not the right ear and the other fits the right, but not the left ear. In other aspects, the two earpieces are shaped such that they fit on either ear. In such a situation, the two earpieces may be distinguished from each other based on shape or color, or by inclusion of indicia on the earpiece or associated cable, and the ear stimulation device control system. A method 4500 as outlined in FIG. 45, which is a further variant of the method of FIG. 43, can be used in connection with the ear stimulation device to ensure that the earpiece with the neural stimulator is used only with the ear with which it is considered to be usable. Method 4500, includes at 4502 the following steps: processing the image, using the image processing circuitry, to determine (at 4502a) the presence of at least one earpiece of the ear stimulation device located at an ear of the user, as indicated at 4502b; the ear of the user at which the at least one earpiece is located, wherein the ear is selected from a right ear of the user and a left ear of the user, as indicated at 4502c, and at least one attribute of the at least one earpiece indicative of usability of the at least one earpiece with one of the left or the right ear of the user, as indicated at 4502d; determining, using application software on the personal computing device, the ear at which the earpiece is usable, based on the at least one attribute of the at least one earpiece 4502e; determining, using application software on the personal computing device, whether the ear at which the at least one earpiece is located is the ear at which the earpiece is usable 4502f; and, if the ear at which the at least one earpiece is located is not the ear at which the earpiece is usable, sending a control signal from the personal computing device to the ear stimulation device, under control of the neural stimulus control signal determination circuitry, to prevent delivery of a stimulus to the ear at which the earpiece is located via the earpiece, as indicated at 4502g. In a further aspect, method 4500 includes receiving, with handshake circuitry on the personal computing device, a handshake signal from ear stimulation device control circuitry associated with the ear stimulation device, as indicated at 4504. This may include capturing the image of the user of the personal computing device responsive to receiving the handshake signal from the ear stimulation device control circuitry, as indicated at 4506. In an aspect, method 4500 includes sending a handshake signal to the ear stimulation device control circuitry responsive to determining the presence of the at least one earpiece located at the ear of the user in the image, as indicated at 4508.

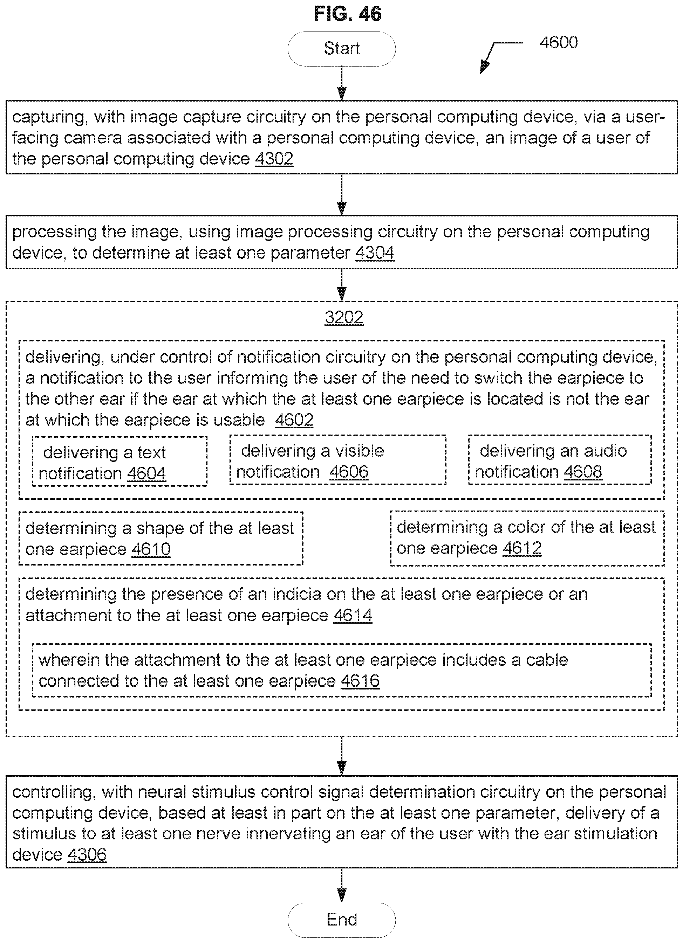

FIG. 46 depicts method 4600, providing further detail regarding the method of FIG. 45, with step 4502 in FIG. 46 the same as in FIG. 45. In an aspect, method 4600 includes delivering, under control of notification circuitry on the personal computing device, a notification to the user informing the user of the need to switch the earpiece to the other ear if the ear at which the at least one earpiece is located is not the ear at which the earpiece is usable, as indicated at 4602. Delivering the notification to the user may include, for example, one or more of delivering a text notification, at 4604, delivering a visible notification, at 4606, or delivering an audio notification, at 4608. In various aspects, determining the at least one attribute 3433 of the at least one earpiece includes determining a shape of the at least one earpiece, at 4610, or determining a color of the at least one earpiece, at 4612, for example. In an aspect, determining the at least one attribute 3433 of the at least one earpiece includes determining the presence of an indicia on the at least one earpiece or an attachment to the at least one earpiece, as indicated at 4614. In an aspect, the attachment to the at least one earpiece includes a cable connected to the at least one earpiece, as indicated at 4616.

An alternative approach to addressing the usability of neural stimulation with the right ear versus the left ear is to include stimulation electrodes in both earpieces, but send a neural stimulus control signal to cause delivery of a neural stimulus only via one of the earpieces (e.g., the left ear). In an aspect, a neural stimulus control signal is sent to only one of the earpieces. In an aspect, this is done if separate neural stimulus control signal outputs are provided for the two earpieces. In another aspect, a neural stimulus control signal is sent to both earpieces, but causes delivery of stimulus via only one of the earpieces. This can be done, e.g., by including ear stimulation electrical circuitry in the two earpieces that produces a neural stimulus in response to different neural stimulus control signals. For example, the neural stimulus control signal can have one or more characteristics (e.g., frequency, polarity, activation code) that cause activation of ear stimulation device control circuitry in one but not the other of the two earpieces. It is assumed that it is known *a priori* that a particular earpiece (as identified by the attribute determined in the user image) will receive and be activated by the neural stimulus control signal.

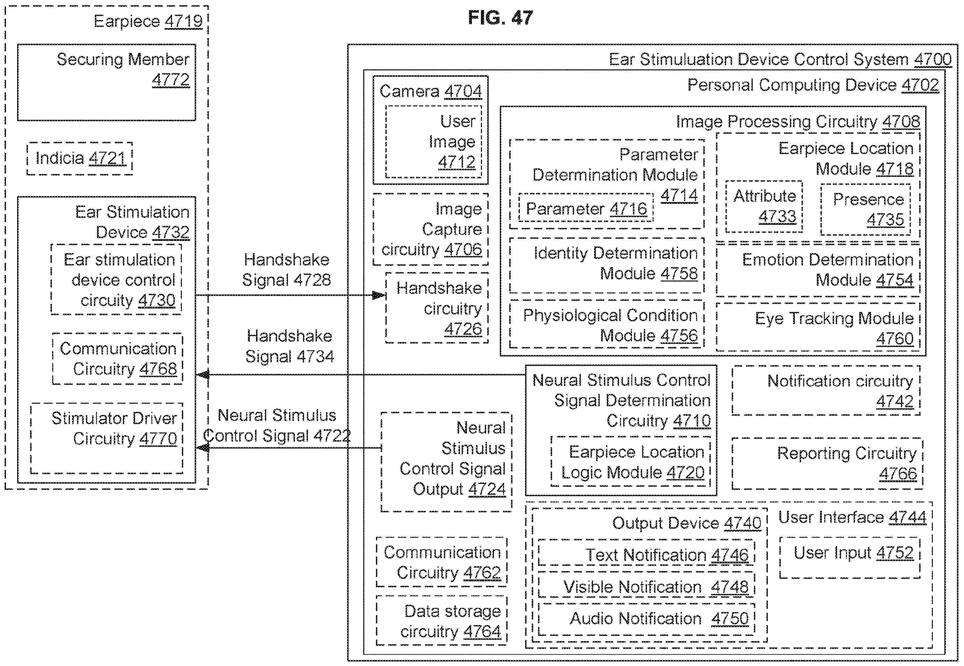

FIG. 47 is a block diagram of an ear stimulation device control system 4700. System 4700 includes a personal computing device 4702, a user-facing camera 4704 associated with the personal computing device, image capture circuitry 4706, image processing circuitry 4708, and neural stimulus control signal determination circuitry 4710. In various aspects, personal computing device 4702 is a phone, watch, wearable device, tablet computer, laptop computer, or desktop computer, for example.

Image capture circuitry 4706 is adapted to capture an image 4712 of a user of personal computing device 4702 from user-facing camera 4704. In an aspect, user-facing camera is built into the personal computing device. In another aspect, user-facing camera 4704 is connected to personal computing device via either a wired or wireless connection. Image processing circuitry 4708 is configured to process image 4712, using parameter determination module 4714, to determine at least one parameter 4716. Neural stimulus control signal determination circuitry 4710 is configured to control delivery of a stimulus to at least one nerve innervating an ear of the user with an ear stimulation device 4732, based at least in part on the at least one parameter 4716. In various aspects, parameter 4716 is indicative of one or more of at least one emotion of the user, a physiological condition of the user, an identity of the user, or a heart rate of the user. In an aspect, parameter 4716 is related to an eye position or eye movement of the user. In an aspect, parameter 4716 is indicative of a position of the earpiece with respect to the ear of the user

In an aspect, image processing circuitry 4708 includes earpiece location module 4718, which is configured to process image 4712 to determine the presence 4735 of at least one earpiece 4719 of the ear stimulation device 4732 located at an ear of the user; the ear of the user at which the at least one earpiece is located, the ear selected from a right ear of the user and a left ear of the user; and at least one attribute 4733 of the at least one earpiece indicative of usability of the at least one earpiece 4719 with one of the left or the right ear of the user, as discussed herein above. Attribute 4733 may be, for example, an indicia 4721 used to indicate that the earpiece in question includes a neural stimulator, for example. In an aspect, neural stimulus control signal determination circuitry 4710 is configured (with earpiece location logic module 4720) to determine the ear at which the earpiece is usable, based on the at least one attribute 4733 of the at least one earpiece; determine whether the ear at which the at least one earpiece is located is the ear at which the earpiece is usable; and if the ear at which the at least one earpiece is located is not the ear at which the earpiece is usable, send a control signal from the personal computing device to the ear stimulation device (neural stimulus control signal 4722 from neural stimulus control signal output 4724) to prevent delivery of the stimulus to the a least one nerve innervating the ear of the user. As discussed herein above, in some circumstances it may be preferred to stimulate the left, but not the right ear, for example. Hence, in a system that includes two earpieces, the two earpieces may be distinguished from each other based on shape or color, or by inclusion of indicia on the earpiece or associated cable. In various aspects, indicia include any sort of markings detectable in the image via image processing. Indicia may include solid colored or patterned markings on an earpiece, or may include a characteristic of the earpiece itself (e.g., the color of the material of which the earpiece is made). Indicia may be detectable in the visible spectrum, or at other wavelengths. In some aspects, indicia may include text, alphanumeric markings, or symbols. The right and left ears of the user may be identified in user image 4712, using image processing methods, e.g. as described in M.M. Fakhir et al., "Face Recognition Based on Features Measurement Technique," 2014 UKSim-AMSS 8th European Modelling Symposium, pp. 158-162; U.S. Patent Application Publication No. 2016/0026781 to Boczek et al.; and U.S. Patent Application Publication No. 2008/0285813 to Holm. In some aspects, the position of one or both ears with respect to the face is determined; in some aspects, the shape and/or features of one or both ears is determined.

In an aspect, ear stimulation device control system 4700 includes handshake circuitry 4726 adapted to receive a handshake signal 4728 from ear stimulation device control circuitry 4730 associated with the ear stimulation device 4732. In an aspect, image capture circuitry 4706 is adapted to capture the image 4712 of the user of the personal computing device 4702 responsive to receiving the handshake signal 4728 from the ear stimulation device control circuitry 4730. For example, in an aspect exchange of information between the ear stimulation device control system 4700 and ear stimulation device 4732 is initiated after user image 4712 has been captured and evaluated by image processing circuitry 4708, and it has been determined, by earpiece location logic module 4720, that the earpiece containing the ear stimulation device has been placed on the appropriate ear of the user. Alternatively, if two earpieces including ear stimulation devices are utilized, the handshake signal from each earpiece can include an earpiece identification code for identifying the earpiece. The earpiece identification code and indicia associated with a particular earpiece can be linked in a lookup table stored in data storage circuitry 4764, for example.

In an aspect, the neural stimulus control signal determination circuitry 4710 is configured to send a handshake signal 4734 to ear stimulation device control circuitry 4730 responsive to determining the presence 4735 of the at least one earpiece located at the ear of the user in the image.

In an aspect, ear stimulation device control system includes output device 4740, and notification circuitry 4742, which is adapted to provide a notification via output device 4740 instructing the user of to switch the earpiece to the other ear if the ear at which the at least one earpiece is located is not the ear at which the earpiece is usable. For example, output device 4740 may be part of user interface 4744. In an aspect, output device is adapted to deliver a text notification 4746 to the user (e.g., output device includes an LED or LCD display, 7-segment display, or other alphanumeric display). In another aspect, output device 4740 is adapted to deliver a visible notification 4748 to the user, which may include a text display, as described previously, a graphic or symbol presented on a display, or a light that can be illuminated, flashed, etc. to attract the attention of the user. In an aspect, output device 4740 is adapted to deliver an audio notification 4750 to the user (e.g., output device 4740 includes a speaker, bell, buzzer, or other audio source for delivering one or both of a verbal notification or an alarm tone). User interface 4744 may also include one or more user input 4752, of various types, e.g. as discussed elsewhere herein.

As noted above, image processing circuitry 4708 includes earpiece location module 4718, which is configured to process image 4712 to determine at least one attribute 4733 of the at least one earpiece, where in an aspect the at least one attribute 4733 is indicative of usability of the at least one earpiece with one of the left or the right ear of the user. In addition, neural stimulus control signal determination circuitry 4710 is configured (with earpiece location logic module 4720) to determine the ear at which the earpiece is usable, based on the at least one attribute of the at least one earpiece. In an aspect, the at least one attribute of the at least one earpiece includes a shape of the at least one earpiece, a color of the at least one earpiece, a presence of an indicia 4721 on the at least one earpiece or a presence of indicia 4721 on an attachment to the at least one earpiece, where, as noted above, the attachment to the at least one earpiece may be, for example, a cable connected to the at least one earpiece. For example, in a system in which only one of two earpieces includes a stimulating electrode, the cable connected to the earpiece with the electrode may include a pattern of light and dark stripes, while the cable connected to the other earpiece may be a solid color. In embodiments in which two earpieces including ear stimulation devices are used, neural stimulus control signal determination logic can send a neural stimulus control signal 4722 sufficient to activate only an earpiece that is usable with the ear upon which it is located, based on the output of earpiece location logic module 4720.

In an aspect, image processing circuitry 4708 includes emotion determination module 4754, which determines an emotion of the user from user image 4712, based upon one or more parameter 4716, e.g. from facial expression, for example using methods as described in Su, "A simple approach to facial expression recognition," Proceedings of the 2007 Int'l Conf on Computer Engineering and Applications, Queensland, Australia, 2007, pp. 456-461; U.S. Patent No. 9,036,018 to Wang et al.; U.S. Patent 8,488,023 to Bacivarov et al., and U.S. Patent Application Publication 2004/0207720 to Miyahara et al.

In an aspect, image processing circuitry 4708 includes physiological condition module 4756, which determines a physiological condition of the user from user image 4712, based upon one or more parameter 4716. Physiological condition of the user can be inferred from eye movement, pupil dilation, heart rate, respiration rate, facial coloration, facial temperature, etc. A visible or IR image of the patient, obtained with a camera built into a personal computing device or operatively connected to the personal computing device can be used. Still or moving (video) image may be used. For example, video images of the subject may be analyzed to determine blood flow using Eulerian video magnification. Further data analysis may be used to determine blood pressure in the subject. See, e.g., Wu et al., ACM Trans. Graph. 31, 4, Article 65, July 2012; (available online at http://doi.acm.org/10.1145/2185520.2185561). An infrared camera may be used to measure corneal temperature (see e.g., Kessel et al., Investigative Opthalmology and Visual Science 51: 6593-6597, 2010). An infrared camera with a focal plane array detector, thermal sensitivity ≤ 0.09 degrees C and an accuracy of 0.1 degrees C is available from Fluke Corp., Everett, WA (see e.g., Fluke_Ti25 Datasheet).

Peripheral sympathetic responses can be detected through image analysis, as described in IEEE TRANSACTIONS ON BIOMEDICAL ENGINEERING, VOL. 56, NO. 2, FEBRUARY 2009 477, Imaging Facial Signs of Neurophysiological Responses, Dvijesh Shastri, Associate Member, IEEE, Arcangelo Merla, Member, IEEE, Panagiotis Tsiamyrtzis, and Ioannis Pavlidis, Senior Member, IEEE. For example, thermal imaging measurements from several different regions of the face provide indication of blood flow, sweat gland activation, and breathing, providing information similar to galvanic skin response. In various aspects, physiological condition module 4756 is used to determine one or more medical condition, including, for example, muscle spasm, seizure, epilepsy (e.g., seizure , spasm, staring), drowsiness, lethargy, fatigue, pain, fever, hypertension (e.g., sweating, flushing), hypotension, mental state

In an aspect, image processing circuitry 4708 includes identity determination module 4758, which determines an identity of the user from user image 4712, based upon one or more parameter 4716. For example, systems and algorithms to obtain iris images, identify unique signatures and rapidly compare key features of iris images to a large database of iris images are described (see *e.g.,* U.S. Patent No. 5,572,596 issued to Wildes et al. on Nov. 5, 1996 and U.S. Patent No. 4,641,349 issued to Flom et al. on Feb. 3, 1987). An iris scanning system which includes a near-infrared (approximately 700-900 nm) illumination source, a 1.3 megapixel camera and algorithms to analyze and compare iris images is available from Bayometric Inc., San Jose, CA (see *e.g.,* the Specification Sheet: "Crossmatch Retinal Scan 2 Iris Scanner"). In another aspect, facial recognition circuitry is used to determine the presence of the user through facial recognition, e.g., using approaches as described in Wheeler, Frederick W.; Weiss, R.L.; and Tu, Peter H., "Face Recognition at a Distance System for Surveillance Applications," Fourth IEEE International Conference on Biometrics: Theory Applications and Systems (BTAS), 2010 Page(s): 1 - 8 (DOI: 10.1109/BTAS.2010.5634523), and Moi Hoon Yap; Ugail, H.; Zwiggelaar, R.; Rajoub, B.; Doherty, V.; Appleyard, S.; and Hurdy, G., "A Short Review of Methods for Face Detection and Multifractal Analysis, " International Conference on CyberWorlds, 2009. CW '09. , Page(s): 231 - 236 (DOI: 10.1109/CW.2009.47). Biometric identification can also include recognition based on a variety of physiological or behavioral characteristics, such as fingerprints, voice, iris, retina, hand geometry, handwriting, keystroke pattern, etc., e.g., as described in Kataria, A.N.; Adhyaru, D.M.; Sharma, A.K.; and Zaveri, T.H., "A Survey of Automated Biometric Authentication Techniques" Nirma University International Conference on Engineering (NUiCONE), 2013, Page(s): 1 - 6 (DOI: 10.1109/NUiCONE.2013.6780190). U.S. Patent 8,229,178 issued July 24, 2012 to Zhang et al, describes a method for acquiring a palm vein image with visible and infrared light and extracting features from the image for authentication of individual identity. Biometric identification can be based on imaging of the retina or iris, as described in U.S. Patent No. 5,572,596 issued to Wildes et al. on Nov. 5, 1996 and U.S. Patent No. 4,641,349 issued to Flom et al. on Feb. 3, 1987. Combinations of several types of identity signals can also be used (e.g., speech and video, as described in Aleksic, P.S. and Katsaggelos, A.K. "Audio-Visual Biometrics," Proceedings of the IEEE Volume: 94 , Issue: 11, Page(s): 2025 - 2044, 2006 (DOI: 10.1109/JPROC.2006.886017)).

In an aspect, image processing circuitry 4708 includes eye tracking module 4760, which determines an eye position or eye movement from user image 4712, based upon one or more parameter 4716. For example, a gaze tracking system for monitoring eye position is available from Seeing Machines Inc., Tucson, AZ (see *e.g.,* the Specification Sheet: "faceLAB™ 5 Specifications"). Eye position, eye rotation, eye gaze position against screen, pupil diameter and eye vergence distance may be monitored. Eye rotation measurements of up to +/- 45 degrees around the y-axis and +/-22 degrees around the x-axis are possible. Typical static accuracy of gaze direction measurement is 0.5-1 degree rotational error. Eye position can be sensed using a method and system as described in U.S. Patent 8,808,195 to Tseng et al., or by other methods described herein or known to those skilled in the relevant art. Eye position may include static or fixed eye position/gaze direction or dynamic eye position/eye movement. In an aspect, eye tracking module 4760 detects pupil diameter. Pupil diameter can be measured, for example, by methods as described in U.S. Patent 6,162,186 to Scinto et al.

Ear stimulation device control system 4700 may include various other components as described generally elsewhere herein, including, but not limited to, e.g., communication circuitry 4762, data storage circuitry 4764, and reporting circuitry 4766. Similarly, ear stimulation device 4732 may include additional components, including but not limited to communication circuitry 4768 and stimulator driver circuitry 4770. In addition, ear stimulation device 4732 may include or be used in combination with a securing member 4772. In an aspect, the securing member includes or is a portion of an earpiece.

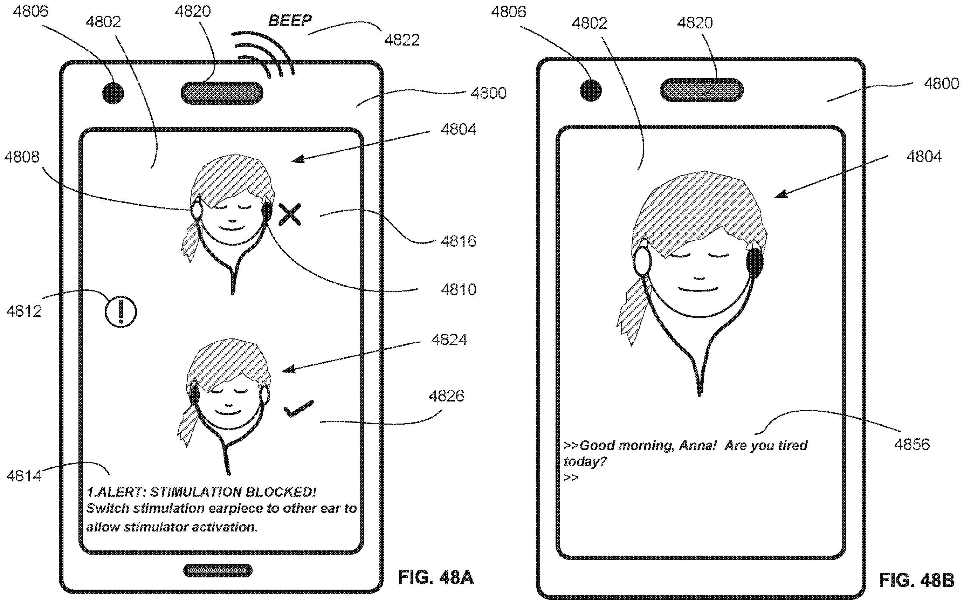

FIGS. 48A and 48B show examples of user interfaces used in connection with an ear stimulation device control system implemented on a personal computing device, and in particular illustrate ways in which processing of a user image captured with a user-facing camera is used in control of the ear stimulation device. In the example of FIG. 48A, the personal computing device is a smart phone 4800 configured with application software that notifies the user of improper placement of the earpieces. Detection and notification is performed, e.g. as described in connection with FIGS. 43 - 47. Delivery of text, visible, and audio notifications to the user (e.g., as in the method of FIG. 46) are illustrated in FIG. 48A. In the systems shown in FIG. 48A and 48B, the ear stimulation device itself is not depicted, but it would be connected to smart phone 4800, e.g. via an audio jack. Touchscreen 4802 of smart phone 4800 functions as a user interface (e.g., user interface 4744 in FIG. 47). User image 4804, captured with user-facing camera 4806 is displayed on touchscreen 4802. Image analysis of user image 4804 is performed by image processing circuitry (e.g., image processing circuitry 4708 in FIG. 47), to determine whether the earpiece including the ear stimulation device is positioned properly. In an aspect, proper positioning of the ear stimulation device means that the earpiece is located on the correct ear, and in some cases also means that the earpiece is located in the proper position on the ear. In the example of FIG. 48A, earpieces 4808 and 4810 in user image 4804 are different colors, allowing the two earpieces to be distinguished. Alert symbol 4812 (an exclamation point in a circle) notifies the user of an alert message, which is delivered via alert text 4814 displayed on touchscreen 4802. In this case, alert text 4814 provides the alert message "***1***. *ALERT: **STIMULATION BLOCKED! Switch stimulation earpiece to other ear to allow*** ***stimulator activation."*** An 'X' 4816 (or other marker) displayed next to user image 4804 indicates to the user that earpieces 4808 and 4810 are positioned incorrectly. An audible notification 4822 (e.g., a 'beep') delivered by speaker 4820 is also provided to attract the user's attention to the incorrectly positioned earpieces. A second user image 4824, serving as an exemplar depicting correctly placed earpieces, is also presented on touchscreen 4802. The colors of earpieces in image 4824 may be enhanced or highlighted in the image to emphasize the importance of placing an earpiece of a particular color at a particular ear. A check mark 4826 (or other marker) is used to indicate that second user image 4824 depicts correct earpiece position. Check mark 4826 may be animated, e.g. to switch from flashing to solid or change color from red to green when the user has switched the earpieces to the proper positions. Once the earpieces are properly positioned, the ear stimulation device control system, implemented with smart phone 4800, controls the ear stimulation device to deliver a stimulus to the ear of the subject, as described elsewhere herein.

As described in connection with FIG. 47, in an aspect ear stimulation device control system 4700 includes emotion determination module 4754, physiological condition module 4756, identity determination module 4758 and eye tracking module 4760. These modules can be used to determine additional information about the user on which to base control of the ear stimulation device. In FIG. 48B, touchscreen 4802 of smart phone 4800 functions as a user interface. User image 4804, captured with user-facing camera 4806 is displayed on touchscreen 4802. Image analysis of user image 4804 is performed by image processing circuitry 4708 as shown in FIG. 47, to determine the identity of the user, and potentially also the emotion and physiological status of the user, as discussed herein above. After the identity of the user has been determined, user-specific information can be used to determine neural stimulation. For example, stimulus level settings that have been optimized for the user can be retrieved from memory and used to configure the ear stimulation device. In addition, the stimulus delivered with the stimulation device may be adjusted depending upon the mood or physiological status of the user.

In addition, in an aspect, application software on smart phone 4800 prompts the user to enter additional information regarding mood or other parameters, similar to application software 2908 described in connection with FIG. 42. In the example of FIG. 48B, the mood of the subject may not be readily determined from the relatively neutral expression of the user in image 4804. However, the application software may prompt the user to enter to enter information regarding mood or other feelings. For example, text prompt 4856 "Good morning, Anna! Are you tired today?" might be followed by additional questions, depending on the user's response, in order to determine how the user is feeling. The use of application software to assess the mood of the user is discussed in greater detail elsewhere herein.

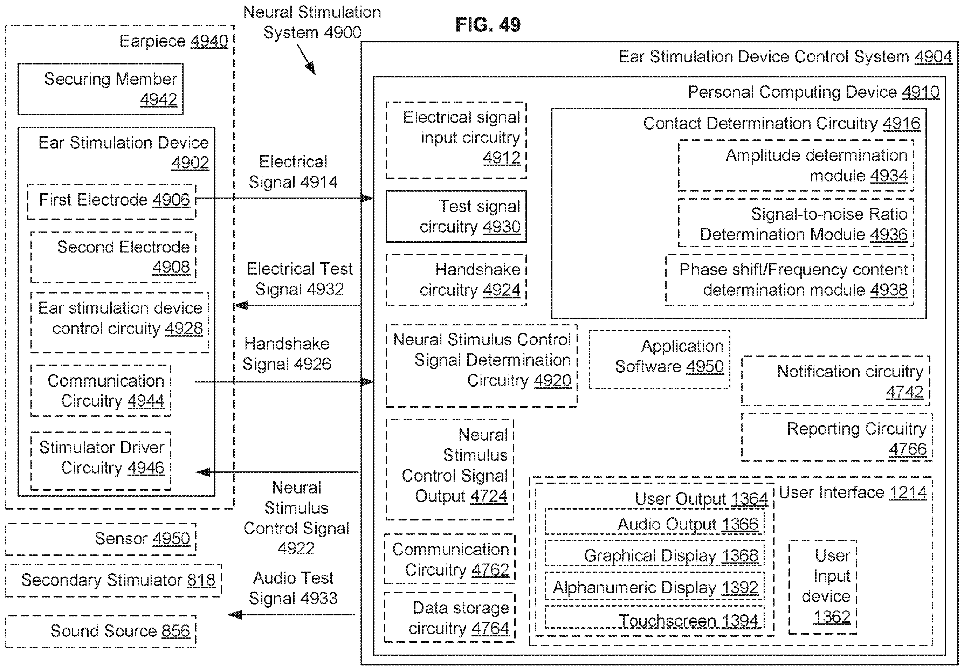

As discussed herein above, in an aspect, image detection and analysis is used to detect improper placement of one or more earpieces on the ear(s) of a user of a personal computing device. In some aspects, it is desirable to detect quality of electrical contact between the ear and an electrode used for delivering electrical stimuli to or sensing electrical signals from the ear. FIG. 49 is a block diagram depicting neural stimulation system 4900, which includes ear stimulation device 4902 and ear stimulation device control system 4904. FIG. 49 depicts further aspects of a neural stimulation system 4900 including an ear stimulation device control system 4904, used for controlling an ear stimulation device 4902 that delivers electrical stimuli via one or more electrodes 4906 and 4908. Ear stimulation device control system 4904 determines whether the one or more electrodes 4906 and 4908 are in good electrical contact with the ear of the user and notifies the user of the status of electrodes 4906 and 4908 so that adjustments can be made, as needed. In addition, delivery of a stimulus via the electrode can be prevented if it is determined that there is not a good electrical contact between the electrode and the ear. Ear stimulation device control system 4904 includes a personal computing device 4910 configured to control delivery, via ear stimulation device 4902, of a stimulus to at least one nerve innervating an ear of a user of personal computing device 4910. Ear stimulation device 4902 includes at least one first electrode 4906. Personal computing device 4910 includes electrical signal input circuitry 4912 adapted to receive an electrical signal 4914 indicative of electrical contact of the at least one first electrode 4906 with the ear of a user of the personal computing device 4910. Personal computing device 4910 includes contact determination circuitry 4916 configured to determine whether the at least one first electrode 4906 is in good electrical contact with the ear of the user, and neural stimulus control signal determination circuitry 4920 configured to send a neural stimulus control signal 4922 from personal computing device 4910 to ear stimulation device 4902 to prevent delivery of the stimulus if the at least one first electrode 4906 is not in good electrical contact with the ear of the user. In addition, personal computing device 4910 includes notification circuitry 4922 configured to deliver a notification to the user relating to the status of the at least one first electrode 4906. Personal computing device 4910 may be, for example, a phone, watch, wearable device, tablet computer, laptop computer, or desktop computer.

In an aspect, ear stimulation device control system 4904 includes handshake circuitry 4924 adapted to receive a handshake signal 4926 from ear stimulation device control circuitry 4928 associated with ear stimulation device 4902. In an aspect, ear stimulation device control system 4904 includes test signal circuitry 4930 configured to deliver an electrical test signal 4932 via at least one second electrode 4908 of the at least one ear stimulation device 4902, and detecting the electrical signal 4914 via the at least one first electrode 4906 responsive to electrical test signal 4932. In an aspect, contact determination circuitry 4916 is configured to determine an electrical impedance between the at least one first electrode 4906 and the at least one second electrode 4908. In an aspect, contact determination circuitry 4916 is configured to determine an amplitude of electrical signal 4914. In an aspect, contact determination circuitry 4916 is configured to determine a signal-to-noise ratio of electrical signal 4914. In an aspect, contact determination circuitry 4916 is configured to determine a phase shift or frequency content of the electrical signal 4914. In various aspects, contact determination circuitry 4916 includes amplitude determination module 4934 for determining the amplitude of electrical signal 4914, signal-to-noise ratio determination module 4936 for determining the signal-to-noise ratio of electrical signal 4914, or phase shift/frequency content determination module 4938 for determining the phase shift or frequency content of electrical signal 4914.

In an aspect, ear stimulation device 4902 includes earpiece 4940 which includes the at least one first electrode 4906. In an aspect, notification circuitry 4922 is configured to instruct the user to reposition earpiece 4940, replace at least a portion of the at least one first electrode 4906, clean at least a portion of the at least one first electrode 4906, moisten at least a portion of the at least one first electrode 4906, or apply gel to at least a portion of the at least one first electrode 4906. As discussed herein above, in various aspects, ear stimulation device 4902 includes or is used in connection with a securing member 4942, and may include additional circuitry components as described elsewhere herein, e.g. communication circuitry 4944 and stimulator driver circuitry 4946. In an aspect, notification circuitry 4922 is configured to deliver one or more of a text notification, a visible notification, or an audio notification. Neural stimulation system 4900 in various aspects includes other components as described elsewhere herein. For example, in various aspects personal computing device 4910 includes user interface 1214 including user input device 1362 and user output 1364, including audio output 1366, graphical display 1368, alphanumeric display 1392, or touchscreen 1394, as depicted in and described in connection with FIG. 13, for example. In various aspects, ear stimulation device control system includes neural stimulus control signal output 4724, communication circuitry 4762, data storage circuitry 4764, as in connection with FIG. 47. In various aspects, neural stimulation system 4900 includes one or more sensor 4950, which may include, for example, a neural signal sensor, other physiological sensor, an environmental sensor, a motion sensor, a location sensor, of various types as described in connection with neural signal sensor 702 or secondary sensor 750 in FIG. 7. In some aspects, neural stimulation system 4900 includes a secondary stimulator 818, for example as described in connection with FIG. 7. In some aspects, neural stimulation system 4900 includes a sound source 856, e.g. as described in connection with FIG. 7. Notification circuitry 4742 and reporting circuitry 4766 are as described in connection with FIG. 47. Personal computing device 4910 is configured with application software 4950, including but not limited to the various modules described specifically herein.

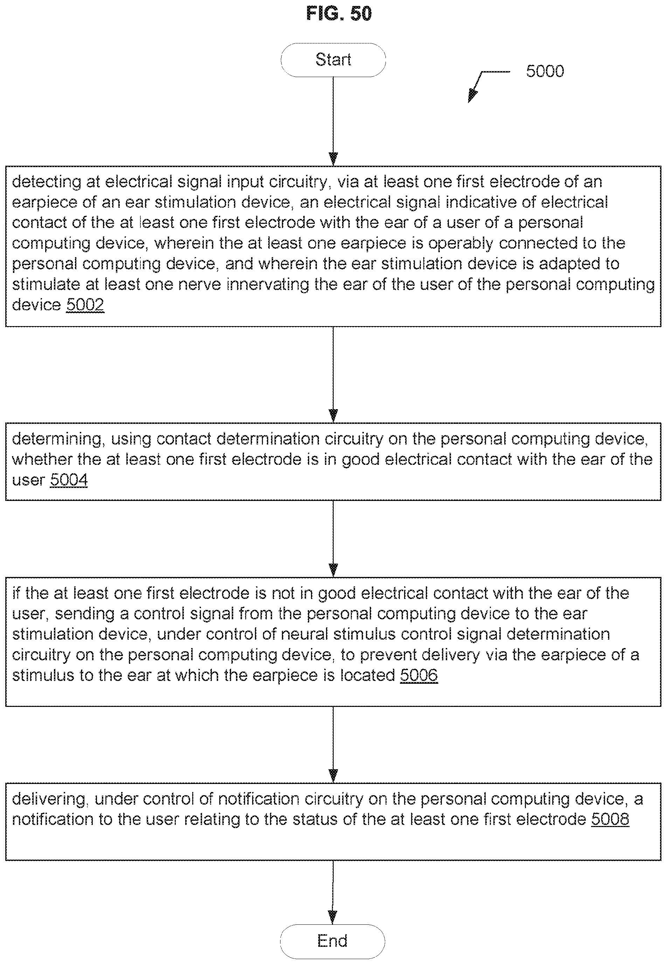

FIG. 50 depicted a method of controlling an ear stimulation device with a personal computing device, responsive to detection of contact between an electrode and the ear of the user, as described in connection with FIG. 49. As noted above, the personal computing device may be, for example, a phone, watch, wearable device, tablet computer, laptop computer, or desktop computer. Method 5000 includes detecting at electrical signal input circuitry, via at least one first electrode of an earpiece of an ear stimulation device, an electrical signal indicative of electrical contact of the at least one first electrode with the ear of a user of a personal computing device, wherein the at least one earpiece is operably connected to the personal computing device, and wherein the ear stimulation device is adapted to stimulate at least one nerve innervating the ear of the user of the personal computing device, as indicated at 5002; determining, using contact determination circuitry on the personal computing device, whether the at least one first electrode is in good electrical contact with the ear of the user, as indicated at 5004; if the at least one first electrode is not in good electrical contact with the ear of the user, sending a control signal from the personal computing device to the ear stimulation device, under control of neural stimulus control signal determination circuitry on the personal computing device, to prevent delivery via the earpiece of a stimulus to the ear at which the earpiece is located, as indicated at 5006; and delivering, under control of notification circuitry on the personal computing device, a notification to the user relating to the status of the at least one first electrode, as indicated at 5008.

Determining whether the at least one first electrode is in good electrical contact with the ear of the user can be performed by various methods, as discussed herein below. Good electrical contact can be defined by setting a threshold value for one or more measured parameter, such that contact is defined to be "good" if the measured parameter(s) are at or above the threshold value and "bad" if they are below the threshold parameter. In an aspect, determining whether the at least one first electrode is in good electrical contact includes providing a rating of the contact quality, e.g., "strong," "moderate," "usable but weak," "unusable," wherein any usable contact qualities are considered "good," but contacts that are not usable are considered "bad."

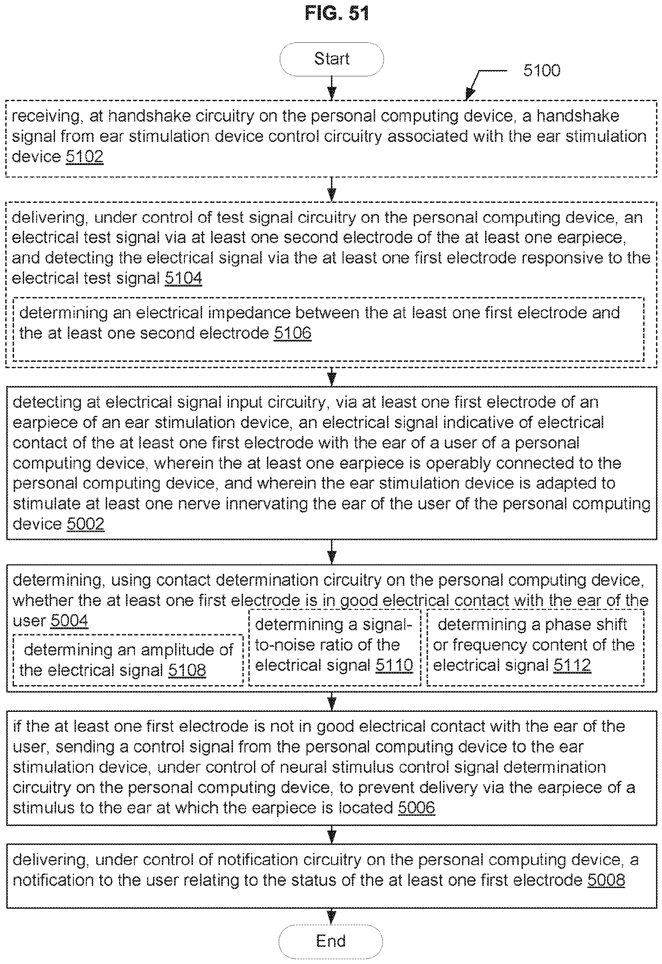

Further aspects and variants of method 5000 are depicted in FIGS. 51-52. In these figures, steps 5002, 5004, 5006, and 5008 are the same as in FIG. 50.

FIG. 51 depicts a method 5100, which includes further elaborations of the method of FIG. 50. Steps 5002, 5004, 5006 and 5008 are as described in connection with FIG. 50. In an aspect, method 5100 includes receiving, at handshake circuitry on the personal computing device, a handshake signal from ear stimulation device control circuitry associated with the ear stimulation device, as indicated at 5102. In another aspect, method 5100 includes delivering, under control of test signal circuitry on the personal computing device, an electrical test signal via at least one second electrode of the at least one earpiece, and detecting the electrical signal via the at least one first electrode responsive to the electrical test signal, as indicated at 5104. This may include, for example, determining an electrical impedance between the at least one first electrode and the at least one second electrode, as indicated at 5106. In various aspects, determining whether the at least one first electrode is in good electrical contact with the ear of the user (at 5004) includes determining an amplitude of the electrical signal, as indicated at 5108, determining a signal-to-noise ratio of the electrical signal, as indicated at 5110, or determining a phase shift or frequency content of the electrical signal, as indicated at 5112.

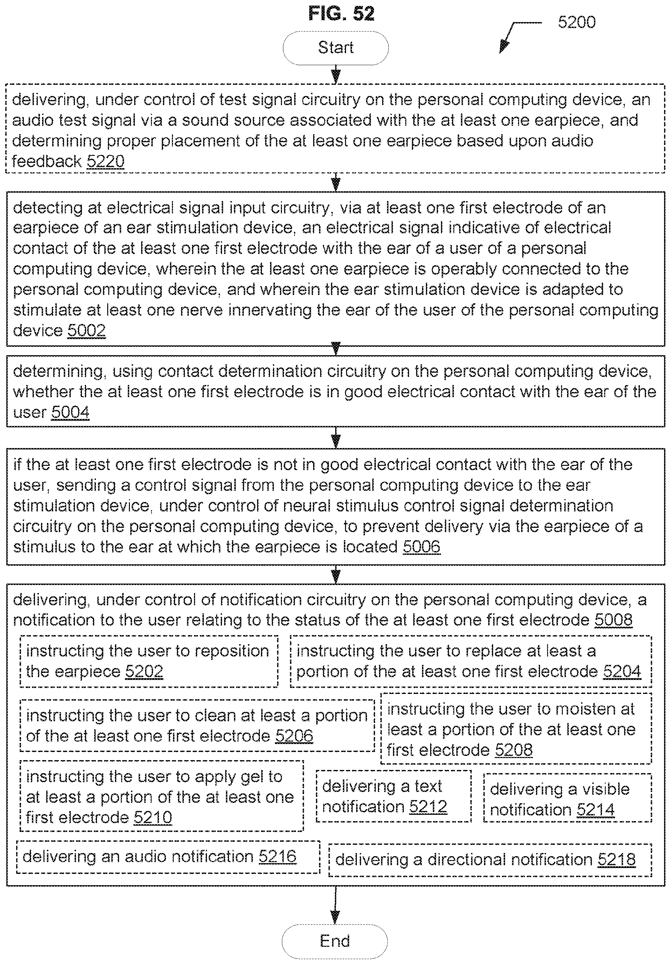

FIG. 52 depicts a method 5200, including further variants of the method shown in FIG. 50, relating to delivering a notification to the user regarding the status of the at least one first electrode. In various aspects, delivering the notification to the user includes instructing the user to reposition the earpiece, as indicated at 5202, instructing the user to replace at least a portion of the at least one first electrode, as indicated at 5204, instructing the user to clean at least a portion of the at least one first electrode, as indicated at 5206, instructing the user to moisten at least a portion of the at least one first electrode, as indicated at 5208, or instructing the user to apply gel to at least a portion of the at least one first electrode, as indicated at 5210. In other aspects, delivering the notification to the user includes delivering a text notification, as indicated at 5212, delivering a visible notification, as indicated at 5214, delivering an audio notification, as indicated at 5216, or delivering a directional notification, at 5218. Notifications can be delivered, for example, as described in connection with FIG. 44, or as illustrated in FIG. 48A. In another aspect, method 5200 includes delivering, under control of test signal circuitry on the personal computing device, an audio test signal via a sound source associated with the at least one earpiece, and determining proper placement of the at least one earpiece based upon audio feedback, as indicated at 5220. In an aspect, audio feedback is determined from an audio signal detected from the earpiece, which will vary depending upon the placement of the earpiece, e.g. whether or not it is firmly seated within the ear canal. In an aspect, audio feedback is determined from the user, e.g. the user self-reporting of audio quality.

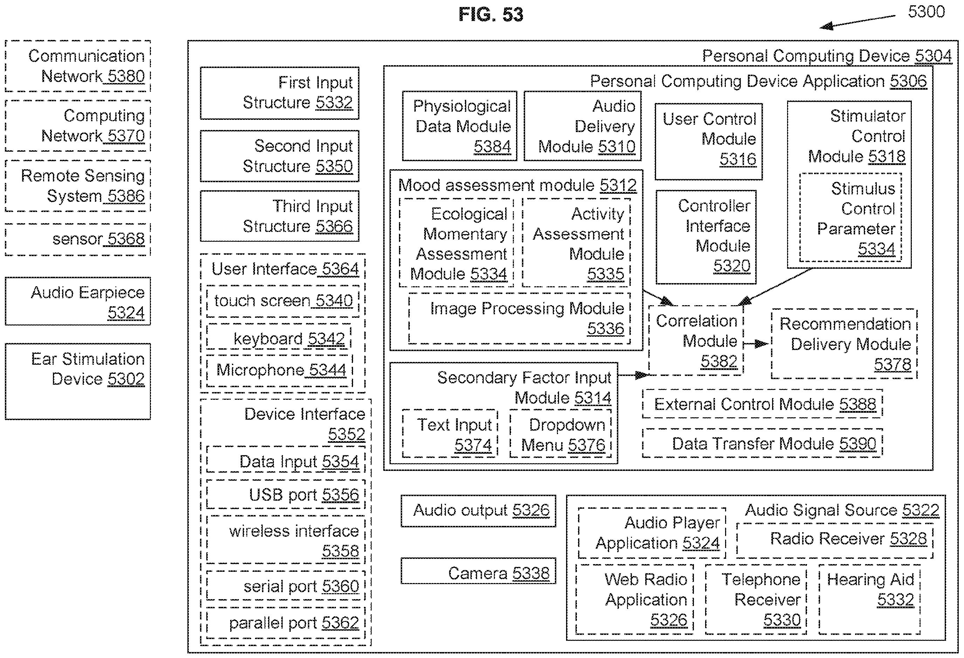

FIG. 53 illustrates a nerve stimulation system 5300 including ear stimulation device 5302 and personal computing device 5304, which is configured personal computing device application 5306 for monitoring use of nerve stimulation system 5300 by a user. As in other examples presented herein, personal computing device 5304 may be, for example a phone, watch, wearable device, tablet computer, laptop computer, or desktop computer. Aspects of nerve stimulation system 5300 not described in detail in connection with FIG. 53 are generally as described in connection with other embodiment depicted and described herein.

Personal computing device application 5306 includes audio delivery module 5310, mood assessment module 5312, secondary factor input module 5314, user control module 5316, stimulator control module 5318, and controller interface module 5320. The various modules include application software operating in connection with personal computing device hardware and software (i.e. the personal computing device hardware is configured by the application software) to provide the module functionality. Personal computing device 5306 includes other hardware and software components as described elsewhere herein as well as conventional hardware and software components not specifically described herein. The term "module," as used herein, refers to application software operating on and used to configure personal computing device hardware to provide specialized circuitry functions of the device. In general, a module utilizes and in an aspect can be considered to incorporate both data storage circuitry and processing circuitry of the personal computing device.

Audio delivery module 5310 is adapted to control delivery of an audio signal from an audio signal source 5322 to an audio earpiece 5324 via an audio output 5326 of the personal computing device 5304, the audio earpiece 5324 having associated therewith ear stimulation device 5302 configured to stimulate a nerve innervating the ear of the user. Audio signal source 5322 may be, for example an audio player application 5324, a web radio application 5326, a radio receiver 5328, a telephone receiver 5330, or a hearing aid 5331.

Mood assessment module 5312 is adapted to receive mood-related input from the user via a first input structure 5332 associated with the personal computing device, and assess a mood of the user based at least in part upon the mood-related input. In an aspect, mood assessment module 5312 includes an ecological momentary assessment module 5334. Ecological momentary assessment module 5334 includes application software on the person computing device that collects information about the user's behaviors and experiences from the user, by querying the user at intervals during the day as they go about their usual activities in their "natural environment" (as contrasted to self-reports of mood based on the user's recollections during a clinic visit, for example), e.g. as described generally in Shiffman et al., "Ecological Momentary Assessment," Annual Review of Clinical Psychology, Vol. 4:1-32, April 2008 (First Published Online November 28, 2007), DOI: 10.1146/annurev.clinpsy.3.022806.091415. In some aspects, mood assessment module 5312 includes activity assessment module 5335, which tracks and analyzes user activities involving use of the personal computing device (e.g. use of social media, web searches, speech patterns, typing patterns, including amount and/or type of use) to determine mood of the user. For example, in an aspect, activity assessment module 5335 analyzes typing patterns using, for example, techniques as described in U.S. Patent 6,231,344 to Merzenich et al., U.S. Published Patent Application 2005/0084832 to Janssen et al. In an aspect, activity assessment module 5335 determines the timing of entry of instructions by the patient. In an aspect, it is not necessary to determine the specific instructions entered by the patient, but only to determine how often the patient is using the personal computing device, and/or how quickly the patient is entering instructions into the personal computing device. In other aspects, the specific instructions can be detected, e.g., to determine whether the patient is choosing to listen to music, play a game, send or read email, receive a phone call, or place a phone call. Sensing and processing of game controller signals, e.g., to determine reaction times, may be substantially as described in U.S. Patent 5,913,310 to Brown, or U.S. Patent 6,186,145 to Brown. It will be appreciated that while Brown describes a video game designed primarily for health care-related teaching purposes, the video game may be for entertainment purposes, and need not include an educational or medical component. In an aspect, activity assessment module 5335 is configured to process an audio signal (detected from a cell phone, for example) to determine a speech pattern of the patient. In an aspect, mood assessment module 5312 includes image processing module 5336 adapted to determine a mood of the user based on image analysis of an image of the user detected with a user-facing camera 5338 of the personal computing device 5304, e.g., as discussed herein above. In various aspects, mood assessment module 5312 is adapted to receive mood-related input relating to depression, stress, or emotion, for example. First input structure 5332 of the personal computing device in various aspects includes a touch screen 5340, a keyboard, or a microphone.

Secondary factor input module 5314 is adapted to receive at least one input relating to at least one secondary factor relating to the user via a second input structure 5350 associated with personal computing device 5304. Secondary input structure 5350 of personal computing device 5304 includes at least one of a touch screen 5340, a keyboard 5342, a microphone 5344, a device interface 5352, a data input 5354, USB port 5356, a wireless interface 5358, a serial port 5360, and a parallel port 5362. Touch screen 5340, a keyboard 5342, a microphone 5344, are examples of components of a user interface 5364, although it will be appreciated that input may be received by other types of user interface devices, as are known to those of skill in the art. Data input 5354, USB port 5356, wireless interface 5358, serial port 5360, and parallel port 5362 are examples of device interface 5352; other device interfaces may be used as well. First input structure 5332, second input structure 5350, and third input structure 5366 may in some aspects be the same type of input structure, and indeed may be the same input structure. In other aspects, one or more of first input structure 5332, second input structure 5350, and third input structure 5366 are the same type of input structure, but are distinct input structures. In some aspects, first input structure 5332, second input structure 5350, and third input structure 5366, are different types of input structures, and, in addition, are distinct input structures.

In an aspect, secondary factor input module 5314 is adapted to receive at least one input from the user, i.e., via user interface 5364. In another aspect, secondary factor input module is adapted to receive at least one input from a sensor 5368, e.g., via data input 5354 or another device interface. In another aspect, secondary factor input module 5314 is adapted to receive at least one input via a computing network 5370.

User control module 5316 is adapted to receive at least one user control input via a third input structure 5366 of the personal computing device, the user control input for controlling user-controllable stimulation parameters of the ear stimulation device.

Stimulator control module 5318 is adapted to determine at least one stimulus control parameter based on at least one of the mood of the user, the at least one secondary factor, and the at least one user control input.

Controller interface module 5320 is used for communicating the at least one stimulus control parameter to a stimulator controller adapted to control the ear stimulation device responsive to the at least one stimulus control parameter.

In an aspect, stimulator control module 5318 is configured to coordinate delivery of the audio signal with delivery of at least one stimulus with ear stimulation device 5302.

In an aspect, secondary factor input module 5314 is adapted to receive at least one input relating to an environmental condition of the user, for example, including at least one of a light level, a temperature, a humidity, a pollen count, a noise level, a day length, a precipitation, an air quality measure. In another aspect, secondary factor input module 5314 is adapted to receive at least one input relating to sleep pattern of the user. In another aspect, secondary factor input module 5314 is adapted to receive at least one input relating to medical history of the user. In another aspect, secondary factor input module 5314 is adapted to receive at least one input relating to an activity of the user, including, but not limited to a physical activity, a health-related activity, a recreational activity, a social activity, an employment activity, a purchasing activity, a mental activity, a spiritual activity, a media-related activity, an activity of daily life, an amount of activity, a duration of activity, a frequency of activity, a timing of activity, a calendar, a schedule, or a cost. In another aspect, secondary factor input module 5314 is adapted to receive at least one input relating to a diet of the user or an appetite of the user. An input relating to a secondary factor may include user input relating to the secondary factor, received for example, via user interface 5364. In an aspect, secondary factor input module 5314 is adapted to receive at least one open ended comment from the user, e.g. via text input 5374. In another aspect, secondary factor input module 5314 is adapted to provide a drop down menu 5376 of selectable items and receive from the user a selection from the drop down menu. For example, in an aspect, the menu of selectable items includes topic areas for discussion with a medical care provider. In addition to text inputs or menus, various types of input elements may be utilized to receive input from the user, voice to text conversion, screen elements with clickable buttons or checkboxes that allow the user to select from multiple options, sliders that allow the user to increase or decrease a parameter value between minimum and maximum values, and various other types of input elements.

In an aspect, personal computing device application 5306 includes a recommendation delivery module 5378, which is configured to present a recommendation to the user. For example, the recommendation can be presented to the user via user interface 5364, or audio output 5326, or sent to a remote device via device interface 5352, computing network 5370, or communication network 5380. In an aspect, recommendation delivery module 5378 is adapted to receive the recommendation from a medical care provider, from an insurance company, a service provider, an advisor, a computation-based system, or a social media source. For example, in an aspect recommendation delivery module 5378 is adapted to receive a recommendation via computing network 5370. For example, in an aspect the recommendation based on patients similar to the user. In an aspect, recommendation delivery module 5378 is adapted to generate the recommendation.

In an aspect, personal computing device application 5306 includes a correlation module 5382 configured to determine at least one correlation between the mood of the user and at least one of the at least one secondary factor and the at least one stimulus control parameter, and recommendation delivery module is configured to generate the recommendation based at least in part on the at least one correlation. For example, if it is determined that a particular secondary factor (e.g., rainy weather) is normally followed by a depressed mood in the user, if occurrence of the secondary factor is detected, a recommendation is generated for increasing stimulation. As another example, if a particular activity of the user is correlated with depression (for example, if the user reports being depressed after staying up late and not getting enough sleep, the recommendation might be to go to bed earlier).

In an aspect, recommendation delivery module 5378 is adapted to provide the recommendation to a medical care provider of the user. This can be done, for example, by sending the recommendation to a remote device used by the medical care provider, via computing network 5370, communication network 5380, device interface 5352, or via user interface 5364. Providing the recommendation to the medical care provider makes it possible for the medical care provider to discuss the recommendation with the user, or not, as deemed appropriate by the medical care provider, as well as to incorporate the recommendation into an overall treatment plan for the user.

In an aspect, recommendation delivery module 5378 is configured to generate the recommendation based on at least one of information regarding a response of the subject to a past treatment regimen, information obtained via social media, information regarding at least one preference of at least one social media contact of the subject, information regarding at least one preference of at least one peer of the subject, information regarding at least one preference of at least one role model of the subject, information from an insurance company, information from a service provider.

In an aspect, recommendation delivery module 5378 is configured to generate the recommendation with a computation-based system; for example, an artificial intelligence, a neural network, or a machine learning system. In an aspect, recommendation delivery module 5378 is configured to generate the recommendation based on a predicted response of the subject to a treatment regimen.

In another aspect, recommendation delivery module 5378 is configured to receive information regarding whether the subject has accepted or rejected the recommendation. In various aspects, recommendation delivery module 5378 is configured to present to the user a recommendation for a configuration of the neural stimulus, or a recommendation for a secondary stimulus to be delivered in association with the neural stimulus.

In an aspect, personal computing device application 5306 includes physiological data module 5384 adapted to receive at least one physiological data signal representing at least one physiological parameter of the user. In an aspect, physiological data module 5384 is adapted to receive the at least one physiological data signal from at least one sensor 5368. In another aspect, physiological data module 5384 is adapted to receive the at least one physiological data signal from at least one computing network 5370, or alternatively, via at least one communication network 5380. In another aspect, physiological data module 5384 is adapted to receive the at least one physiological data signal from at least one remote sensing system 5386. A remote sensing system may include one or more sensors in the environment of the user, including but not limited to cameras, motion sensors, pressure sensors, force sensors, infrared sensors, etc. In various aspects, physiological data module 5384 is adapted to receive the at least one physiological data signal from at least one of a blood pressure sensor, a heart rate sensor, a chemical sensor, a biosensor, a pH sensor, a blood oxygen sensor, a galvanic skin response sensor, an EEG sensor, an EMG sensor, an ECG sensor, a wearable item, an eye tracking system, an acoustic sensor, a motion sensor, a force transducer, or an activity sensor.

In an aspect, user control module 5316 is adapted to receive user input (e.g., via user interface 5364) for controlling at least one of stimulus pulse amplitude, stimulus pulse duration, stimulus frequency, stimulus pulse pattern, and stimulus pulse envelope.

In an aspect, personal computing device application 5306 includes external control module 5388, which is configured to receive an external control input for controlling at least one externally controllable stimulation parameter of the ear stimulation device. For example, in various aspects, external control module 5388 is configured to receive the external control input via computing network 5370 or communication network 5380. In an aspect, external control module 5388 is configured to receive the external control input from an external party or entity. In an aspect, the external party or entity is a medical care provider. In other aspects, the external control input may be received from other external parties or entities, e.g., a family member, an insurance company, the device manufacturer, etc. The at least one externally controllable stimulation parameter includes, for example, at least one of stimulus pulse amplitude, stimulus pulse duration, stimulus frequency, stimulus pulse pattern, and stimulus pulse envelope. Externally controllable stimulation parameters may include preferred values for efficacy of treatment, including preferred values to be used in connection with different patent conditions, or upper and lower limits for stimulus values, for purposes of patient safety or efficacy of treatment.

In another aspect, personal computing device application 5306 includes data transfer module 5390 for providing data relating to the user to an external party or entity. In an aspect, data transfer module 5390 is configured to provide the data to the external party or entity via computing network 5370. In another aspect, data transfer module is configured to provide the data to the external party or entity via communication network 5380. For example, the external party or entity may be a medical care provider, a family member, an insurance company, a service provider, a social media contact (or 'friend') of the subject, a peer of the subject, an advisor, a computation based system, a social media source, a device manufacturer, a merchant, an electronic medical record, a sensor network, or an additional program or application, for example. In an aspect, a sensor network includes or is part of a health diary platform, a smart home, or an internet of things.

In an aspect, stimulator control module 5318 is configured to determine the at least one stimulus control parameter 5334 by overriding the at least one user control input for controlling the at least one user-controllable stimulation parameter based on a medical-care provider control input. Alternatively, in another aspect, stimulator control module 5318 is configured to override a medical-care provider control input based on the at least one user control input for controlling the at least one user-controllable stimulation parameter. In yet another aspect, stimulator control module 5318 is configured to override the at least one user control input for controlling the at least one user-controllable stimulation parameter based on a computing system-generated stimulus control parameter. In another aspect, stimulator control module 5318 is configured to override a computing system-generated stimulus control parameter based on the at least one user control input for controlling the at least one user-controllable stimulation parameter. For example, stimulator control module 5318 is configured to determine the at least one stimulus control parameter to provide an initial setting of the ear stimulation device based on the at least one user-controllable stimulation parameter. In another aspect, stimulator control module 5318 is configured to determine the at least one stimulus control parameter to update a setting of the ear stimulation device based on the at least one user-controllable stimulation parameter.

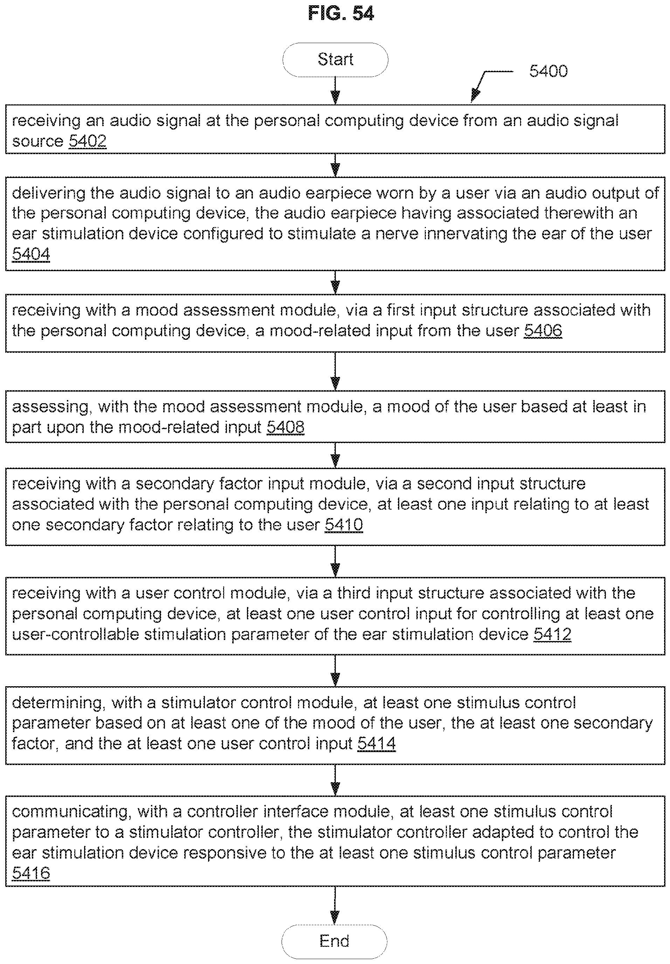

FIG. 54 is a flow diagram of a method of controlling an ear stimulation device with a personal computing device. Method 5400 may be performed, for example, using a personal computing device configured with application software, as depicted and discussed in connection with FIG. 53. Method 5400 includes receiving an audio signal at the personal computing device from an audio signal source, as indicated at 5402; delivering the audio signal to an audio earpiece worn by a user via an audio output of the personal computing device, the audio earpiece having associated therewith an ear stimulation device configured to stimulate a nerve innervating the ear of the user, as indicated at 5404; receiving with a mood assessment module, via a first input structure associated with the personal computing device, a mood-related input from the user, as indicated at 4106; assessing, with the mood assessment module, a mood of the user based at least in part upon the mood-related input, as indicated at 4108; receiving with a secondary factor input module, via a second input structure associated with the personal computing device, at least one input relating to at least one secondary factor relating to the user, as indicated at 4110; receiving with a user control module, via a third input structure associated with the personal computing device, at least one user control input for controlling at least one user-controllable stimulation parameter of the ear stimulation device, as indicated at 4112; determining, with a stimulator control module, at least one stimulus control parameter based on at least one of the mood of the user, the at least one secondary factor, and the at least one user control input, as indicated at 4114; and communicating, with a controller interface module, at least one stimulus control parameter to a stimulator controller, the stimulator controller adapted to control the ear stimulation device responsive to the at least one stimulus control parameter, as indicated at 4116.

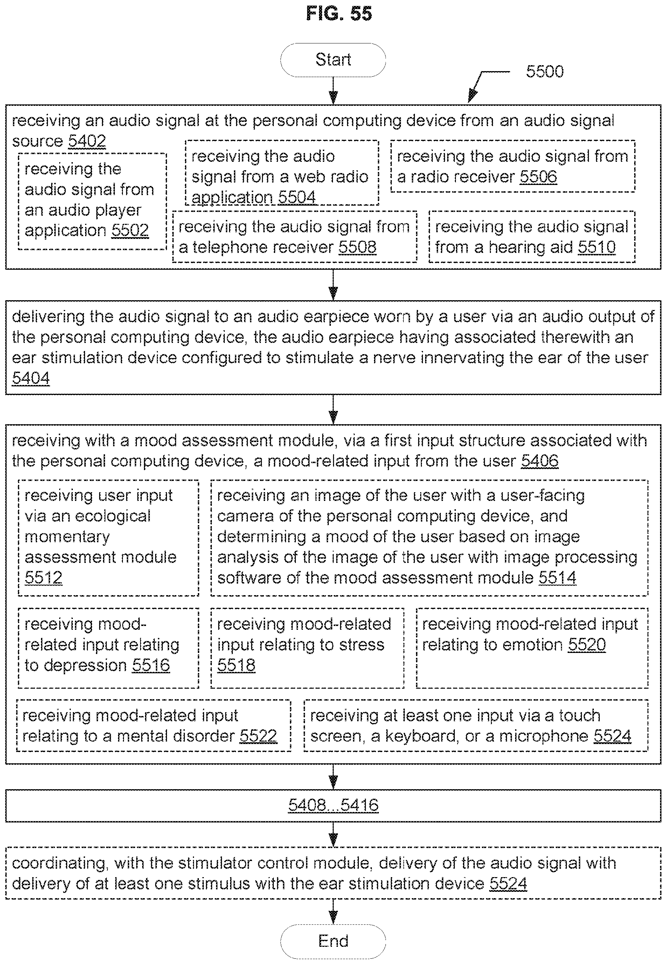

Further aspects of the method shown in FIG. 54 are shown in FIGS. 55-60.

For example, as shown in FIG. 55, in various aspects of a method 5500, receiving the audio signal at the personal computing device from the audio signal source includes receiving the audio signal from an audio player application, as indicated at 5502; receiving the audio signal from a web radio application, as indicated at 5504; receiving the audio signal from a radio receiver, as indicated at 5506, receiving the audio signal from a telephone receiver, as indicated at 5508, or receiving the audio signal from a hearing aid, as indicated at 5510.

Receiving the mood-related input in various aspects includes receiving user input via an ecological momentary assessment module, as indicated at 5512; receiving an image of the user with a user-facing camera of the personal computing device, and determining a mood of the user based on image analysis of the image of the user with image processing software of the mood assessment module, as indicated at 5514. Mood assessment based on image analysis is discussed in greater detail herein above. Receiving the mood-related input may include receiving mood-related input relating to one or more of depression, as indicated at 5516; stress, as indicated at 5518; emotion, as indicated at 5520; or a mental disorder, as indicated at 5522. In an aspect, receiving the mood-related input via the first input structure includes receiving at least one input via a touch screen, a keyboard, or a microphone, as indicated at 5524. In an aspect, method 5500 includes coordinating, with the stimulator control module, delivery of the audio signal with delivery of at least one stimulus with the ear stimulation device, as indicated at 5524.

FIG. 56 provides further variants of the method of FIG. 41, relating to receiving at least one input relating to at least one secondary factor relating to the user, at 4110. In an aspect of method 5600, receiving the at least one input relating to the at least one secondary factor includes receiving at least one input via at least one of a touch screen, a keyboard, a microphone, a device interface, a data input, a USB port, a wireless interface, a serial port, and a parallel port, as indicated at 5602. In further aspects, receiving at least one input relating to at least one secondary factor relating to the user with the secondary factor input module includes receiving at least one input from the user, as indicated at 5604; receiving at least one input from a sensor, as indicated at 5606; or receiving at least one input via a computing network, as indicated at 5608.

In another aspect, receiving at least one input relating to at least one secondary factor relating to the user with the secondary factor input module includes receiving at least one input relating to an environmental condition of the user, as indicated at 5610. An environmental condition may include, for example, at least one of a light level, a temperature, a humidity, a pollen count, a noise level, a day length, a precipitation, an air quality measure, as indicated at 5612.

In other aspects, receiving at least one input relating to at least one secondary factor relating to the user with the secondary factor input module includes receiving at least one input relating to a sleep pattern of the user, as indicated at 5614; a medical history of the user, as indicated at 5615; a diet of the user, as indicated at 5616; an appetite of the user, as indicated at 5618; or an activity of the user, as indicated at 5620. For example, receiving at least one input relating to an activity includes receiving at least one input relating to, e.g., a physical activity, a recreational activity, a social activity, an employment activity, a purchasing activity, a mental activity, a spiritual activity, a media-related activity, an activity of daily life, an amount of activity, a duration of activity, a frequency of activity, a timing of activity, a calendar, a schedule, or a cost, as indicated at 5622.

In further aspect, receiving at least one input relating to at least one secondary factor relating to the user with the secondary factor input module includes receiving at least one open ended comment from the user, as indicated at 5624. In other aspects, receiving at least one input relating to at least one secondary factor relating to the user with the secondary factor input module includes providing a drop down menu of selectable items and receiving from the user a selection from the drop down menu, as indicated at 5626. For example, in an aspect, providing a drop down menu of selectable items includes providing a drop down menu of selectable topic areas for discussion with medical care provider, as indicated at 5628.

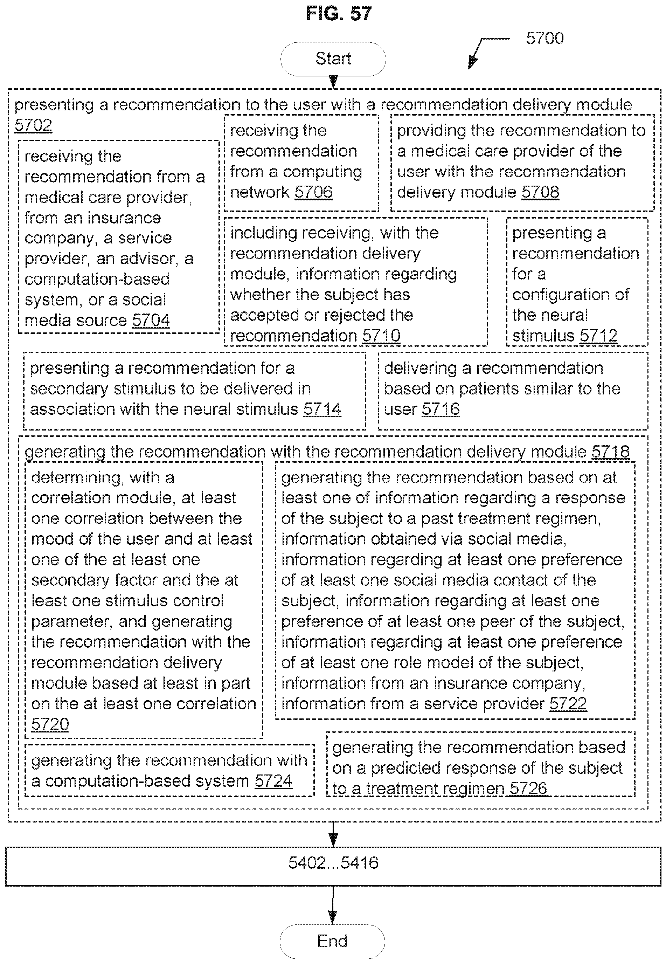

As shown in FIG. 57, in an aspect a method 5700 includes presenting a recommendation to the user with a recommendation delivery module, as indicated at 5702. The recommendation may be received from a medical care provider, from an insurance company, a service provider, an advisor, a computation-based system, or a social media source, as indicated at 5704, or from a computing network, as indicated at 5706. In another aspect, method 5700 includes providing the recommendation to a medical care provider of the user with the recommendation delivery module, as indicated at 5708. In a further aspect, method 5700 includes receiving, with the recommendation delivery module, information regarding whether the subject has accepted or rejected the recommendation, as indicated at 5710.

In various aspects, presenting the recommendation includes presenting a recommendation for a configuration of the neural stimulus, as indicated at 5712, or presenting a recommendation for a secondary stimulus to be delivered in association with the neural stimulus, as indicated at 5714.

In an aspect, method 5700 includes delivering a recommendation based on patients similar to the user, as indicated at 5716.

In an aspect, method 5700 includes generating the recommendation with the recommendation delivery module, as indicated at 5718. For example, the method may include determining, with a correlation module, at least one correlation between the mood of the user and at least one of the at least one secondary factor and the at least one stimulus control parameter, and generating the recommendation with the recommendation delivery module based at least in part on the at least one correlation, as indicated at 5720. In an aspect, the method includes generating the recommendation based on at least one of information regarding a response of the subject to a past treatment regimen, information obtained via social media, information regarding at least one preference of at least one social media contact of the subject, information regarding at least one preference of at least one peer of the subject, information regarding at least one preference of at least one role model of the subject, information from an insurance company, information from a service provider, as indicated at 5722. In some aspects, the method includes generating the recommendation with a computation-based system, as indicated at 5724, or generating the recommendation based on a predicted response of the subject to a treatment regimen, as indicated at 5726.

FIG. 58 depicts aspects of a related method 5800. In an aspect, method 5800 includes receiving, with a physiological data module, at least one physiological data signal representing at least one physiological parameter of the user, as indicated at 5802. Receiving the at least one physiological data signal includes, for example, receiving the at least one physiological data signal from at least one sensor, as indicated at 5804. In an aspect, the at least one sensor is located on the audio earpiece associated with the ear stimulation device, as indicated at 5806. In an aspect, the audio earpiece having the ear stimulation device associated therewith is a first audio earpiece worn on a first ear of the subject, and wherein the at least one sensor is located on a second audio earpiece located on a second ear of the subject, as indicated at 5808.

In an aspect, receiving the at least one physiological data signal includes receiving the at least one physiological data signal from at least one computing network, as indicated at 5810, or from at least one remote sensing system, as indicated at 5812. In various aspects, receiving the at least one physiological data signal includes receiving the at least one physiological data signal from at least one of a blood pressure sensor, a heart rate sensor, a chemical sensor, a biosensor, a pH sensor, a blood oxygen sensor, a galvanic skin response sensor, an EEG sensor, an EMG sensor, an ECG sensor, a wearable item, an eye tracking system, an acoustic sensor, a motion sensor, a force transducer, or an activity sensor, as indicated at 5814.

In a further aspect, method 5800 includes providing data relating to the user to an external party or entity, as indicated at 5816. In various aspects, providing the data to the external party or entity includes providing the data via a computing network, as indicated at 5818, or providing the data via a communication network, as indicated at 5820. In an aspect, providing the data to the external party or entity includes providing the data to a medical care provider, as indicated at 5822. In various aspects, providing the data to the external party or entity includes providing the data to a family member, an insurance company, a service provider, a social media contact of the subject, a peer of the subject, an advisor, a computation based system, a social media source, a device manufacturer, a merchant, an electronic medical record, a sensor network, a program or an application, as indicated at 5824.

FIG. 59 depicts further aspects of a method 5900. In one aspect of method 5900, receiving the at least one user control input for controlling the at least one user-controllable stimulation parameter includes receiving at least one user input for controlling at least one of stimulus pulse amplitude, stimulus pulse duration, stimulus frequency, stimulus pulse pattern, and stimulus pulse envelope, as indicated at 5902.

In another aspect, method 5900 includes determining, with a correlation module, at least one correlation between the mood of the user and at least one of the at least one secondary factor and the at least one stimulus control parameter, as indicated at 5904.

In another aspect, method 5900 includes receiving, with an external control module, an external control input for controlling at least one externally controllable stimulation parameter of the ear stimulation device, as indicated at 5906. In various aspects, this includes receiving the external control input via a computing network or a communication network, as indicated at 5908. In an aspect, receiving the external control input includes receiving the external control input from an external party or entity, as indicated at 5910, for example, a medical care provider, as indicated at 5912, or a family member, an insurance company, a service provider, a social media contact of the subject, a peer of the subject, an advisor, a computation based system, a social media source, a device manufacturer, a merchant, an electronic medical record, a sensor network, a program or an application, as indicated at 5914. In various aspect, the at least one externally controllable stimulation parameter includes at least one of stimulus pulse amplitude, stimulus pulse duration, stimulus frequency, stimulus pulse pattern, and stimulus pulse envelope, as indicated at 5916.

FIG. 60 depicts further aspects of a method 6000, relating to determining the at least one stimulus control parameter. In an aspect, determining the at least one stimulus control parameter includes overriding the at least one user control input for controlling the at least one user-controllable stimulation parameter based on a medical-care provider control input, as indicated at 6002. In another aspect, determining the at least one stimulus control parameter includes overriding a medical-care provider control input based on the at least one user control input for controlling the at least one user-controllable stimulation parameter, as indicated at 6004. In yet another aspect, determining the at least one stimulus control parameter includes overriding the at least one user control input for controlling the at least one user-controllable stimulation parameter based on a computing system-generated stimulus control parameter, as indicated at 6006. In still another aspect, determining the at least one stimulus control parameter includes overriding a computing system-generated stimulus control parameter based on the at least one user control input for controlling the at least one user-controllable stimulation parameter, as indicated at 6008.

In an aspect, determining the at least one stimulus control parameter includes determining an initial setting of the ear stimulation device based on the at least one user-controllable stimulation parameter, as indicated at 6010. In another aspect, determining the at least one stimulus control parameter includes updating a setting of the ear stimulation device based on the at least one user-controllable stimulation parameter, as indicated at 6012.

The herein described subject matter sometimes illustrates different components contained within, or connected with, different other components. It is to be understood that such depicted architectures are merely exemplary, and that in fact many other architectures may be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected", or "operably coupled," to each other to achieve the desired functionality, and any two components capable of being so associated can also be viewed as being "operably couplable," to each other to achieve the desired functionality. Specific examples of operably couplable include but are not limited to physically mateable and/or physically interacting components, and/or wirelessly interactable, and/or wirelessly interacting components, and/or logically interacting, and/or logically interactable components.

In some instances, one or more components may be referred to herein as "configured to," "configured by," "configurable to," "operable/operative to," "adapted/adaptable," "able to," "conformable/conformed to," etc. Those skilled in the art will recognize that such terms (e.g., "configured to") generally encompass active-state components and/or inactive-state components and/or standby-state components, unless context requires otherwise.

A personal computing device, as described herein, may include circuitry and other hardware components, provided, for example in the form of a custom board installed in the case of the personal computing device during or after manufacture, or in a separate package that may be operably connected to the personal computing device via one or more wired and/or wireless connection. Unless context dictates otherwise, as used herein, the term personal computing device is intended to encompass systems including circuitry and other hardware components packaged with the personal computing device and circuitry and other hardware components packaged separately but used in combination with the personal computing device.

While particular aspects of the present subject matter described herein have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true scope of the subject matter described herein. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation *is* explicitly recited, those skilled in the art will recognize that such recitation should typically be interpreted to mean *at least* the recited number (e.g., the bare recitation of "two recitations," without other modifiers, typically means *at least* two recitations, or *two or more* recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flows are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

## Claims

1. A nerve stimulation earpiece (3000) comprising:
an ear canal insert (3005) adapted to fit into an ear canal of a human subject, the ear canal insert including at least one first electrode (3030) configured to electrically contact skin within the ear canal of the subject;
a concha insert (3010) adapted to fit within a concha of the subject, the concha insert including
a base portion (3015) configured to fit within a cavum of the concha of the subject;
a wing portion (3020) configured to fit within a cymba of the concha of the subject; and
at least one second electrode (3035) configured to electrically contact at least a portion of the concha of the subject;
at least one first electrical connector for connecting the at least one first electrode on the ear canal insert to a first electrical current source; and
at least one second electrical connector for connecting the at least one second electrode on the concha insert to a second electrical current source.

2. The nerve stimulation earpiece of claim 1, wherein the ear canal insert includes a sound delivery portion adapted to deliver sound to the ear canal of the subject.

3. The nerve stimulation earpiece of claim 1 or claim 2, further including wireless communication electrical circuitry adapted to transmit a data signal, receive an audio signal, or receive an audio signal.

4. The nerve stimulation earpiece of claim 3, wherein the wireless communication electrical circuitry includes Bluetooth@ communication circuitry.

5. The nerve stimulation earpiece of any one of claims 2 to 4, wherein the sound delivery portion includes a channel through the ear canal insert to permit passage of sound through the ear canal insert to the ear canal of the subject.

6. A nerve stimulation system comprising the nerve stimulation earpiece of claims 1-5, further comprising:
an ear stimulation device controller including
a first analog output connector adapted to connect a first current signal to the at least one first electrode of the nerve stimulation earpiece;
a second analog output connector adapted to connect a second current signal to the at least one second electrode of the nerve stimulation earpiece;
a wireless microcontroller configured to control wireless communication between the ear stimulation device controller and a personal computing device to receive one or more stimulation parameters from the personal computing device;
a digital stimulus signal generator configured to generate a digital stimulus signal based at least in part on the one or more stimulation parameters received from the personal computing device;
a digital-to-analog converter for converting the digital stimulus signal to an analog voltage waveform;
a current driver operably connected to the digital-to-analog converter and adapted generate a controlled current stimulus waveform responsive to the analog voltage waveform, wherein the controlled current stimulus waveform is provided to the ear stimulation device via at least the first analog output connector and the second analog output connector; and
a power source operably connected to at least one of the wireless microcontroller, the digital stimulus signal generator, the digital-to-analog converter, and the current driver.

7. The ear stimulation device controller of claim 6, further including a physiological signal input adapted to receive a physiological signal.

8. The ear stimulation device controller of claim 7, wherein the first analog output connector and the second analog output connector are configured to be connected to the ear stimulation device located on a first ear of the user and wherein the physiological signal input is configured to receive the physiological signal from a physiological sensor located on a second ear of the user.

## Patentansprüche

1. Nervenstimulationsohrstück (3000), umfassend:
einen Gehörgangeinsatz (3005), der dazu geeignet ist, in einen Gehörgang eines menschlichen Subjekts zu passen, wobei der Gehörgangeinsatz aufweist:
mindestens eine erste Elektrode (3030), die dazu konfiguriert ist, die Haut innerhalb des Gehörgangs des Subjekts elektrisch zu kontaktieren;
einen Conchaeinsatz (3010), der dazu geeignet ist, in eine Concha des Subjekts zu passen, wobei der Conchaeinsatz aufweist:
einen Basisabschnitt (3015), der dazu konfiguriert ist, in ein Cavum conchae des Subjekts zu passen;
einen Flügelabschnitt (3020), der dazu konfiguriert ist, in eine Cymba conchae des Subjekts zu passen; und
mindestens eine zweite Elektrode (3035), die dazu konfiguriert ist, mindestens einen Abschnitt der Concha des Subjekts elektrisch zu kontaktieren;
mindestens einen ersten elektrischen Steckverbinder zum Verbinden der mindestens einen ersten Elektrode am Gehörgangeinsatz mit einer ersten elektrischen Stromquelle; und
mindestens einen zweiten elektrischen Steckverbinder zum Verbinden der mindestens einen zweiten Elektrode am Conchaeinsatz mit einer zweiten elektrischen Stromquelle.

2. Nervenstimulationsohrstück nach Anspruch 1, wobei der Gehörgangeinsatz einen Tonabgabeabschnitt aufweist, der dazu geeignet ist, Töne an den Gehörgang des Subjekts abzugeben.

3. Nervenstimulationsohrstück nach Anspruch 1 oder Anspruch 2, ferner eine drahtlose elektrische Kommunikationsschaltung aufweisend, die dazu geeignet ist, ein Datensignal zu senden, ein Audiosignal zu empfangen oder ein Audiosignal zu empfangen.

4. Nervenstimulationsohrstück nach Anspruch 3, wobei die drahtlose elektrische Kommunikationsschaltung eine Bluetooth®-Kommunikationsschaltung aufweist.

5. Nervenstimulationsohrstück nach einem der Ansprüche 2 bis 4, wobei der Tonabgabeabschnitt einen Kanal durch den Gehörgangeinsatz aufweist, um einen Tondurchgang durch den Gehörgangeinsatz in den Gehörgang des Subjekts zu ermöglichen.

6. Nervenstimulationssystem, umfassend das Nervenstimulationsohrstück nach den Ansprüchen 1 bis 5, ferner umfassend:
eine Steuereinheit der Ohrstimulationsvorrichtung, aufweisend
einen ersten analogen Ausgabesteckverbinder, der dazu geeignet ist, ein erstes Stromsignal mit der mindestens einen ersten Elektrode des Nervenstimulationsohrstücks zu verbinden;
einen zweiten analogen Ausgabesteckverbinder, der dazu geeignet ist, ein zweites Stromsignal mit der mindestens einen zweiten Elektrode des Nervenstimulationsohrstücks zu verbinden;
eine drahtlose Mikrosteuereinheit, die dazu konfiguriert ist, eine drahtlose Kommunikation zwischen der Steuereinheit der Ohrstimulationsvorrichtung und einer persönlichen Rechenvorrichtung zu steuern, um einen oder mehrere Stimulationsparameter von der persönlichen Rechenvorrichtung zu empfangen;
ein Erzeuger digitaler Stimulussignale, der dazu konfiguriert ist, ein digitales Stimulussignal zumindest teilweise auf der Grundlage des einen oder der mehreren Stimulationsparameter zu erzeugen, die von der persönlichen Rechenvorrichtung empfangen werden;
einen Digital-Analog-Wandler zum Umwandeln des digitalen Stimulussignals in eine analoge Spannungswellenform;
einen Stromtreiber, der mit dem Digital-Analog-Wandler wirkverbunden und dazu geeignet ist, eine gesteuerte Stromstimulus-Wellenform zu erzeugen, die auf die analoge Spannungswellenform reagiert, wobei die gesteuerte Stromstimulus-Wellenform für die Ohrstimulationsvorrichtung über mindestens den ersten analogen Ausgabesteckverbinder und den zweiten analogen Ausgabesteckverbinder bereitgestellt wird; und
eine Leistungsquelle, die mit mindestens einem von der drahtlosen Mikrosteuereinheit, dem Erzeuger digitaler Stimulussignale, dem Digital-Analog-Wandler und dem Stromtreiber wirkverbunden ist.

7. Steuereinheit der Ohrstimulationsvorrichtung nach Anspruch 6, ferner einen physiologischen Signaleingang aufweisend, der dazu geeignet ist, ein physiologisches Signal zu empfangen.

8. Steuereinheit der Ohrstimulationsvorrichtung nach Anspruch 7, wobei der erste analoge Ausgabesteckverbinder und der zweite analoge Ausgabesteckverbinder dazu konfiguriert sind, mit der an einem ersten Ohr des Benutzers befindlichen Ohrstimulationsvorrichtung verbunden zu werden, und wobei der physiologische Signaleingang dazu konfiguriert ist, das physiologische Signal von einem an einem zweiten Ohr des Benutzers befindlichen physiologischen Sensor zu empfangen.

## Revendications

1. Écouteur de stimulation nerveuse (3000) comprenant :
un élément inséré (3005) de conduit auditif conçu pour s'ajuster dans un conduit auditif d'un sujet humain, l'élément inséré de conduit auditif comprenant
au moins une première électrode (3030) configurée pour entrer en contact électrique avec la peau à l'intérieur du conduit auditif du sujet ;
un élément inséré (3010) de conque conçu pour s'ajuster à l'intérieur d'une conque du sujet, l'élément inséré de conque comprenant
une partie de base (3015) conçue pour s'ajuster à l'intérieur d'une cavité de la conque du sujet ;
une partie ailette (3020) conçue pour s'ajuster à l'intérieur d'une cymba de la conque du sujet ; et
au moins une seconde électrode (3035) configurée pour être en contact électrique avec au moins une partie de la conque du sujet ;
au moins un premier connecteur électrique pour connecter l'au moins une première électrode sur l'élément inséré de conduit auditif à une première source de courant électrique ; et
au moins un second connecteur électrique pour connecter l'au moins une seconde électrode sur l'élément inséré de conduit auditif à une seconde source de courant électrique.

2. Écouteur de stimulation nerveuse selon la revendication 1, dans lequel l'élément inséré de conduit auditif comprend une partie de distribution sonore conçue pour distribuer un son au conduit auditif du sujet.

3. Écouteur de stimulation nerveuse selon la revendication 1 ou revendication 2, comprenant en outre un ensemble de circuits électriques de communication sans fil conçus pour émettre un signal de données, recevoir un signal audio, ou recevoir un signal audio.

4. Écouteur de stimulation nerveuse selon la revendication 3, dans lequel l'ensemble de circuits électriques de communication sans fil comprend un ensemble de circuits de communication Bluetooth®.

5. Écouteur de stimulation nerveuse selon l'une quelconque des revendications 2 à 4, dans lequel la partie de distribution sonore comprend un canal à travers l'élément inséré de conduit auditif pour permettre le passage du son à travers l'élément inséré de conduit auditif vers le conduit auditif du sujet.

6. Système de stimulation nerveuse comprenant l'écouteur de stimulation nerveuse selon les revendications 1 à 5, comprenant en outre :
un organe de commande de dispositif de stimulation auditive comprenant
un premier connecteur de sortie analogique conçu pour connecter un premier signal de courant à l'au moins une première électrode de l'écouteur de stimulation nerveuse ;
un second connecteur de sortie analogique conçu pour connecter un second signal de courant à l'au moins une seconde électrode de l'écouteur de stimulation nerveuse ;
un microcontrôleur sans fil configuré pour commander une communication sans fil entre l'organe de commande de dispositif de stimulation auditive et un dispositif informatique personnel pour recevoir un ou plusieurs paramètres de stimulation en provenance du dispositif informatique personnel ;
un générateur de signal de stimulus numérique configuré pour générer un signal de stimulus numérique basé au moins en partie sur le ou les paramètres reçus du dispositif informatique personnel ;
un convertisseur numérique-analogique pour convertir le signal de stimulus numérique en une forme d'onde de tension analogique ;
un pilote de courant connecté fonctionnellement au convertisseur numérique-analogique et conçu pour générer une forme d'onde de stimulus de courant piloté en réponse à la forme d'onde de tension analogique, dans lequel la forme d'onde de stimulus de courant piloté est fournie au dispositif de stimulation auditive par l'intermédiaire au moins du premier connecteur de sortie analogique et du second connecteur de sortie analogique ; et
une source d'alimentation connectée fonctionnellement au microcontrôleur sans fil et/ou au générateur de signal de stimulus numérique et/ou au convertisseur numérique-analogique et/ou au pilote de courant.

7. Organe de commande de dispositif de stimulation auditive selon la revendication 6, comprenant en outre une entrée de signal physiologique conçue pour recevoir un signal physiologique.

8. Organe de commande de dispositif de stimulation auditive selon la revendication 7, dans lequel le premier connecteur de sortie analogique et le second connecteur de sortie analogique sont configurés pour être connectés au dispositif de stimulation auditive situé sur une première oreille de l'utilisateur et dans lequel l'entrée de signal physiologique est configurée pour recevoir le signal physiologique en provenance d'un capteur physiologique situé sur une seconde oreille de l'utilisateur.
